(19) Europäisches Patentamt
European Patent Office
Office européen des brevets

(11) EP 0 868 420 B1

(12) **FASCICULE DE BREVET EUROPEEN**

(45) Date de publication et mention de la délivrance du brevet:
**29.03.2000 Bulletin 2000/13**

(21) Numéro de dépôt: **96941716.1**

(22) Date de dépôt: **06.12.1996**

(51) Int Cl.[7]: **C07D 231/14**, A61K 31/415

(86) Numéro de dépôt international:
**PCT/FR96/01953**

(87) Numéro de publication internationale:
**WO 97/21682 (19.06.1997 Gazette 1997/26)**

(54) **DERIVES DE 3-PYRAZOLECARBOXAMIDE AVEC UNE AFFINITE POUR LE RECEPTEUR DES CANNABINOIDES**

3-PYRAZOLCARBOXAMIDDERIVATE MIT CANNABINOIDREZEPTOR-AFFINITÄT

3-PYRAZOLECARBOXAMIDE DERIVATIVES HAVING CANNABINOID RECEPTOR AFFINITY

(84) Etats contractants désignés:
**AT BE CH DE DK ES FI FR GB GR IE IT LI LU MC NL PT SE**
Etats d'extension désignés:
**LT LV SI**

(30) Priorité: **08.12.1995 FR 9514547**

(43) Date de publication de la demande:
**07.10.1998 Bulletin 1998/41**

(60) Demande divisionnaire: **98401565.1 / 0 885 889**

(73) Titulaire: **Sanofi-Synthélabo**
**75013 Paris (FR)**

(72) Inventeurs:
- **BARTH, Francis**
  **F-34070 Montpellier (FR)**
- **CASELLAS, Pierre**
  **F-34090 Montpellier (FR)**
- **MILLAN, Joseph**
  **F-34990 Juvignac (FR)**
- **OUSTRIC, Didier**
  **F-34920 Le Crès (FR)**
- **RINALDI, Murielle**
  **F-34680 Saint-Georges-d'Orques (FR)**
- **SARRAN, Martine**
  **F-30870 Clarensac (FR)**

(74) Mandataire: **Gillard, Marie-Louise et al**
**Cabinet Beau de Loménie,**
**158, rue de l'Université**
**75007 Paris (FR)**

(56) Documents cités:
**EP-A- 0 477 049**
**EP-A- 0 576 357**
**EP-A- 0 656 354**
**EP-A- 0 658 546**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 868 420 B1

## Description

La présente invention concerne de nouveaux dérivés du pyrazole et leurs sels éventuels, un procédé pour leur préparation et des compositions pharmaceutiques les contenant.

Plus particulièrement la présente invention concerne de nouveaux dérivés du pyrazole qui possèdent une très bonne affinité pour les récepteurs périphériques des cannabinoïdes, appelés récepteurs $CB_2$, et sont utiles dans les domaines thérapeutiques où les récepteurs $CB_2$ sont impliqués.

Le $\Delta^9$-THC est le principal constituant actif de Cannabis Sativa (Tuner, 1985 ; In Marijuana 1984, Ed. Harvey, DY, IRL Press, Oxford).

La caractérisation des récepteurs des cannabinoïdes a été rendue possible par la mise au point de ligands synthétiques tels que les agonistes WIN 55212-2 (J. Pharmacol. Exp. Ther., 1993, 264, 1352-1363) ou CP-55940 (J. Pharmacol. Exp. Ther., 1988, 247, 1046-1051).

De nombreux articles ont décrit non seulement des effets psychotropes des cannabinoïdes mais aussi une influence de ces derniers sur la fonction immunitaire (HOLLISTER L.E., J. Psychoact. Drugs 24, 1992, 159-164). La plupart des études in vitro ont montré des effets immunosuppresseurs des cannabinoïdes : l'inhibition des réponses prolifératives des lymphocytes T et des lymphocytes B induites par les mitogènes (Luo Y.D. et al., Int. J. Immunopharmacol., 1992, 14, 49-56; Schwartz H. et al., J. Ncuroimmunol., 1994, 55, 107-115), l'inhibition de l'activité des cellules T cytotoxiques (Klein et al., J. Toxicol. Environ. Health, 1991, 32, 465-477), l'inhibition de l'activité microbicide des macrophages et de la synthèse du TNF$\alpha$ (Arata S. et al., Life Sci., 1991, 49, 473-479 ; Fisher-Stenger et al., J. Pharm. Exp. Ther., 1993, 267, 1558-1565), l'inhibition de l'activité cytolytique et de la production de TNF$\alpha$ de certains lymphocytes (Kusher et al., Cell. Immun., 1994, 154, 99-108). Inversement dans certaines études, des effets d'amplification ont été observés : augmentation de la bioactivité de l'interleukine-1 par les macrophages résidents de souris ou les lignées cellulaires macrophagiques différenciées, due à des niveaux accrus de TNF$\alpha$ (Zhu et al., J. Pharm. Exp. Ther., 1994, 270, 1334-1339 ; Shivers S.C. et al., Life Sci., 1994, 54, 1281-1289).

Les effets des cannabinoïdes sont dûs à une interaction avec des récepteurs spécifiques de haute affinité présents au niveau central (Devane et al., Molecular Pharmacology, 1988, 34, 605-613) et périphérique (Nye et al., The Journal of Pharmacology and Experimental Therapeutics, 1985, 234, 784-791 ; Kaminski et al., Molecular Pharmacology, 1992, 42, 736-742 ; Munro et al., Nature 1993, 365, 61-65).

Les effets centraux relèvent d'un premier type de récepteurs des cannabinoïdes ($CB_1$) qui est présent dans le cerveau. Par ailleurs, Munro et al. (Nature 1993, 365, 61-65) ont cloné un second récepteur des cannabinoïdes couplé aux protéines G, appelé $CB_2$, qui est seulement présent à la périphérie et plus particulièrement dans les cellules d'origine immune. La présence de récepteurs aux cannabinoïdes $CB_2$ dans les cellules lymphoïdes peut expliquer l'immunomodulation exercée par les agonistes des récepteurs aux cannabinoïdes évoquée ci-dessus.

De nombreux dérivés du pyrazole ont été décrits dans la littérature : plus particulièrement EP-A-268554 et DE-A-3910248 revendiquent des pyrazoles possédant des propriétés herbicides, EP-A-430186 et JP-A-3031840 revendiquent des composés utiles pour la photographie et EP-A-418845 revendique des pyrazoles pourvus d'activité antiinflammatoire, analgésique et antithrombotique.

Des dérivés du pyrazolecarboxamide ont également été décrits, notamment dans les demandes de brevet EP-A-0289879 et EP-A-0492125 : ces composés possèdent des propriétés insecticides.

En outre, la demande de brevet EP-A-0477049 décrit des dérivés du pyrazole-3-carboxamide de formule :

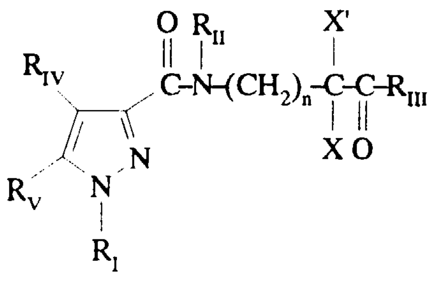

(1)

dans laquelle par exemple :

- $R_I$ représente un groupe aryle diversement substitué ;
- $R_{II}$ représente l'hydrogène ou un $(C_1-C_4)$alkyle ;
- $R_{III}$ représente un groupe hydroxyle, un $(C_1-C_6)$alcoxy, un groupe amino ;
- $R_{IV}$ représente l'hydrogène, un halogène ou un $(C_1-C_6)$alkyle ;

- $R_V$ représente un groupe phényle diversement substitué ;
- n est 0, 1, 2, ou 3 ;

Ces composés possèdent une activité sur le système nerveux central et plus particulièrement par interaction avec le récepteur de la neurotensine.
De plus, les demandes de brevet EP-A-576357 et EP-A-658546 décrivent des dérivés de pyrazole présentant une affinité pour les récepteurs aux cannabinoïdes. La demande de brevet EP-A-656354 revendique par ailleurs le N-pipéridino-5-(4-chlorophényl)-1-(2,4-dichlorophényl)-4-méthylpyrazole-3-carboxamide ou SR 141716 et ses sels pharmaceutiquement acceptables qui présentent une très bonne affinité pour les récepteurs centraux aux cannabinoïdes.
On a maintenant trouvé de nouveaux dérivés du pyrazole qui présentent une affinité élevée pour le récepteur $CB_2$ humain et une spécificité pour ledit récepteur et qui sont de puissants immunomodulateurs.
Dans la présente description, on désigne par "affinité élevée pour le récepteur $CB_2$ humain" une affinité caractérisée par une constante d'affinité généralement inférieure à 100 nM allant jusqu'à 0,1 nM et par "spécifique" les composés dont la constante d'affinité pour le récepteur $CB_2$ est généralement au moins 10 fois inférieure à la constante d'affinité pour le récepteur $CB_1$.
Selon un de ses aspects, la présente invention a pour objet des composés de formule :

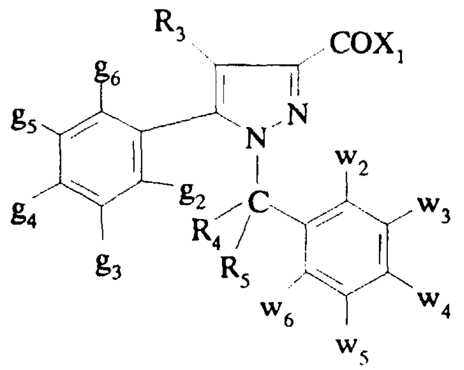

(I)

dans laquelle :

- $X_1$ représente un groupe $-NR_1R_2$ ou un groupe $-OR_2$ ;
- $g_2$, $g_3$, $g_4$, $g_5$, $g_6$ et $w_2$, $w_3$, $w_4$, $w_5$, $w_6$ sont identiques ou différents, et représentent chacun indépendamment l'hydrogène, un atome d'halogène, un $(C_1\text{-}C_4)$alkyle, un $(C_1\text{-}C_4)$alcoxj, un trifluorométhyle, un nitro, un $(C_1\text{-}C_4)$ alkylthio ; à la condition qu'au moins un des substituants $g_2$, $g_3$, $g_4$, $g_5$, $g_6$ et au moins un des substituants $w_2$, $w_3$, $w_4$, $w_5$, $w_6$ soient différents de l'hydrogène ;
- $R_1$ représente l'hydrogène ou un $(C_1\text{-}C_4)$alkyle ;
- $R_2$ représente un radical carbocyclique non aromatique en $(C_3\text{-}C_{15})$ non substitué ou substitué une ou plusieurs fois par un substituant choisi parmi un atome d'halogène, un $(C_1\text{-}C_4)$alkyle ou un $(C_1\text{-}C_4)$alcoxy ;
- $R_3$ représente l'hydrogène ou un groupe $-CH_2\text{-}R_6$ ;
- $R_4$ et $R_5$ représentent chacun indépendamment un hydrogène, un $(C_1\text{-}C_4)$alkyle ou un trifluorométhyle ;
- ou bien $R_4$ représente l'hydrogène et $R_5$ et $w_6$ ensemble constituent un radical éthylène ou un radical triméthylène ;
- $R_6$ représente l'hydrogène, ou lorsque les substituants $g_2$, $g_3$, $g_4$, $g_5$ et/ou $g_6$ sont autres qu'un $(C_1\text{-}C_4)$alkyle, $R_6$ représente l'hydrogène, un $(C_1\text{-}C_4)$alkyle, un fluor, un hydroxy, un $(C_1\text{-}C_5)$alcoxy, un $(C_1\text{-}C_5)$alkylthio, un hydroxy $(C_1\text{-}C_5)$alcoxy, un cyano, un $(C_1\text{-}C_5)$alkylsulfinyle, un $(C_1\text{-}C_5)$alkylsulfonyle ;

ainsi que leurs sels éventuels.
Lorsqu'un composé de formule (I) selon l'invention comprend un ou plusieurs atomes de carbone asymétriques, les différents isomères optiques ainsi que les racémiques font partie de l'invention.
Les sels éventuels des composés de formule (I) comprennent les sels d'addition d'acides pharmaceutiquement acceptables tels que le chlorhydrate, le bromhydrate, le sulfate, l'hydrogénosulfate, le dihydrogénophosphate, le méthanesulfonate, le méthylsulfate, le maléate, l'oxalate, le fumarate, le naphtalènesulfonate, le glyconate, le gluconate, le citrate, l'iséthionate, le paratoluènesulfonate, le mésitylènesulfonate ou le benzènesulfonate.
Les radicaux carbocycliques non aromatiques en $C_3\text{-}C_{15}$ comprennent les radicaux mono ou polycycliques, condensés, pontés ou spiraniques, saturés ou insaturés, éventuellement terpéniques. Ces radicaux sont éventuellement substitués, une ou plusieurs fois par un groupe choisi parmi les groupes $(C_1\text{-}C_4)$alkyle, $(C_1\text{-}C_4)$alcoxy ou halogè-

ne, étant entendu que dans le cas des terpènes ou des radicaux terpéniques, par exemple bornyle, menthyle ou menthényle, les groupes alkyles du terpène ne sont pas considérés comme des substituants.

Les radicaux monocycliques incluent les cycloalkyles par exemple cyclopropyle, cyclopentyle, cyclohexyle, cycloheptyle, cyclooctyle, cyclododécyle non substitués ou substitués une ou plusieurs fois par un groupe $(C_1-C_4)$ alkyle, un groupe $(C_1-C_4)$alcoxy ou un halogène, comme par exemple le 2-méthylcyclohex-1-yle, le 2,6-diméthylcyclohex-1-yle, le 2,2,6,6-tétraméthylcyclohex-1-yle.

Les radicaux di- ou tricycliques condensés, pontés, ou spiraniques, éventuellement terpéniques incluent par exemple les radicaux bicyclo[2.2.1]heptyle ou norbornyle, le bornyle, l'isobornyle, le noradamantyle, l'adamantyle, le bicyclo[3.2.1]octyle, le bicyclo[2.2.2]octyle, le tricyclo[5.2.1.$0^{2,6}$]décyle, le spiro[5,5]undécyle, le bicyclo[2.2.2]oct-2-èn-5-yle, le tricyclo[2.2.1.$0^{2,6}$]hept-3-yle, lesdits radicaux étant non substitués ou substitués une ou plusieurs fois par un $(C_1-C_4)$alkyle, un halogène ou un $(C_1-C_4)$alcoxy, comme par exemple le 1,3,3-triméthyl-bicyclo[2.2.1]hept-2-yle ou fenchyle.

Dans la présente description les groupes alkyles ou les groupes alcoxy sont droits ou ramifiés. Par atome d'halogène on entend un atome de chlore, de brome, de fluor ou d'iode.

Selon la présente invention, on préfère les composés de formule (I) dans laquelle :

- $X_1$ représente un groupe $-NR_1R_2$ ;
- $g_2$, $g_3$, $g_4$, $g_5$, $g_6$ et $w_2$, $w_3$, $w_4$, $w_5$, $w_6$ sont identiques ou différents, et représentent chacun indépendamment l'hydrogène, un atome d'halogène, un $(C_1-C_4)$alkyle, un $(C_1-C_4)$alcoxy, un trifluorométhyle, un nitro, un $(C_1-C_4)$ alkylthio ; à la condition qu'au moins un des substituants $g_2$, $g_3$, $g_4$, $g_5$, $g_6$ et au moins un des substituants $w_2$, $w_3$, $w_4$, $w_5$, $w_6$ soient différents de l'hydrogène ;
- $R_1$ représente l'hydrogène ou un $(C_1-C_4)$alkyle ;
- $R_2$ représente un radical carbocyclique non aromatique en $(C_3-C_{15})$ non substitué ou substitué une ou plusieurs fois par un substituant choisi parmi un atome d'halogène, un $(C_1-C_4)$alkyle ou un $(C_1-C_4)$alcoxy ;
- $R_3$ représente l'hydrogène ou un groupe $-CH_2-R_6$ ;
- $R_4$ et $R_5$ représentent chacun indépendamment un hydrogène, un $(C_1-C_4)$alkyle ou un trifluorométhyle ;
- $R_6$ représente l'hydrogène, un groupe méthyle ou un groupe éthyle ;

ainsi que leurs sels éventuels.

Parmi les composés de formule (I), dans laquelle $X_1$ représente un groupe $-NR_1R_2$, on préfère ceux pour lesquels $R_1$ représente l'hydrogène.

Parmi les composés de formule (I), dans laquelle $X_1$ représente un groupe $-NR_1R_2$ ou un groupe $-OR_2$, on préfère ceux pour lesquels $R_2$ représente un radical 1,3,3-triméthylbicyclo[2.2.1]hept-2-yle ou un radical bicyclo[3.2.1]oct-3-yle.

Parmi les composés de formule (I), on préfère ceux pour lesquels $R_3$ représente l'hydrogène ou un groupe $-CH_2-R_6$ avec $R_6$ représentant l'hydrogène.

Parmi les composés de formule (I), on préfère ceux pour lesquels soit $R_4$ et $R_5$ représentent chacun l'hydrogène, soit $R_4$ représente l'hydrogène et $R_5$ représente un $(C_1-C_4)$alkyle.

Parmi les composés de formule (I), on préfère ceux pour lesquels $g_2$, $g_5$ et $g_6$ représentent l'hydrogène et $g_3$ et $g_4$ sont tels que définis ci-dessus pour les composés de formule (I).

Parmi les composés de formule (I), on préfère ceux pour lesquels $w_5$ et $w_6$ représentent l'hydrogène, $w_4$ représente un atome d'halogène, un $(C_1-C_4)$alkyle, un $(C_1-C_4)$alcoxy, un trifluorométhyle ou un $(C_1-C_4)$alkylthio, et soit $w_2$ et $w_3$ représentent chacun l'hydrogène, soit l'un représente l'hydrogène et l'autre représente un atome d'halogène, un $(C_1-C_4)$alkyle ou un trifluorométhyle.

Un groupe de composés préférés selon la présente invention est le groupe des composés de formule:

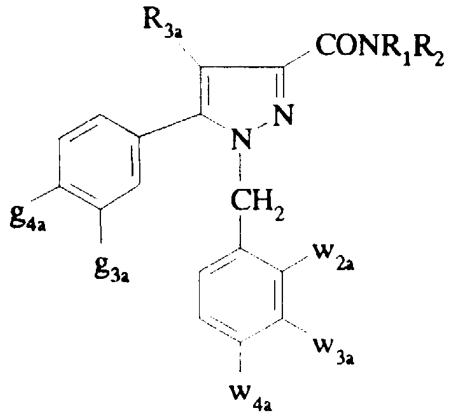

(Ia)

dans laquelle :

- $R_1$, $R_2$ sont tels que définis pour les composés de formule (I) ;
- $R_{3a}$ représente l'hydrogène ou un groupe $-CH_2-R_{6a}$ ;
- $R_{6a}$ représente l'hydrogène ou à la condition que les substituants $g_{3a}$ et $g_{4a}$ soient autres qu'un $(C_1-C_4)$alkyle, $R_{6a}$ représente l'hydrogène, un groupe méthyle ou un groupe éthyle ;
- $g_{3a}$ représente l'hydrogène, un atome d'halogène, un $(C_1-C_4)$alkyle ou un trifluorométhyle ;
- $g_{4a}$ représente un atome d'halogène, un $(C_1-C_4)$alkyle ou un trifluorométhyle ;
- $w_{4a}$ représente un atome d'halogène, un $(C_1-C_4)$alkyle ou un trifluorométhyle ;
- $w_{2a}$ et $w_{3a}$ représentent chacun l'hydogène ou l'un représente l'hydrogène et l'autre représente un atome d'halogène, un $(C_1-C_4)$alkyle ou un trifluorométhyle ;

ainsi que leurs sels éventuels.

Parmi ces composés, ceux de formule :

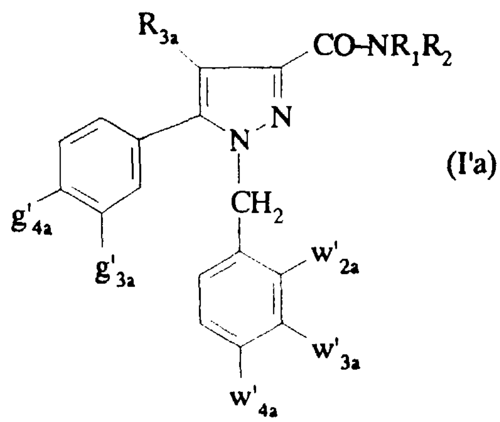

(I'a)

dans laquelle :

- $R_1$, $R_2$ sont tels que définis pour les composés de formule (I) ;
- $R_{3a}$ est tel que défini pour les composés de formule (Ia);
- $g'_{3a}$ représente l'hydrogène, un atome de chlore, un atome de fluor, un groupe méthyle ou un trifluorométhyle ;
- $g'_{4a}$ représente un atome de chlore, un atome de fluor, un groupe méthyle ou un trifluorométhyle ;
- $w'_{4a}$ représente un atome de chlore, un atome de fluor, un groupe méthyle ou un trifluorométhyle ;
- $w'_{2a}$ et $w'_{3a}$ représentent chacun l'hydrogène ou l'un représente l'hydrogène et l'autre représente un atome de chlore, un atome de fluor, un groupe méthyle ou un trifluorométhyle ;

et leurs sels éventuels, sont particulièrement préférés.

Les composés plus particulièrement préférés sont les composés de formule (I'a) dans laquelle :

- $R_1$ représente l'hydrogène ;
- $R_2$ représente un radical 1,3,3-triméthylbicyclo[2.2.1]hept-2-yle ou un radical bicyclo[3.2.1]oct-3-yle ;
- $R_{3a}$ est tel que défini pour les composés de formule (Ia);
- -$w'_{2a}$, $w'_{3a}$, $w'_{4a}$, $g'_{3a}$ et $g'_{4a}$ sont tels que définis pour un composé de formule (I'a) ;

ainsi que leurs sels éventuels.

Tout particulièrement on préfère les composés de formule (I'a) dans laquelle :

- $g'_{3a}$ représente l'hydrogène, un atome de chlore, un atome de fluor ou un groupe méthyle ;
- $g'_{4a}$ représente un atome de chlore, un atome de fluor ou un groupe méthyle ;
- $w'_{4a}$ représente un atome de chlore, un atome de fluor ou un groupe méthyle ;
- $w'_{2a}$ et $w'_{3a}$ représentent chacun l'hydrogène ou l'un représente l'hydrogène et l'autre représente un atome de chlore, un atome de fluor ou un groupe méthyle ;
- $R_1$, $R_2$ et $R_{3a}$ sont tels que définis pour les composés de formule (I'a) ; ainsi que leurs sels éventuels.

Un autre groupe de composés préférés selon l'invention est le groupe des composés de formule :

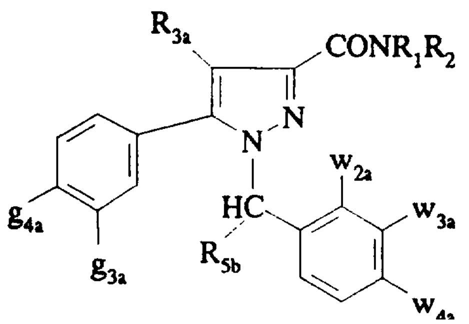

(Ib)

dans laquelle :

- $R_1$, $R_2$ sont tels que définis pour les composés de formule (I) ;
- $R_{3a}$, $w_{2a}$, $w_{3a}$, $w_{4a}$, $g_{3a}$ et $g_{4a}$ sont tels que définis pour les composés de formule (Ia);
- $R_{5b}$ représente un $(C_1-C_4)$alkyle ;

ainsi que leurs sels éventuels.

Parmi ces composés, ceux de formule :

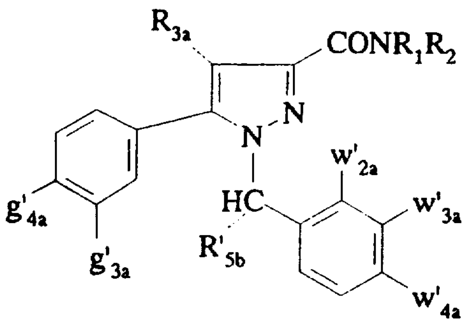

(I'b)

dans laquelle :

- $R_1$, $R_2$ sont tels que définis pour les composés de formule (I) ;
- $R_{3a}$ est tel que défini pour les composés de formule (Ia);
- $w'_{2a}$, $w'_{3a}$, $w'_{4a}$, $g'_{3a}$ et $g'_{4a}$ sont tels que définis pour les composés de formule (I'a) ;
- $R'_{5b}$ représente un groupe méthyle;

ainsi que leurs sels éventuels, sont particulièrement préférés.
Les composés plus particulièrement préférés sont les composés de formule (I'b) dans laquelle :

- $R_1$ représente l'hydrogène ;
- $R_2$ représente un radical 1,3,3-triméthylbicyclo[2.2.1]hept-2-yle ou un radical bicyclo[3.2.1]oct-3-yle ;
- $R_{3a}$ est tel que défini pour les composés de formule (Ia) ;
- $R'_{5b}$ représente un groupe méthyle ;
- $w'_{2a}$, $w'_{3a}$, $w'_{4a}$, $g'_{3a}$ et $g'_{4a}$ sont tels que définis pour les composés de formule (I'a) ;

ainsi que leurs sels éventuels.
Tout particulièrement on préfère les composés de formule (I'b) dans laquelle :

- $g'_{3a}$ représente l'hydrogène, un atome de chlore, un atome de fluor ou un groupe méthyle ;
- $g'_{4a}$ représente un atome de chlore, un atome de fluor ou un groupe méthyle ;
- $w'_{4a}$ représente un atome de chlore, un atome de fluor ou un groupe méthyle ;
- $w'_{2a}$ et $w'_{3a}$ représentent chacun l'hydrogène ou l'un représente l'hydrogène et l'autre représente un atome de chlore, un atome de fluor ou un groupe méthyle ;
- $R_1$, $R_2$, $R_{3a}$ et $R'_{5b}$ sont tels que définis pour les composés de formule (I'b);

ainsi que leurs sels éventuels.
Un autre groupe de composés préférés selon l'invention est le groupe des composés de formule :

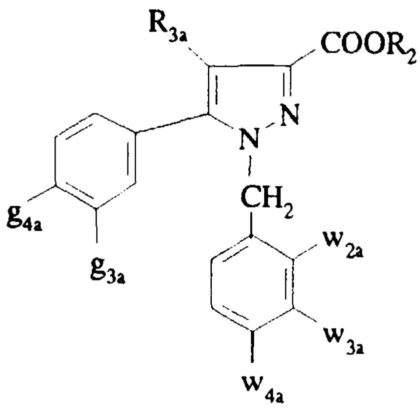

(Ic)

dans laquelle :

- $R_2$ est tel que défini pour un composé de formule (I) ;
- $R_{3a}$, $w_{2a}$, $w_{3a}$, $w_{4a}$, $g_{3a}$ et $g_{4a}$ sont tels que définis pour les composés de formule (Ia) ;

ainsi que leurs sels éventuels.
Selon un autre de ses aspects, la présente invention concerne un procédé pour la préparation des composés de formule (I) et de leurs sels, caractérisé en ce que :

1) on traite un dérivé fonctionnel de l'acide pyrazole-3-carboxylique de formule:

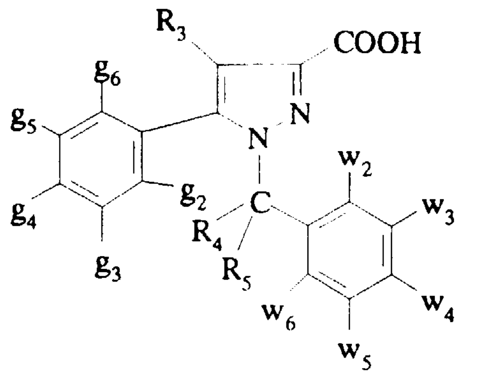

(II)

dans laquelle $w_2$, $w_3$, $w_4$, $w_5$, $w_6$, $g_2$, $g_3$, $g_4$, $g_5$, $g_6$, $R_3$, $R_4$ et $R_5$ sont tels que définis pour les composés de formule (I), avec un composé de formule :

$$H\text{-}X_1 \qquad \text{(XXIV)}$$

dans laquelle $X_1$ est tel que défini pour les composés de formule (I) ;
2) et, éventuellement on transforme le composé ainsi obtenu en l'un de ses sels.

L'un des procédés d'obtention selon l'invention (procédé A) convient pour la préparation des composés de formule (I) dans laquelle $X_1$ représente un groupe $-NR_1R_2$.
Ce procédé est caractérisé en ce que :

1) on traite un dérivé fonctionnel de l'acide pyrazole-3-carboxylique de formule (II) tel que défini précédemment avec une amine de formule :

$$HNR_1R_2 \qquad \text{(III)}$$

dans laquelle $R_1$ et $R_2$ sont tels que définis pour les composés de formule (I) ;
2) et, éventuellement on transforme le composé ainsi obtenu en l'un de ses sels.

Comme dérivé fonctionnel de l'acide (II) on peut utiliser le chlorure d'acide, l'anhydride, un anhydride mixte, un ester alkylique en $C_1$-$C_4$ dans lequel l'alkyle est droit ou ramifié, un ester activé, par exemple l'ester de *p*-nitrophényle ou l'acide libre opportunément activé, par exemple, avec le N,N-dicyclohexylcarbodiimide ou avec l'hexafluorophosphate de benzotriazol-1-yloxytris(diméthylamino)phosphonium (BOP).
Ainsi dans le procédé A selon l'invention, on peut faire réagir le chlorure de l'acide pyrazole-3-carboxylique, obtenu par réaction du chlorure de thionyle sur l'acide de formule (II), avec une amine $HNR_1R_2$, dans un solvant inerte tel qu'un solvant chloré (le dichlorométhane, le dichloroéthane, le chloroforme par exemple), un éther (tétrahydrofurane, dioxane par exemple) ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.
Une variante au mode opératoire du procédé A consiste à préparer l'anhydride mixte de l'acide de formule (II) par réaction du chlorofonniate d'éthyle avec l'acide de formule (II), en présence d'une base telle que la triéthylamine, et à le faire réagir avec une amine $HNR_1R_2$, dans un solvant tel que le dichlorométhane, sous une atmosphère inerte, à la température ambiante, en présence d'une base telle que la triéthylamine.
Un autre procédé d'obtention (procédé B) selon l'invention convient pour la préparation des composés de formule (I) dans laquelle $X_1$ représente un groupe $-OR_2$.
Ce procédé est caractérisé en ce que :

1) on traite un dérivé fonctionnel de l'acide pyrazole-3-carboxylique de formule (II) tel que défini précédemment avec un alcool de formule :

$$HO\text{-}R_2 \quad (XIV)$$

dans laquelle $R_2$ est tel que défini pour un composé de formule (I) ;

2) et, éventuellement on transforme le composé ainsi obtenu en l'un de ses sels.

Comme dérivé fonctionnel de l'acide (II) on peut utiliser le chlorure d'acide, l'anhydride, un anhydride mixte ou l'acide libre opportunément activé, par exemple, avec le N,N-dicyclohexylcarbodiimide ou avec l'hexafluorophosphate de benzotriazol-1-yloxytris(diméthylamino)phosphonium (BOP).

Ainsi dans le procédé B selon l'invention, on peut faire réagir le chlorure de l'acide pyrazole-3-carboxylique, obtenu par réaction du chlorure de thionyle sur l'acide de formule (II), avec un alcool HO-$R_2$ soit dans un solvant inerte tel qu'un solvant chloré (le dichlorométhane, le dichloroéthane, le chloroforme par exemple), un éther (tétrahydrofurane, dioxane par exemple) ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine, soit dans la pyridine à température ambiante en présence de 4-diméthylaminopyridine.

Une variante au mode opératoire du procédé B consiste à préparer l'anhydride mixte de l'acide de formule (II) par réaction du chloroformiate d'éthyle avec l'acide de formule (II), en présence d'une base telle que la triéthylamine, et à le faire réagir avec un alcool HO-$R_2$, dans un solvant tel que le dichlorométhane, sous une atmosphère inerte, à la température ambiante, en présence d'une base telle que la triéthylamine.

Au cours de l'une quelconque des étapes de préparation des composés de formule (I) et plus particulièrement dans la préparation du composé intermédiaire de formule (II), il peut être nécessaire et/ou souhaitable de protéger les groupes fonctionnels réactifs ou sensibles, tels que les groupes amine, hydroxyle, ou carboxy, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, Ed. Plunum Press, 1973 et dans Protective Groups in Organic Synthesis, T.W. Greene et P.G.M. Wutts, Ed. John Wiley et Sons, 1991. L'élimination des groupes protecteurs peut s'effectuer à une étape ultérieure opportune en utilisant les méthodes connues de l'homme de l'art et qui n'affectent pas le reste de la molécule concernée.

Le composé de formule (I) ainsi obtenu est isolé selon les techniques conventionnelles.

Selon la nature des substituants, le composé de formule (I) peut éventuellement être salifié. La salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane, avec une solution de l'acide choisi dans le même solvant, on obtient le sel correspondant qui est isolé selon les techniques classiques.

Ainsi, on prépare par exemple le chlorhydrate, le bromhydrate, le sulfate, l'hydrogénosulfonate, le dihydrogénophosphate, le méthanesulfonate, l'oxalate, le maléate, le fumarate, le naphtalènesulfonate, le benzènesulfonate.

Les composés de formule (II) se préparent selon différents modes opératoires.

Les composés de formule (II) dans laquelle $R_3$ = $R'_3$ et représente l'hydrogène ou un groupe -$CH_2$-$R_6$ dans lequel $R_6$ représente l'hydrogène ou un groupe ($C_1$-$C_4$)alkyle se préparent selon le SCHEMA 1 ci-après :

SCHEMA 1

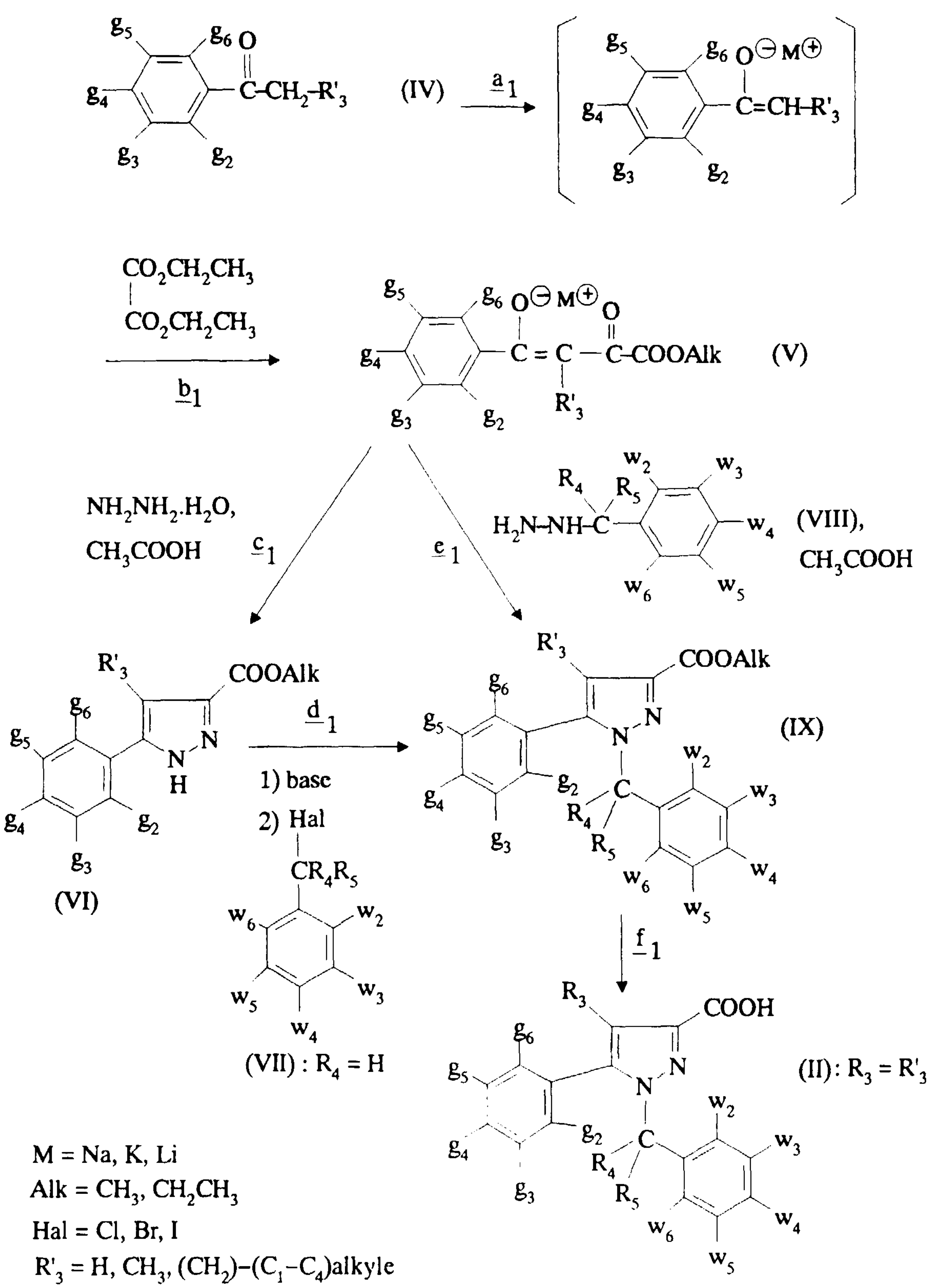


La première étape $\underline{a}_1$ consiste en la préparation d'un sel de métal alcalin d'un dérivé de l'acétophénone de

formule (IV) dans laquelle $R'_3$ représente l'hydrogène ou un groupe $-CH_2-R_6$ dans lequel $R_6$ représente l'hydrogène ou un $(C_1-C_4)$alkyle et $g_2$, $g_3$, $g_4$, $g_5$ et $g_6$ sont tels que définis pour (I) sur lequel est ensuite additionnée, une quantité équimolaire d'oxalate de diéthyle (étape $b_1$) pour obtenir le sel du cétoester de formule (V).

Dans le cas particulier où $R'_3$ = H, le métal alcalin sera préférentiellement le sodium (M = Na), et le sel du cétoester (V, Alk = $CH_3$) sera obtenu selon le procédé décrit dans Bull. Soc. Chim. Fr., 1947, 14, 1098 en utilisant le méthylate de sodium dans le méthanol pour effectuer l'étape $a_1$. On peut également utiliser à l'étape $a_1$ l'action du *tert*-butylate de potassium dans l'éthanol sur le dérivé de formule (IV) puis additionner l'oxalate de diéthyle comme décrit précédemment. La réaction s'effectue à la température de reflux du solvant. On obtient ainsi le composé de formule (V) dans laquelle M = K et Alk = $CH_2CH_3$.

Dans le cas particulier où $R'_3$ = $CH_3$, le métal alcalin sera préférentiellement le lithium (M = Li) et le sel du cétoester de formule (V, Alk = $CH_2CH_3$) sera obtenu selon le procédé décrit dans J. Heterocyclic. Chem., 1989, 26, 1389-1392 en utilisant le sel de lithium de l'hexaméthyldisilazane dans un solvant inerte tel que l'éther diéthylique ou le cyclohexane pour effectuer l'étape $a_1$.

A l'étape $c_1$ le composé de formule (V) ainsi préparé et un excès d'hydrazine (hydrazine monohydrate ou solution aqueuse d'hydrazine) sont chauffés au reflux de l'acide acétique. Par précipitation dans l'eau glacée, on obtient ainsi les pyrazole-3-carboxylates de formule (VI).

A l'étape $d_1$, on traite le composé de formule (VI) ainsi obtenu par une base forte tel que l'hydrure de sodium ou l'amidure de sodium dans un solvant pour fournir un anion qui est mis en réaction avec un composé de formule (VII) dans laquelle Hal représente un halogène de préférence le chlore, le brome ou l'iode, $R_4$ représente l'hydrogène et $w_2$, $w_3$, $w_4$, $w_5$, $w_6$ et $R_5$ sont tels que définis pour les composés de formule (I), pour obtenir le composé de formule (IX). La réaction s'effectue préférentiellement dans le toluène à une température comprise entre la température ambiante et la température de reflux du solvant de façon à obtenir majoritairement le composé de formule (IX) attendu. Lorsqu'on effectue la réaction dans le N,N-diméthylformamide à une température comprise entre 0°C et la température ambiante, on observe majoritairement la formation de l'isomère de position de formule :

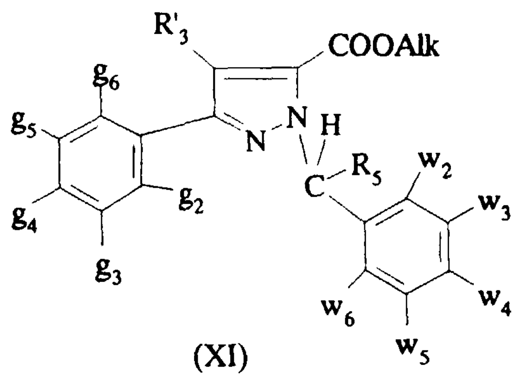

(XI)

Alternativement, selon l'étape $e_1$, le composé de formule (V) et un excès de dérivé de l'hydrazine de formule (VIII) dans laquelle $R_4$, $R_5$, $w_2$, $w_3$, $w_4$, $w_5$, $w_6$ sont tels que définis pour les composés de formule (I) sont chauffés au reflux de l'acide acétique ; par précipitation dans l'eau glacée, on obtient les composés de formule (IX).

A l'étape $f_1$ par hydrolyse en milieu alcalin des composés de formule (IX) puis acidification, on obtient les composés de formule (II) attendus. L'hydrolyse s'effectue en utilisant par exemple un hydroxyde de métal alcalin tel que l'hydroxyde de potassium, l'hydroxyde de sodium, ou l'hydroxyde de lithium dans un solvant inerte tel que l'eau, le méthanol, l'éthanol, le dioxane ou un mélange de ces solvants, à une température comprise entre 0°C et la température de reflux du solvant.

Préférentiellement, on prépare les composés de formule (IX) dans laquelle $R_4$ représente l'hydrogène, en utilisant les étapes $c_1$ puis $d_1$ décrites précédemment.

Préférentiellement, on prépare les composés de formule (IX) dans laquelle $R_4$ et $R_5$ sont différents de l'hydrogène en utilisant l'étape $e_1$ décrite précédemment.

Préférentiellement, on prépare les composés de formule (IX) dans laquelle $R'_3$ = $CH_2-(C_1-C_4)$alkyle soit, lorsque $R_4$ et $R_5$ sont différents de l'hydrogène, à partir des composés de formule (IX) eux-mêmes, soit, lorsque $R_4$ = H, à partir des composés de formule (VI), selon le SCHEMA 2 ci-après.

SCHEMA 2

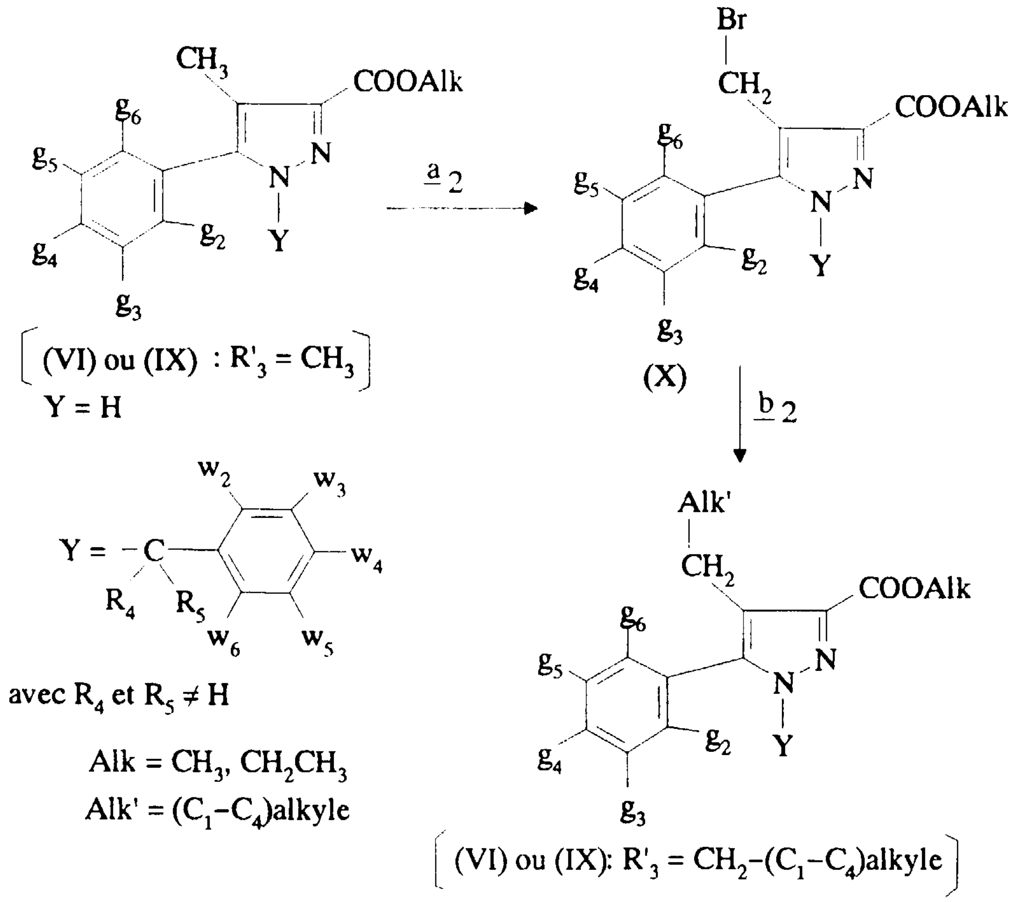


[(VI) ou (IX) : $R'_3 = CH_3$]
Y = H

(X)

avec $R_4$ et $R_5 \neq H$

Alk = $CH_3$, $CH_2CH_3$
Alk' = $(C_1-C_4)$alkyle

[(VI) ou (IX): $R'_3 = CH_2-(C_1-C_4)$alkyle]

L'étape a$_2$ consiste en la préparation d'un 4-bromométhylpyrazole-3-carboxylate de formule (X) par action du N-bromosuccinimide sur un composé de formule (VI) ou (IX) dans lequel $R'_3$ représente un groupe méthyle. La réaction s'effectue dans un solvant inerte comme le tétrachlorure de carbone en présence de peroxyde de dibenzoyle et à la température de reflux du solvant.

Lorsqu'on utilise un composé de formule (VI) on effectue préférentiellement la bromation de l'étape a$_2$, sur un composé dont l'azote du pyrazole est protégé (Y = groupe N-protecteur). Comme groupe N-protecteur on utilise les groupes N-protecteurs classiques bien connus de l'homme de l'art tel que le *tert*-butoxycarbonyle.

L'étape b$_2$ consiste en la préparation d'un composé de formule (VI) ou (IX) dans laquelle $R'_3$ représente un groupe $-CH_2-(C_1-C_4)$alkyle par action d'un organocuprate $(Alk')_2CuLi$ dans lequel Alk' représente un groupe $(C_1-C_4)$ alkyle. La réaction s'effectue selon le procédé décrit dans la demande de brevet EP-A-0658546.

Eventuellement lorsqu'on utilise un composé de formule (VI) protégé sur l'azote du pyrazole, on élimine après l'étape b2 le groupe N-protecteur selon les méthodes connues de l'homme de l'art.

Les composés de formule (II) dans laquelle $R_4$ représente l'hydrogène et $R_3 = R''_3$ et représente un groupe $-CH_2-R_6$ dans lequel $R_6$ est autre que l'hydrogène ou autre qu'un $(C_1-C_4)$alkyle se préparent selon le SCHEMA 3 ci-après :

SCHEMA 3

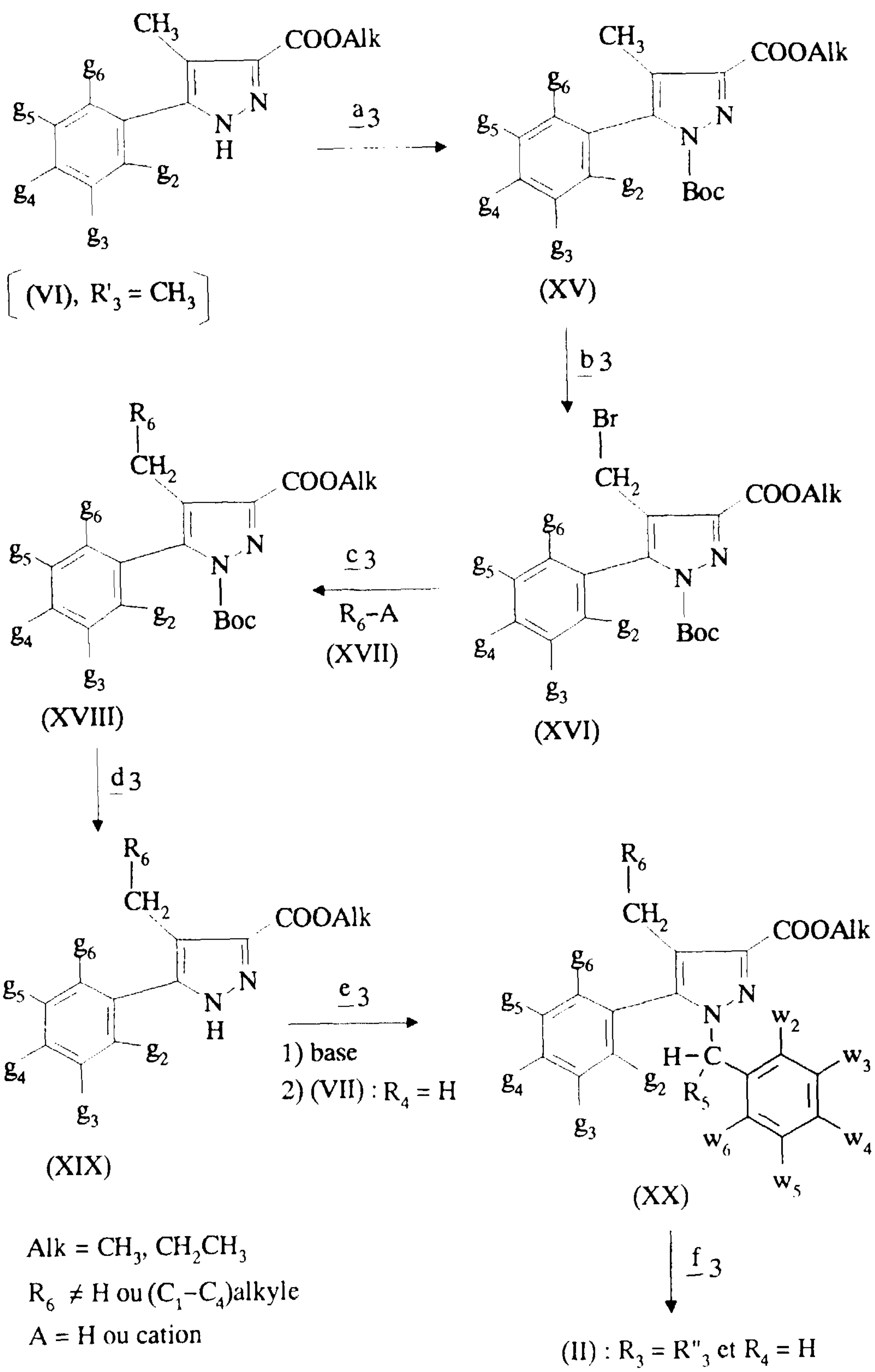


(VI), R'3 = CH3

(XV)

(XVI)

(XVIII)

(XIX)

(XX)

$Alk = CH_3, CH_2CH_3$

$R_6 \neq H$ ou $(C_1-C_4)$alkyle

A = H ou cation

(II) : $R_3 = R''_3$ et $R_4 = H$

A l'étape a3, l'atome d'azote du composé de formule (VI) ($R'_3 = CH_3$) est protégé par un groupe N-protecteur

tel que le *tert*-butoxycarbonyle (Boc), selon les méthodes connues de l'homme de l'art.

L'étape $\underline{b_3}$ consiste en la préparation d'un 4-bromométhylpyrazole-3-carboxylate de formule (XVI) selon la méthode décrite précédemment à l'étape $\underline{a_2}$ du SCHEMA 2.

A l'étape $\underline{c_3}$ on traite le composé de formule (XVI) par un composé de formule $R_6$-A (XVII) dans laquelle $R_6$, tel que défini pour (I), est autre que l'hydrogène ou autre qu'un ($C_1$-$C_4$)alkyle et A représente l'hydrogène ou un cation tel qu'un cation de métal alcalin ou alcalino-terreux ou un groupe ammonium quaternaire tel que le tétraéthylammonium.

Pour préparer un composé de formule (XVIII) dans laquelle $R_6$ est un ($C_1$-$C_5$)alcoxy ou un hydroxy($C_1$-$C_5$) alcoxy, on utilise comme réactif de formule (XVII), un alcool en ($C_1$-$C_5$) ou un dialcool en ($C_1$-$C_5$) en présence d'une base non nucléophile telle qu'un hydrure métallique comme l'hydrure de sodium ou de potassium. Selon les valeurs de $R_6$ on peut obtenir à l'étape $\underline{c_3}$ du procédé un mélange d'esters qui est saponifié à l'étape $\underline{f_3}$ pour donner l'acide de formule (II).

Pour préparer un composé de formule (XVIII) dans laquelle $R_6$ est un ($C_1$-$C_5$)alkylthio, on utilise comme réactif de formule (XVII) un thioalcool en ($C_1$-$C_5$) en présence d'une base non nucléophile telle qu'un hydrure métallique comme l'hydrure de sodium ou de potassium.

Le cas échéant, on peut transformer l'ester de formule (XVIII) obtenu à l'étape $\underline{c_3}$ dans lequel $R_6$ est un ($C_1$-$C_5$)alkylthio par action d'un agent oxydant tel que l'eau oxygénée ou l'acide *meta*chloroperbenzoïque pour obtenir un composé de formule (XVIII) dans lequel $R_6$ représente un ($C_1$-$C_5$)alkylsulfonyle ou un ($C_1$-$C_5$)alkylsulfinyle.

Pour préparer un composé de formule (XVIII) dans lequel $R_6$ est un cyano, on peut utiliser comme réactif de formule (XVII), un cyanure d'ammonium quaternaire, par exemple le cyanure de tétraéthylammonium ou un cyanure métallique tel que le cyanure de sodium ; dans ce dernier cas la réaction de substitution nucléophile de l'étape $\underline{c_3}$ est réalisée en présence d'un catalyseur de transfert de phase.

Pour préparer un composé de formule (XVIII) dans laquelle $R_6$ est le fluor, on peut utiliser comme réactif de formule (XVII) un agent de fluorination ; comme agent de fluorination, on peut citer un fluorure métallique, par exemple le fluorure de potassium utilisé en présence d'un agent complexant comme le Kryptofix®.

Pour préparer un composé de formule (XVIII) dans lequel $R_6$ = OH, on utilise comme réactif (XVII) un hydroxyde de métal alcalin ou alcalino-terreux tel que la soude ou la potasse.

A l'étape $\underline{d_3}$, on élimine le groupe N-protecteur selon les méthodes connues de l'homme de l'art.

A l'étape $\underline{e_3}$, on traite le composé de formule (XIX) ainsi obtenu par une base forte tel que l'hydrure de sodium ou l'amidurc de sodium dans un solvant pour fournir un anion qui est mis en réaction avec un composé de formule (VII) dans laquelle Hal représente un halogène de préférence le chlore, le brome ou l'iode, $R_4$ représente l'hydrogène et $w_2$, $w_3$, $w_4$, $w_5$, $w_6$ et $R_5$ sont tels que définis pour les composés de formule (I), pour obtenir le composé de formule (XX). La réaction s'effectue préférentiellement dans le toluène à une température comprise entre la température ambiante et la température de reflux du solvant de façon à obtenir majoritairement le composé de formule (XX) attendu. Lorsqu'on effectue la réaction dans le N,N-diméthylformamide à température comprise entre 0°C et la température ambiante, on observe majoritairement la formation de l'isomère de nnsitinn de formule:

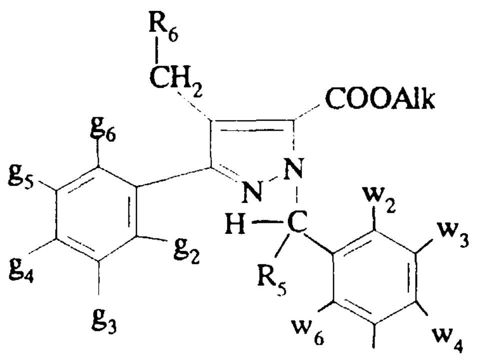

(XXIII)

A l'étape $\underline{f_3}$ par hydrolyse en milieu alcalin des composés de formule (XX) puis acidification, on obtient les composés de formule (II) attendus. L'hydrolyse s'effectue en utilisant par exemple un hydroxyde de métal alcalin tel que l'hydroxyde de potassium, l'hydroxyde de sodium, ou l'hydroxyde de lithium dans un solvant inerte tel que l'eau, le méthanol, l'éthanol, le dioxane ou un mélange de ces solvants, à une température comprise entre 0°C et la température de reflux du solvant.

Les composés de formule (II) dans laquelle $R_3$ = $R''_3$ et représente un groupe -$CH_2$-$R_6$ dans lequel $R_6$ est autre que l'hydrogène ou autre qu'un ($C_1$-$C_4$)alkyle et $R_4$ et $R_5$ sont différents de l'hydrogène, se préparent selon le SCHEMA 4 ci-après dans lequel Alk représente un groupe méthyle ou un groupe éthyle.

SCHEMA 4

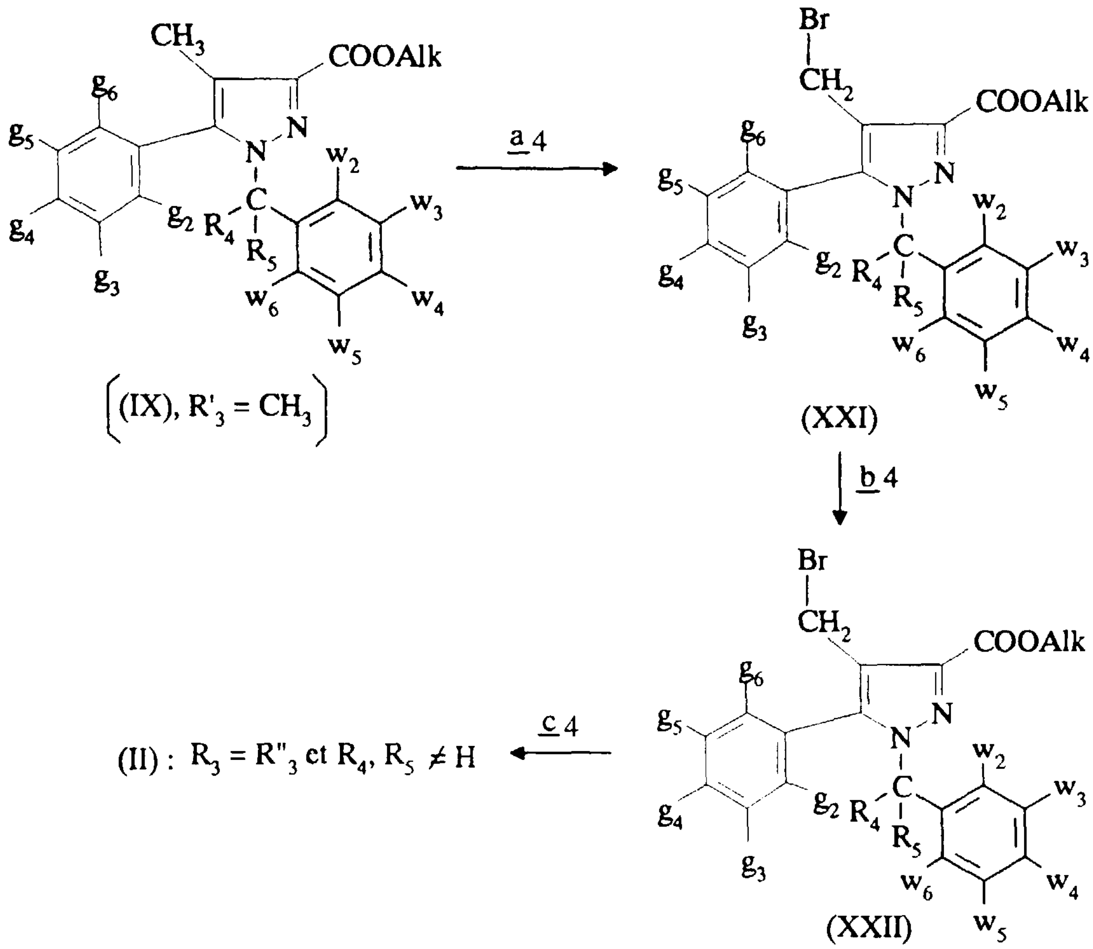


((IX), $R'_3 = CH_3$)

(XXI)

(XXII)

(II) : $R_3 = R''_3$ et $R_4, R_5 \neq H$

L'étape $a_4$ consiste en la préparation d'un 4-bromométhylpyrazole-3-carboxylate de formule (XXI) selon la méthode décrite précédemment à l'étape $a_2$ du SCHEMA 2.

A l'étape $b_4$ on traite le composé de formule (XXI) par un composé de formule $R_6$-A (XVII) tel que défini précédemment et selon les modes opératoires décrits à l'étape $c_3$ du SCHEMA 3.

A l'étape $c_4$ par hydrolyse en milieu alcalin des composés de formule (XXII) puis acidification, on obtient les composés de formule (II) attendus. L'hydrolyse s'effectue selon les méthodes décrites à l'étape $f_1$ du SCHEMA 1.

A l'étape $d_1$ du SCHEMA 1 ou à l'étape $e_3$ du SCHEMA 3, lors de la réaction du composé de formule (VI) ou du composé de formule (XIX) avec le dérivé halogéné de formule (VII), on peut obtenir un mélange en proportions variables du composé de formule (IX) ou du composé de formule (XX) et de leurs isomères respectifs de formule :

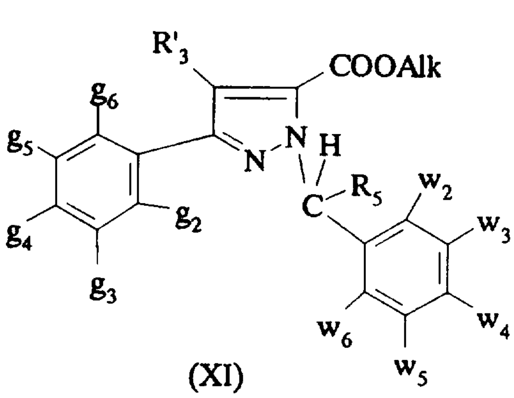

(XI)

ou

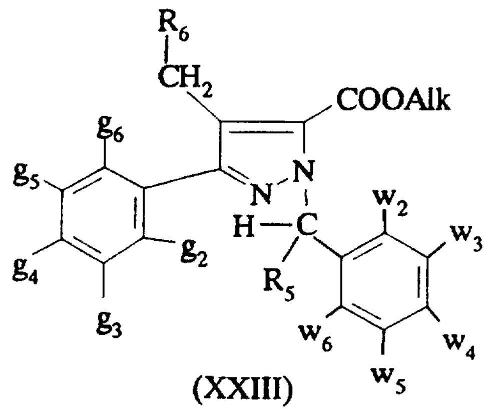

(XXIII)

Les deux isomères (IX) et (XI) ou les deux isomères (XX) et (XXIII) peuvent être séparés par chromatographie sur gel de silice selon les méthodes classiques. Les deux isomères (IX) et (XI) ou (XX) et (XXIII) sont caractérisés par leur spectre de RMN, notamment par l'étude de l'effet Overhauser (N.O.E.).

On peut également effectuer l'étape $f_1$ du procédé ou l'étape $f_3$, telle que décrite dans le SCHEMA 1 ou le SCHEMA 3, sur le mélange des isomères pour obtenir un mélange de l'acide de formule (II) et de son isomère de formule :

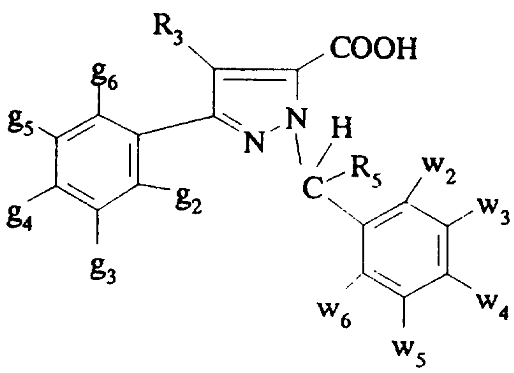

(XII)

On applique alors au mélange des deux isomères (II) et (XII), le procédé selon l'invention précédemment décrit pour obtenir un mélange d'un composé de formule (I) dans laquelle $R_4$ = H et de son isomère de formule:

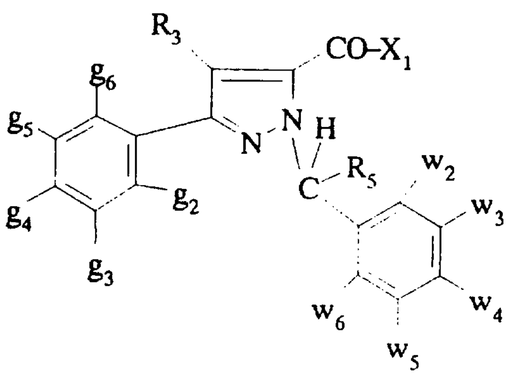

(XIII)

On effectue alors la séparation des deux isomères selon les méthodes classiques telles que par exemple la chromatographie sur gel de silice ou la cristallisation, et on obtient finalement le composé de formule (I) selon l'invention.

Selon un autre de ses aspects, la présente invention a pour objet des composés, sous-produits du procédé de préparation des composés de formule (I), de formule :

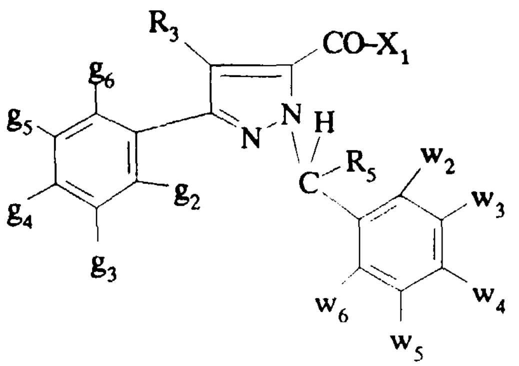

(XIII)

dans laquelle :

- $X_1$, $g_2$, $g_3$, $g_4$, $g_5$, $g_6$, $w_2$, $w_3$, $w_4$, $w_5$, $w_6$, $R_3$ et $R_5$ sont tels que définis pour les composés de formule (I) ;

ainsi que leurs sels éventuels.

Les composés intermédiaires de formule (II) et des composés de formule (XII), utiles pour la préparation des composés de formule (I) dans laquelle $R_4$ = H et des composés de formule (XIII) peuvent être préparés de la manière suivante :

1) on traite un composé de formule :

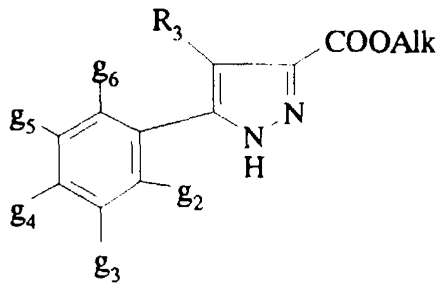

(XXV)

dans laquelle $g_2$, $g_3$, $g_4$, $g_5$, $g_6$ et $R_3$ sont tels que définis pour les composés de formule (I) et Alk représente un groupe méthyle ou éthyle, par une base forte dans un solvant, puis on fait réagir l'anion ainsi obtenu avec un composé de formule :

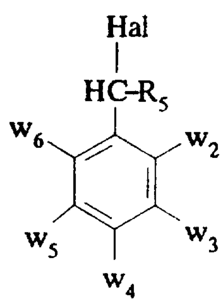

(VII)

dans laquelle $w_2$, $w_3$, $w_4$, $w_5$, $w_6$ et $R_5$ sont tels que définis pour les composés de formule (I), et Hal représente un atome d'halogène, pour obtenir :

- soit, lorsqu'on effectue la réaction dans le toluène à une température comprise entre la température ambiante et la température de reflux du solvant, un composé de formule :

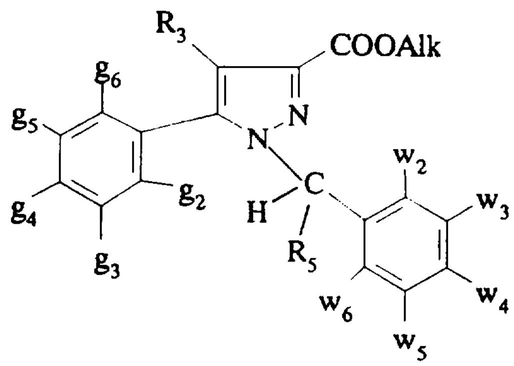

(XXVI)

- soit, lorsqu'on effectue la réaction dans le N,N-diméthylformamide à une température comprise entre 0°C et la température ambiante, un composé de formule :

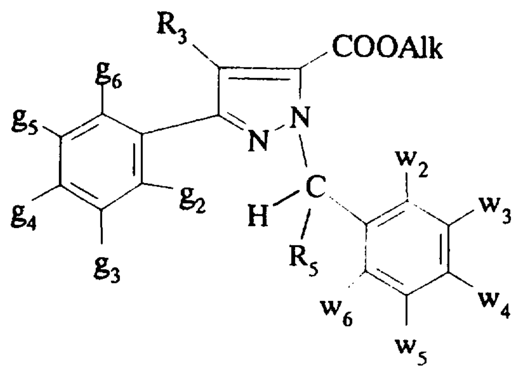

(XXVII)

2) on hydrolyse en milieu alcalin, soit le composé de formule (XXVI), soit le composé de formule (XXVII), pour obtenir respectivement :

- soit, le composé de formule :

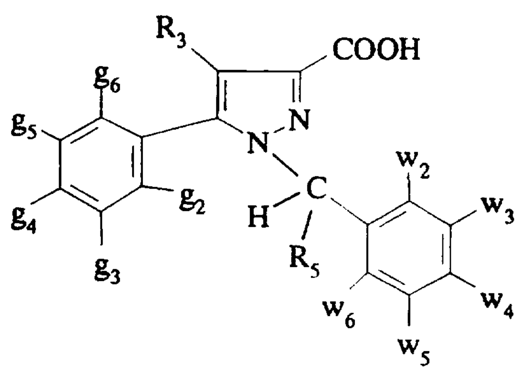

(II : $R_4$ = H)

- soit, le composé de formule :

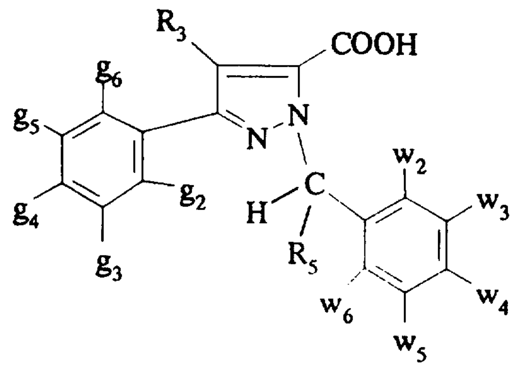

(XII)

Selon un autre de ses aspects, la présente invention concerne un procédé pour la préparation des composés de formule (XIII), et de leurs sels, caractérisé en ce que :

1) on traite un dérivé fonctionnel de l'acide de formule :

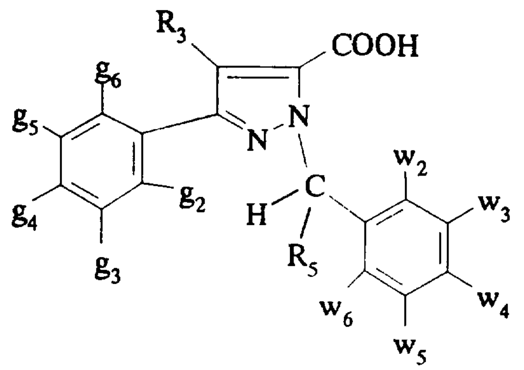

(XII)

dans laquelle $g_2$, $g_3$, $g_4$, $g_5$, $g_6$, $w_2$, $w_3$, $w_4$, $w_5$, $w_6$, $R_3$ et $R_5$ sont tels que définis pour les composés de formule (I), avec un composé de formule :

$$H\text{-}X_1 \qquad (XXIV)$$

dans laquelle $X_1$ est tel que défini pour les composés de formule (I), pour obtenir le composé de formule :

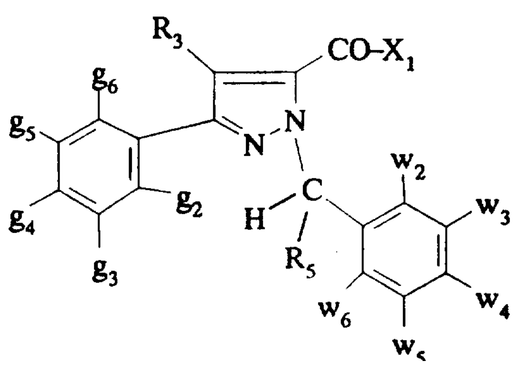

(XIII)

2) et, éventuellement on transforme le composé ainsi obtenu en l'un de ses sels.

Les halogénures de benzyle de formule (VII) sont connus ou préparés par des méthodes connues.

D'une manière générale, les composés de formule (VII) dans laquelle Hal représente un atome de brome

peuvent être préparés par action du N-bromosuccinimide sur les dérivés de méthylbenzène correspondants en présence de peroxyde de dibenzoyle. On peut également préparer un bromure de benzyle à partir d'un alcool benzylique correspondant par action de l'acide bromhydrique en solution dans l'eau ou dans l'acide acétique. On peut aussi utiliser l'action du tribromure de phosphore sur un alcool benzylique correspondant pour préparer un composé de formule (VII) dans laquelle Hal représente un atome de brome.

Les composés de formule (VII) dans laquelle Hal représente un atome d'iode peuvent être préparés par action de l'iodure de sodium sur un composé de formule (VII) dans laquelle Hal représente un atome de chlore dans un solvant tel que l'acétone ou la butan-2-one.

Les composés de formule (VII) dans laquelle Hal représente un atome de chlore peuvent être préparés par action du chlorure de thionyle sur un alcool benzylique correspondant.

D'une manière particulière, on peut préparer les composés de formule (VII) dans laquelle $R_5$ représente un trifluorométhyle et Hal représente un atome de chlore selon la méthode décrite dans J. Fluorine Chem., 1986, 32 (4), 361-366.

On peut également préparer les composés de formule (VII) dans laquelle $R_5$ représente un trifluorométhyle à partir des alcools $\alpha$-(trifluorométhyl)benzyliques correspondants selon les méthodes précédemment décrites. Les alcools $\alpha$-(trifluorométhyl)benzyliques peuvent se préparer selon Tetrahedron, 1989, 45 (5), 1423 ou selon J. Org. Chem., 1991, 56 (1), 2.

Les composés de formule (VIII) sont connus ou préparés par des méthodes connues, telles que celles décrites dans J. Org. Chem., 1988, 53, 1768-1774 ou dans J. Am. Chem. Soc., 1958, 80, 6562-6568.

Les amines de formule $HNR_1R_2$ sont soit disponibles commercialement, soit décrites dans la littérature, soit préparées par des méthodes connues selon les Préparations décrites ci-après :

*endo* et *exo* bicyclo[3.2.1]octan-2-ylamine préparées selon H. Maskill et al., J. Chem. Soc. Perkin II, 1984, 119;

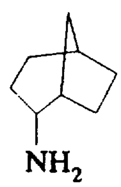 et 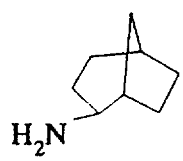

bicyclo[2.2.2]octan-2-ylamine préparée selon R. Seka et al., Ber. 1942, 1379 ;

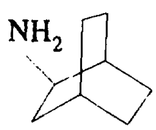

*endo* et *exo* bicyclo[3.2.1]octan-3-ylaminc préparées selon H. Maskill et al., J. Chem. Soc. Perkin Trans II, 1984, 1369 ;

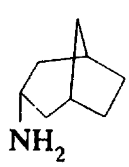 et 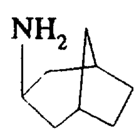

endo tricyclo[5.2.1.0$^{2,6}$]décan-8-ylamine préparée selon G. Buchbauer et al., Arch. Pharm., 1990, 323, 367 ;

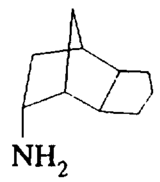

*endo* et *exo,* 1R et 1S 1,3,3-triméthylbicyclo[2.2.1]heptan-2-ylamine préparées selon Ingersoll et al., J. Am. Chem. Soc., 1951, 73, 3360 ou selon J. A. Suchocki et al., J. Med. Chem., 1991, 34, 1003-1010 ;

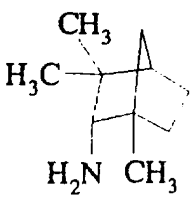

et

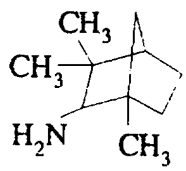

3-méthylcyclohexylamine préparée selon Smith et al., J. Org. Chem., 1952, 17, 294;

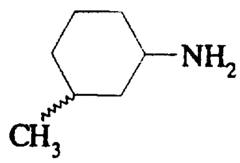

2,6-diméthylcyclohexylamine préparée selon Cornubert et al., Bull. Soc. Chim. Fr., 1945, 12, 367 ;

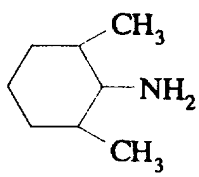

2-méthoxycyclohexylamine préparée selon Noyce et al., J. Am. Chem. Soc., 1954, 76, 768 ;

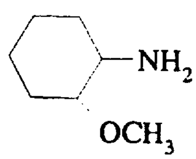

4-éthylcyclohexylamine préparée selon A. Shirahata et al., Biochem. Pharmacol., 1991, 41, 205 ;

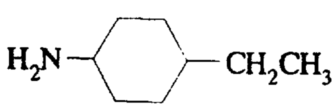

bicyclo[2.2.2]oct-2-èn-5-amine préparée selon H.L. Goering et al., J. Am. Chem. Soc., 1961, 83 1391 ;

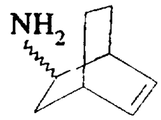

N-éthyl-1-adamantylamine préparée selon V.L. Narayanan et al., J. Med. Chem., 1972, 15, 443 ;

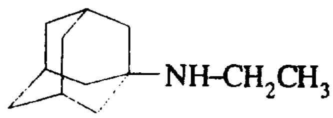

tricyclo[2.2.1.0$^{2,6}$]heptan-3-ylaminc préparée selon G. Muller et al., Chem. Ber., 1965, 98, 1097 ;

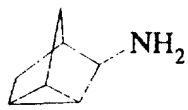

N-méthyl-*exo*-bicyclo[2.2.1]heptan-2-ylamine préparée selon W.G. Kabalka et al., Synth. Commun., 1991, 20, 231 ;

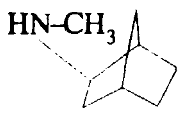

2,2,6,6-tétraméthylcyclohexylamine préparée selon J. Chem. Soc., C, 1970, 1845.

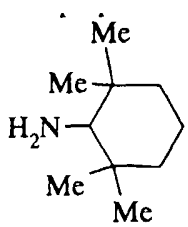

Les alcools de formule HO-$R_2$ sont soit disponibles commercialement, soit décrits dans la littérature, soit préparés par des méthodes connues. Par exemple, on peut effectuer la réduction des cétones correspondantes pour obtenir les alcools de formule (XIV). La réduction s'effectue au moyen d'un agent réducteur tel que le borohydrure de sodium dans un solvant tel que le méthanol ou l'hydrure d'aluminium et de lithium dans un solvant tel que le tétrahydrofurane ou l'éther diéthylique, à une température comprise entre la température ambiante et la température de reflux du solvant.

De façon particulière le 2,2,6,6-tétraméthylcyclohexanol se prépare selon le compte-rendu hebdomadaire des séances de l'Académie des Sciences, 156, 1201.

L'utilisation des composés de formule (III) sous forme énantiomériquement pure à l'étape 1) du procédé A, ou l'utilisation des composés de formule (XIV) sous forme énantiomériquement pure à l'étape 1) du procédé B, et l'utilisation des composés de formule (VII) ou (VIII) sous forme énantiomériquement pure aux étapes $d_1$ et $e_1$ du SCHEMA 1 ou à l'étape $e_3$ du SCHEMA 3 de préparation du composé de formule (II), permettent d'obtenir les composés de formule (I) sous forme énantiomériquement pure.

La résolution des mélanges racémiques des composés de formule (III), (VII), (VIII) ou (XIV) s'effectue selon les méthodes bien connues de l'homme de l'art.

Les composés de formule (I) possèdent une très bonne affinité *in vitro* pour les récepteurs $CB_2$, dans les conditions expérimentales décrites par Bouaboula et al., Eur. J. Biochem., 1993, 214, 173-180.

Plus particulièrement, les composés de la présente invention et leurs sels éventuels sont des ligands puissants et sélectifs des récepteurs $CB_2$, ayant un Ki généralement compris entre 0,1 et 100nM. Ils sont généralement entre 10 et 1000 fois plus actifs sur les récepteurs $CB_2$ que sur les récepteurs $CB_1$, et sont actifs par voie orale.

Les composés (I) selon l'invention sont des antagonistes des récepteurs $CB_2$. L'activité antagoniste de ces composés vis à vis du récepteur $CB_2$ a été déterminée dans différents modèles. Il est connu que les agonistes des récepteurs aux cannabinoïdes ($\Delta^9$-THC, WIN 55212-2 ou CP 55940) sont capables d'inhiber l'activité de l'adénylate cyclase induite par de la Forskoline comme décrit par M. Rinaldi-Carmona et al., Journal of Pharmacology and Experimental Therapeutics, 1996, 278, 871-878. Dans ce modèle les composés (I) selon l'invention sont capables de bloquer totalement l'effet des agonistes des récepteurs aux cannabinoïdes.

D'autre part, il est connu qu'à des concentrations nanomolaires, les agonistes des récepteurs aux cannabinoïdes (WIN 55212-2 ou CP-55940) sont capables d'augmenter le taux de synthèse d'ADN de cellules B humaines costimulées avec des anticorps anti-Ig : augmentation d'environ 40 % de l'incorporation de la thymidine (J.M. Derocq et al., FEBS Letters, 1995, 369, 177-182). Lorsque l'on utilise les composés (I) selon l'invention ou un de leurs sels éventuels dans un large domaine de concentration, de $10^{-10}$M à $10^{-5}$M, on observe que ceux-ci bloquent l'augmentation du taux de synthèse d'ADN de cellules B humaines (stimulées comme précédemment décrit) induite par les agonistes des récepteurs aux cannabinoïdes (WIN 55212-2 ou CP 55940).

Par ailleurs, les agonistes des récepteurs aux cannabinoïdes (CP 55940 ou WIN 55212-2) induisent l'activation des protéines kinases activées par les mitogènes (MAPKs: "mitogen activated protein kinases") dans les cellules exprimant le récepteur $CB_2$. Les composés (I) selon l'invention bloquent spécifiquement cette activation des MAPKs induite par les agonistes des récepteurs aux cannabinoïdes (CP 55940 ou WIN 55212-2).

Les composés selon l'invention ou leurs sels éventuels possèdent également une affinité *in vivo* pour les récepteurs aux cannabinoïdes $CB_2$ présents au niveau de la rate de souris lorsqu'il sont administrés par voie intraveineuse, intrapéritonéale ou orale. Leur activité a été mise en évidence par des expériences de liaison ex vivo du [$^3$H]-CP 55940. Les essais ont été réalisés selon les conditions expérimentales décrites par M. Rinaldi-Carmona et al., Life Sciences, 1995, 56, 1941-1947.

Les composés de la présente invention ont une toxicité compatible avec leur utilisation en tant que médicaments.

Grâce à leurs remarquables propriétés, notamment à leur grande affinité et à leur sélectivité pour le récepteur périphérique $CB_2$, les composés de formule (I), tels quels ou sous forme de sels pharmaceutiquement acceptables peuvent être utilisés comme principes actifs de médicaments.

Les maladies pour le traitement desquelles les composés (I) et, éventuellement leurs sels pharmaceutiquement acceptables peuvent être utilisés, sont les pathologies impliquant les cellules du système immunitaire ou les désordres immunitaires, par exemple les maladies autoimmunes, les maladies associées aux transplantations d'organes, les maladies infectieuses, les maladies allergiques, les maladies du système gastrointestinal par exemple la maladie de Crohn. Plus particulièrement on peut citer, les maladies autoimmunes suivantes : lupus érythémateux disséminé, les maladies du tissu conjonctif ou connectivites, le syndrome de Sjôgren's, la spondylarthrite ankylosante, l'arthrite réactive, la polyarthrite rhumatoïde, la spondylarthrite indifférenciée, la maladie de Behcet, les anémies autoimmunes hémolytiques, la sclérose en plaques, le psoriasis. Les maladies allergiques à traiter peuvent être du type hypersensibilité immédiate ou asthme, par exemple. De même les composés et leurs sels éventuels pharmaceutiquement acceptables peuvent être utilisés pour traiter les vascularites, les infections parasitaires, les infections virales, les infections bactériennes, l'amylose, les maladies affectant les lignées du système lymphohématopoïetique.

Ainsi, selon un autre de ses aspects, la présente invention concerne une méthode de traitement des maladies ci-dessus qui consiste à administrer à un patient en ayant besoin une quantité efficace d'un composé de formule (I) ou d'un de ses sels pharmaceutiquement acceptables.

Selon un autre de ses aspects, la présente invention concerne également l'utilisation des composés de formule (I) pour la préparation de médicaments destinés à traiter des troubles en relation avec les récepteurs aux cannabinoïdes $CB_2$, plus particulièrement les désordres immunitaires et également les maladies dans lesquelles le système immunitaire est impliqué.

De plus, les composés (I) ou (XIII) selon l'invention, tels quels ou sous forme radiomarquée peuvent être utilisés comme outils pharmacologiques chez l'homme ou chez l'animal, pour la détection et le marquage des récepteurs périphériques $CB_2$ aux cannabinoïdes. Cela constitue un aspect ultérieur de la présente invention.

Les composés de la présente invention sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou un de ses sels pharmaceutiquement acceptables.

Les composés de formule (I) ci-dessus et leurs sels pharmaceutiquement acceptables penvent être utilisés à des doses journalières de 0,01 à 100 mg par kilo de poids corporel du mammifère à traiter, de préférence à des

doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale ou intraoculaire et les formes d'administration rectale.

Lorsque l'on prépare une composition solide sous forme de comprimés, on peut ajouter au principe actif, micronisé ou non, un agent mouillant tel que le laurylsulfate de sodium et on mélange le tout avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le polyéthylèneglycol.

Ainsi, pour préparer une solution aqueuse injectable par voie intraveineuse on peut utiliser un cosolvant : un alcool tel que l'éthanol, un glycol tel que le polyéthylèneglycol ou le propylèneglycol et un tensioactif hydrophile tel que le Tween® 80. Pour préparer une solution huileuse injectable par voie intramusculaire, on peut solubiliser le principe actif par un triglycéride ou un ester de glycérol.

Pour l'administration locale on peut utiliser des crèmes, des pommades, des gels.

Pour l'administration transdermique, on peut utiliser des patches sous forme multilaminée ou à réservoir dans lesquels le principe actif peut être en solution alcoolique.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif seul ou associé à un excipient, sous forme de poudre.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple $\alpha$-, $\beta$- ou $\gamma$- cyclodextrine, 2-hydroxypropyl-$\beta$-cyclodextrine ou méthyl-$\beta$-cyclodextrine.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Les exemples suivants illustrent l'invention sans toutefois la limiter.

Les points de fusion ou de décomposition des produits, F, sont mesurés en tube capillaire avec un appareil de Tottoli.

Les spectres de RMN du proton sont enregistrés à 200 MHz dans le DMSO-$d_6$.

L'appareil utilisé en HPLC préparative est un modèle Prochrom LC 50 avec un diamètre de colonne de 50 mm et une hauteur de lit maximale comprise entre 35 et 40 cm.

Les conditions utilisées sont :

* Phase stationnaire : Kromasil C 18-100 Å 10 µ
* Phase mobile
  : Eluant A : $H_2O$
  : Eluant B : MeOH/$H_2O$ (90/10 ; v/v).
* Débit : 114 ml/mn ; Position des pompes 8 mm.
* Gradient d'élution :

| Temps (mn) | % A | % B |
|---|---|---|
| 0 | 10 | 90 |
| 5 | **10** | **90** |
| **80** | **4** | **96** |

* Détection UV à λ = 230 nm
  Longueur de la cellule 0
  Atténuation = 0,5 AUFS

L'appareil utilisé en HPLC analytique est une chaîne HPLC Hewlett Packard. Les conditions utilisées sont :

* Colonne : phase stationnaire Kromasil (Waters)
  C 18-100 Å 10 µm
* Phase mobile :
  Eluant A : $H_2O$
  Eluant B : MeOH
* Gradient d'élution :

| Temps (mn) | % A | % B |
|---|---|---|
| 0 | 20 | 80 |
| 5 | 20 | 80 |
| 50 | 7 | 93 |

* Débit : 1 ml/mn
* Détection : UVλ = 230 nm, atténuation = 8
* Volume injecté : 30 µl.

Dans les Préparations et dans les EXEMPLES, les abréviations suivantes sont utilisées :

Me, OMe : méthyle, méthoxy
Et, OEt : éthyle, éthoxy
EtOH : éthanol
MeOH : méthanol
Ether : éther diéthylique
Ether iso : éther diisopropylique
DMF : diméthylformamide
DMSO : diméthylsulfoxyde
DCM : dichlorométhane
$CCl_4$ : tétrachlorure de carbone
THF : tétrahydrofurane
AcOEt : acétate d'éthyle
$K_2CO_3$ : carbonate de potassium
$Na_2CO_3$ : carbonate de sodium
$KHCO_3$ : hydrogénocarbonate de potassium
$NaHCO_3$ : hydrogénocarbonate de sodium
NaCl : chlorure de sodium
$Na_2SO_4$ : sulfate de sodium

$MgSO_4$ : sulfate de magnésium
NaOH : soude
KOH : potasse
AcOH : acide acétique
$H_2SO_4$ : acide sulfurique
HCl : acide chlorhydrique
HBr : acide bromhydrique
éther chlorhydrique : solution saturée d'acide chlorhydrique dans l'éther
BOP : benzotriazol-1-yloxytris(diméthylamino) phosphonium hexafluorophosphate
DBU : 1,8-diazabicyclo[5.4.0]undec-7-ène
$NH_4Cl$ : chlorure d'ammonium
F : point de fusion
Eb : température d'ébullition
TA : température ambiante
silice H : gel de silice 60H commercialisé par Merck (DARMSTADT)
HPLC : chromatographie liquide haute performance
TR : temps de rétention
RMN : résonnance magnétique nucléaire
δ : déplacement chimique exprimé en partie par million
s : singulet ; se : singulet élargi ; sd : singulet dédoublé ; d : doublet ;
dd : doublet de doublet ; t : triplet ; qd : quadruplet ; sept : septuplet ;
mt : multiplet ; m : massif.

PREPARATIONS

Préparation 1.1

Acide 1-(3,4-dichlorobenzyl)-5-(4-méthylphényl)pyrazole-3-carboxylique.

A) Sel de sodium du 4-(4-méthylphényl)-2-oxo-4-oxydobut-3-énoate de méthyle.

On dissout 6,24 g de sodium dans 150 ml de MeOH. Après refroidissement à TA, on ajoute 36,4 ml de 4'-méthylacétophénone puis une solution de 37 ml d'oxalate de diéthyle dans 50 ml de MeOH. On ajoute ensuite 100 ml de MeOH pour fluidifier le mélange réactionnel et laisse 2 heures sous agitation à TA. On ajoute 500 ml d'éther et laisse 30 minutes sous agitation à TA. On essore le précipité formé, le lave à l'éther et le sèche. On obtient 57,4 g du produit attendu.

B) Ester méthylique de l'acide 5-(4-méthylphényl)pyrazole-3-carboxylique.

On refroidit au bain de glace un mélange de 30 g du composé obtenu à l'étape précédente dans 100 ml d'AcOH, et ajoute goutte à goutte 7,94 ml d'une solution à 55 % d'hydrazine dans l'eau. Puis on chauffe à reflux le mélange réactionnel pendant 5 heures et laisse une nuit sous agitation à TA. On essore le précipité formé, le lave à l'eau et obtient ainsi un premier jet. On verse le filtrat dans un mélange eau/glace, essore le précipité formé, le lave à l'eau, le sèche et obtient un deuxième jet. On rassemble le premier jet et le deuxième jet, lave à l'AcOEt et sèche sous vide. On obtient 21,44 g du produit attendu.

C) Ester méthylique de l'acide 1-(3,4-dichlorobenzyl)-5-(4-méthylphényl)pyrazole-3-carboxylique.

A une suspension de 5 g du composé obtenu à l'étape précédente dans 50 ml de toluène, on ajoute à TA et par portions 2,03 g d'hydrure de sodium à 60 % dans l'huile, puis chauffe le mélange réactionnel à 65°C pendant 1 heure. Après refroidissement à TA, on ajoute goutte à goutte 5,82 g de bromure de 3,4-dichlorobenzyle puis chauffe à reflux pendant 20 heures. On refroidit le mélange réactionnel à TA, et ajoute, goutte à goutte, 100 ml d'une solution à 50 % de $NH_4Cl$ dans l'eau. Après décantation, on lave la phase organique par une solution saturée de NaCl, sèche sur $MgSO_4$ et évapore sous vide le solvant. On chromatographie le résidu sur silice en éluant par le mélange cyclohexane/AcOEt (75/25 ; v/v). On obtient 5,28 g du produit attendu, F = 98,6°C.

D) Acide 1-(3,4-dichlorobenzyl)-5-(4-méthylphényl)pyrazole-3-carboxylique.

A une solution de 5,2 g du composé obtenu à l'étape précédente dans 100 ml d'EtOH on ajoute à TA une solution de 1,16 g de KOH dans 20 ml d'eau puis chauffe à reflux pendant 5 heures. On laisse le mélange réactionnel une nuit sous agitation à TA, ajoute 200 ml d'une solution aqueuse d'HCl N, essore le précipité formé et le sèche sous vide. On obtient 5,12 g du produit attendu, F = 171,5°C.

RMN : δ (ppm) : 2,4 : s : 3H ; 5,5 : s : 2H ; 6,9 : s : 1H ; 7,0 : dd : 1H 7,3 : d : 1H ;7,35 : système AA'-BB' : 4H ;

7,65 : d : 1H; 13,0 : se : 1H

On observe un effet Overhauser (N.O.E) entre les protons benzyliques ($R_4 = R_5 = H$) et les protons $g_2 = g_6 = H$.

Préparation 1.2

Acide 1-(3-chloro-4-méthylbenzyl)-5-(4-méthylphényl)pyrazole-3-carboxylique.

A) Ester méthylique de l'acide 1-(3-chloro-4-méthylbenzyl)-5-(4-méthylphényl) pyrazole-3-carboxylique.

On prépare ce composé selon le mode opératoire décrit à l'étape C de la Préparation 1.1 à partir de 5 g du composé obtenu à l'étape B de la Préparation 1.1, 2,03 g d'hydrure de sodium à 60 % dans l'huile, 100 ml de toluène et 4,22 g de chlorure de 3-chloro-4-méthylbenzyle. On obtient 1,52 g du produit attendu.

B) Acide 1-(3-chloro-4-méthylbenzyl)-5-(4-méthylphényl)pyrazole-3-carboxylique.

A une solution de 1,52 g du composé obtenu à l'étape précédente dans 50 ml d'EtOH, on ajoute à TA une solution de 0,36 g de KOH dans 10 ml d'eau puis chauffe à reflux pendant une nuit. On concentre sous vide le mélange réactionnel, reprend le résidu à l'eau, lave la phase aqueuse à l'éther, acidifie la phase aqueuse à pH = 2 par ajout d'une solution d'HCl 6N, essore le précipité formé, le lave à l'eau et le sèche sous vide. On obtient 1,42 g du produit attendu, F = 108,5°C.

RMN : δ (ppm) : 2,1 à 2,45 : 2s : 6H ; 5,4 : s : 2H ; 6,7 à 7,5 : m : 8H ; 12,85 : se : 1H.

Préparation 1.3

Acide 1-(3-fluoro-4-méthylbenzyl)-5-(4-méthylphényl) pyrazole-3-carboxylique et acide 1-(3-fluoro-4-méthylbenzyl)-3-(4-méthylphényl) pyrazole-5-carboxylique.

A) Ester méthylique de l'acide 1-(3-fluoro-4-méthylbenzyl)-5-(4-méthylphényl) pyrazole-3-carboxylique et Ester méthylique de l'acide 1-(3-fluoro-4-méthylbenzyl)-3-(4-méthylphényl) pyrazole-5-carboxylique.

A une suspension de 2,5 g du composé obtenu à l'étape B de la Préparation 1.1 dans 100 ml de toluène on ajoute à TA et par portions 1,01 g d'hydrure de sodium à 60 % dans l'huile, puis chauffe le mélange réactionnel à 65°C pendant 1 heure. Après refroidissement à TA, on ajoute goutte à goutte 2,45 g de bromure de 3-fluoro-4-méthylbenzyle (Préparation 3.1) puis chauffe à reflux pendant 48 heures. Après refroidissement à TA, on ajoute goutte à goutte 50 ml d'une solution à 50 % de $NH_4Cl$ dans l'eau. Après décantation, on lave la phase organique par une solution saturée de NaCl, sèche sur $MgSO_4$ et évapore sous vide le solvant. On chromatographie le résidu sur silice en éluant par le mélange AcOEt/cyclohexane (50/50 ; v/v). On obtient 1,29 g du mélange des produits attendus.

B) Acide 1-(3-fluoro-4-méthylbenzyl)-5-(4-méthylphényl)pyrazole-3-carboxylique et acide 1-(3-fluoro-4-méthylbenzyl)-3-(4-méthylphényl)pyrazole-5-carboxylique.

A une solution de 1,2 g du mélange des composés obtenus à l'étape précédente dans 50 ml d'EtOH, on ajoute à TA une solution de 0,3 g de KOH dans 10 ml d'eau puis chauffe à reflux pendant 5 heures et laisse une nuit sous agitation à TA. On concentre sous vide le mélange réactionnel, reprend le résidu à l'eau, lave la phase aqueuse à l'AcOEt, acidifie la phase aqueuse à pH = 2 par ajout d'une solution d'HCl 6N, essore le précipité formé, le lave à l'eau et le sèche sous vide. On obtient 0,95 g du mélange des produits attendus.

Préparation 1.4

Acide 1-(3,4-dichlorobenzyl)-5-(4-méthoxyphényl)pyrazole-3-carboxylique.

A) Sel de sodium du 4-(4-méthoxyphényl)-2-oxo-4-oxydobut-3-énoate de méthyle.

On dissout 3,9 g de sodium dans 100 ml de MeOH. Après refroidissement à TA, on ajoute 25,4 g de 4'-méthoxyacétophénone puis une solution de 23 ml d'oxalate de diéthyle dans 50 ml de MeOH et laisse 2 heures sous agitation à TA. On ajoute 500 ml d'éther et laisse 30 minutes sous agitation à TA. On essore le précipité formé, le lave à l'éther et le sèche. On obtient 33,4 g du produit attendu.

B) Ester méthylique de l'acide 5-(4-méthoxyphényl)pyrazole-3-carboxylique.

On refroidit au bain de glace un mélange de 9 g du composé obtenu à l'étape précédente dans 100 ml d'AcOH et ajoute goutte à goutte 2,2 ml d'hydrazine monohydrate. Puis on chauffe à reflux le mélange réactionnel pendant 5 heures et laisse une nuit sous agitation à TA. On verse le mélange réactionnel sur un mélange eau/glace, essore le précipité formé et le lave à l'eau. On reprend le précipité au DCM, filtre un insoluble, sèche le filtrat sur $MgSO_4$ et évapore sous vide le solvant On obtient 7,1 g du produit attendu.

C) Ester méthylique de l'acide 1-(3,4-dichlorobenzyl)-5-(4-méthoxyphényl)pyrazole-3-carboxylique.

On prépare ce composé selon le mode opératoire décrit à l'étape C de la Préparation 1.1 à partir de 3,5 g du composé obtenu à l'étape précédente, 1,32 g d'hydrure de sodium à 60 % dans l'huile, 150 ml de toluène et 3,77 g de bromure de 3,4-dichlorobenzyle. On chromatographie le produit sur silice en éluant par le mélange cyclohexane/AcOEt (60/40; v/v). On obtient 2,4 g du produit attendu, F = 95,5°C.

RMN : $\delta$ (ppm) : 3,8 : 2s : 6H ; 5,45 : s : 2H ; 6,8 à 7,1 : m : 4H ; 7,25 : d : 1H ; 7,35 : d : 2H ; 7,55 : d : 1H.

D) Acide 1-(3,4-dichlorobenzyl)-5-(4-méthoxyphényl)pyrazole-3-carboxylique.

On prépare ce composé selon le mode opératoire décrit à l'étape B de la Préparation 1.2 à partir de 2,4 g du composé obtenu à l'étape précédente, 50 ml d'EtOH, 0,51 g de KOH et 10 ml d'eau. On obtient 2,23 g du produit attendu.

Préparation 1.5

Acide 1-(3-chloro-4-méthylbenzyl)-5-(4-méthoxyphényl)pyrazole-3-carboxylique et Acide 1-(3-chloro-4-méthylbenzyl)-3-(4-méthoxyphényl)pyrazole-5-carboxylique

A) Ester méthylique de l'acide 1-(3-chloro-4-méthylbenzyl)-5-(4-méthoxyphényl) pyrazole-3-carboxylique et Ester méthylique de l'acide 1-(3-chloro-4-méthylbenzyl)-3-(4-méthoxyphényl) pyrazole-5-carboxylique

On prépare le mélange de ces deux composés selon le mode opératoire décrit à l'étape A de la Préparation 1.3 à partir de 3,5 g du composé obtenu à l'étape B de la Préparation 1.4, 1,32 g d'hydrure de sodium à 60 % dans l'huile, 100 ml de toluène et 2,75 g de chlorure de 3-chloro-4-méthylbenzyle. On obtient 1,93 g du mélange des produits attendus.

B) Acide 1-(3-chloro-4-méthylbenzyl)-5-(4-méthoxyphényl)pyrazole-3-carboxylique et Acide 1-(3-chloro-4-méthylbenzyl)-3-(4-méthoxyphényl)pyrazole-5-carboxylique

On prépare le mélange de ces deux composés selon le mode opératoire décrit à l'étape B de la Préparation 1.3 à partir de 1,9 g du mélange des composés obtenus à l'étape précédente, 50 ml d'EtOH, 0,43 g de KOH et 10 ml d'eau. On obtient 1,73 g du mélange des produits attendus.

Préparation 1.6

Acide 1-(3-chloro-4-méthylbenzyl)-5-(4-fluorophényl)pyrazole-3-carboxylique.

A) Sel de sodium du 4-(4-fluorophényl)-2-oxo-4-oxydobut-3-énoate de méthyle.

On prépare ce composé selon le mode opératoire décrit à l'étape A de la Préparation 1.1 à partir de 4,16 g de sodium dans 100 ml de MeOH, 21,87 ml de 4'-fluoroacétophénone et 24,72 ml d'oxalate de diéthyle dans 50 ml de MeOH. On obtient 42,68 g du produit attendu.

B) Ester méthylique de l'acide 5-(4-fluorophényl)pyrazole-3-carboxylique.

On prépare ce composé selon le mode opératoire décrit à l'étape B de la Préparation 1.1 à partir de 15 g du composé obtenu à l'étape précédente, 100 ml d'AcOH, et 3,73 ml d'une solution à 55 % d'hydrazine dans l'eau. Après une nuit sous agitation à TA, on verse le mélange réactionnel dans un mélange eau/glace, essore le précipité formé, le lave à l'eau et le sèche. On obtient 10,74 g du produit attendu.

C) Ester méthylique de l'acide 1-(3-chloro-4-méthylbenzyl)-5-(4-fluorophényl) pyrazole-3-carboxylique.

On prépare ce composé selon le mode opératoire décrit à l'étape C de la Préparation 1.1 à partir de 3,5 g du composé obtenu à l'étape précédente, 1,4 g d'hydrure de sodium à 60 % dans l'huile, 100 ml de toluène et 4,45 g d'iodure de 3-chloro-4-méthylbenzyle (Préparation 3.2) dans 50 ml de toluène. On chromatographie sur silice en éluant par le mélange cyclohexane/AcOEt (70/30 ; v/v). On obtient 1,57 g du produit attendu, F = 110°C.

D) Acide 1-(3-chloro-4-méthylbenzyl)-5-(4-fluorophényl)pyrazole-3-carboxylique.

On prépare ce composé selon le mode opératoire décrit à l'étape B de la Préparation 1.2 à partir de 1,55 g

du composé obtenu à l'étape précédente, 50 ml d'EtOH, 0,36 g de KOH et 10 ml d'eau. On obtient 1,46 g du produit attendu, F = 120°C.

Préparation 1.7

Acide 1-(2,4-dichlorobenzyl)-5-(4-chlorophényl)pyrazole-3-carboxylique.

A) Sel de sodium du 4-(4-chlorophényl)-2-oxo-4-oxydobut-3-énoate de méthyle.

On prépare ce composé selon le mode opératoire décrit à l'étape A de la Préparation 1.1 à partir de 12,66 g de sodium dans 270 ml de MeOH, 68,4 ml de 4'-chloroacétophénone et 71,8 ml d'oxalate de diéthyle dans 110 ml de MeOH. On obtient 97 g du produit attendu.

B) Ester méthylique de l'acide 5-(4-chlorophényl)pyrazole-3-carboxylique.

On prépare ce composé selon le mode opératoire décrit à l'étape B de la Préparation 1.4 à partir de 15 g du composé obtenu à l'étape précédente, 65 ml d'AcOH, 3,05 ml d'hydrazine monohydrate. On triture le précipité obtenu dans un mélange de 100 ml de DCM et 50 ml d'AcOEt, l'essore et le sèche. On obtient 8,13 g du produit attendu, F = 215°C.

C) Ester méthylique de l'acide 1-(2,4-dichlorobenzyl)-5-(4-chlorophényl)pyrazole-3-carboxylique.

A une suspension de 1,02 g d'hydrure de sodium à 60 % dans l'huile, dans 100 ml de toluène, on ajoute à TA et goutte à goutte une solution de 5,07 g du composé obtenu à l'étape précédente dans 100 ml de toluène, puis chauffe à 65°C pendant 1 heure. On ajoute ensuite 3,12 ml de chlorure de 2,4-dichlorobenzyle et chauffe à reflux pendant 20 heures. On refroidit le mélange réactionnel à TA, ajoute 100 ml d'une solution à 50 % de $NH_4Cl$ dans l'eau. Après décantation on lave la phase organique par une solution saturée de NaCl, sèche sur $MgSO_4$ et évapore sous vide le solvant. On chromatographie le résidu sur silice en éluant par le mélange toluène/DCM/AcOEt (80/10/10 ; v/v/v). On obtient 3,68 g du produit attendu, F = 105°C.

D) Acide 1-(2,4-dichlorobenzyl)-5-(4-chlorophényl)pyrazole-3-carboxylique.

On prépare ce composé selon le mode opératoire décrit à l'étape D de la Préparation 1.1 à partir de 3,6 g du composé obtenu à l'étape précédente, 60 ml de MeOH, 1,27 g de KOH et 6 ml d'eau. On obtient 3,52 g du produit attendu, F= 185°C.

Préparation 1.8

Acide 1-(3-chloro-4-méthylbenzyl)-5-(3,4-diméthylphényl)pyrazole-3-carboxylique.

A) Sel de sodium du 4-(3,4-diméthylphényl)-2-oxo-4-oxydobut-3-énoate de méthyle.

On prépare ce composé selon le mode opératoire décrit à l'étape A de la Préparation 1.1 à partir de 3,9 g de sodium dans 100 ml de MeOH, 25 g de 3',4'-diméthylacétophénone et 23 ml d'oxalate de diéthyle dans 50 ml de MeOH. On obtient 39,42 g du produit attendu.

B) Ester méthylique de l'acide 5-(3,4-diméthylphényl)pyrazole-3-carboxylique.

On prépare ce composé selon le mode opératoire décrit à l'étape B de la Préparation 1.6 à partir de 10 g du composé obtenu à l'étape précédente dans 150 ml d'AcOH et 2,2 ml d'une solution à 55 % d'hydrazine dans l'eau. On obtient 9 g du produit attendu.

C) Ester méthylique de l'acide 1-(3-chloro-4-méthylbenzyl)-5-(3,4-diméthylphényl) pyrazole-3-carboxylique.

On prépare ce composé selon le mode opératoire décrit à l'étape C de la Préparation 1.1 à partir de 1,52 g d'hydrure de sodium à 60 % dans l'huile, 4 g du composé obtenu à l'étape précédente, 70 ml de toluène et 5,8 g d'iodure de 3-chloro-4-méthylbenzyle (Préparation 3.2). On purifie le produit obtenu par trituration dans l'hexane puis essorage et lavage à l'hexane. On obtient 2,84 g du produit attendu, F = 88°C.

RMN : δ (ppm) : 2,0 à 2,35 : m : 9H ; 3,95 : s : 3H ; 5,3 : s : 2H ; 6,6 à 7,4 : m : 7H.

D) Acide 1-(3-chloro-4-méthylbenzyl)-5-(3,4-diméthylphényl)pyrazole-3-carboxylique.

On prépare ce composé selon le mode opératoire décrit à l'étape B de la Préparation 1.2 à partir de 1,8 g du composé obtenu à l'étape précédente dans 30 ml d'EtOH et 0,392 g de KOH dans 30 ml d'eau. On obtient 1,6 g du produit attendu, F = 163°C.

Préparation 1.9

Acide 1-(3-chloro-4-fluorobenzyl)-5-(3,4-diméthylphényl)pyrazole-3-carboxylique.

A) Ester méthylique de l'acide 1-(3-chloro-4-fluorobenzyl)-5-(3,4-diméthylphényl) pyrazole-3-carboxylique

On prépare ce composé selon le mode opératoire décrit à l'étape C de la Préparation 1.1 à partir de 2,5 g du composé obtenu à l'étape B de la Préparation 1.8, 50 ml de toluène, 0,88 g d'hydrure de sodium à 60 % dans l'huile et 2,5 g de bromure de 3-chloro-4-fluorobenzyle (Préparation 3.3). On purifie le produit par trituration dans l'AcOEt puis essorage et séchage. On obtient 3,6 g du produit attendu.

B) Acide 1 -(3-chloro-4-fluorobenzyl)-5-(3,4-diméthylphényl)pyrazole-3-carboxylique.

On prépare ce composé selon le mode opératoire décrit à l'étape B de la Préparation 1.2 à partir de 1,6 g du composé obtenu à l'étape précédente dans 25 ml d'EtOH et 0,481 g de KOH dans 10 ml d'eau. On obtient 1,22 g du produit attendu.

RMN : δ (ppm) : 2,2 : 2s : 6H ; 5,35 : s : 2H ; 6,6 à 7,4 : m : 7H.

Préparation 1.10

Acide 1-(4-méthylbenzyl)-5-(4-chloro-3-méthylphényl)pyrazole-3-carboxylique.

A) Sel de sodium du 4-(4-chloro-3-méthylphényl)-2-oxo-4-oxydo-but-3-énoate de méthyle.

On prépare ce composé selon le mode opératoire décrit à l'étape A de la Préparation 1.1, à partir de 7,6 g de sodium dans 100 ml de MeOH, 55,6 g de 4'-chloro-3'-méthylacétophénone et 45 ml d'oxalate de diéthyle dans 100 ml de MeOH. On obtient 85,8 g du produit attendu.

B) Ester méthylique de l'acide 5-(4-chloro-3-méthylphényl)pyrazole-3-carboxylique.

On prépare ce composé selon le mode opératoire décrit à l'étape B de la Préparation 1.4, à partir de 15 g du composé obtenu à l'étape précédente dans 150 ml d'AcOH et 2,9 ml d'hydrazine monohydrate. Après une nuit sous agitation à TA, on verse le mélange réactionnel dans de l'eau glacée, essore le précipité formé et le lave à l'eau. On obtient 13 g du produit attendu après séchage.

C) Ester méthylique de l'acide 1-(4-méthylbenzyl)-5-(4-chloro-3-méthylphényl) pyrazole-3-carboxylique.

On prépare ce composé selon le mode opératoire décrit à l'étape C de la Préparation 1.1 à partir de 5 g du composé obtenu à l'étape précédente dans 50 ml de toluène, 1,8 g d'hydrure de sodium à 60 % dans l'huile et 4,07 g de bromure de 4-méthylbenzyle. On obtient 4,6 g du produit attendu, F = 98°C.

RMN : δ (ppm) : 2,0 à 2,4 : 2s : 6H ; 3,8 : s : 3H ; 5,4 : s : 2H ; 6,7 à 7,6 : m : 8H

D) Acide 1-(4-méthylbenzyl)-5-(4-chloro-3-méthylphényl)pyrazole-3-carboxylique.

On prépare ce composé selon le mode opératoire décrit à l'étape B de la Préparation 1.2, à partir de 4 g du composé obtenu à l'étape précédente dans 100 ml d'EtOH et 0,95 g de KOH dans 20 ml d'eau. On obtient 3,4 g du produit attendu, F = 180°C.

RMN : δ (ppm) : 2,25 : s : 3H ; 2,35 : s : 3H ; 5,4 : s : 2H ; 6,7 à 7,15 : m : 5H ; 7,25 : dd : 1H ; 7,4 à 7,6 : m : 2H.

En procédant selon les modes opératoires décrits à l'étape C (à partir du composé obtenu à l'étape B de la Préparation 1.10 et des halogénures de benzyle appropriés), puis à l'étape D de la Préparation 1.10, on prépare les esters puis les acides décrits dans le TABLEAU 1 ci-dessous.

TABLEAU 1

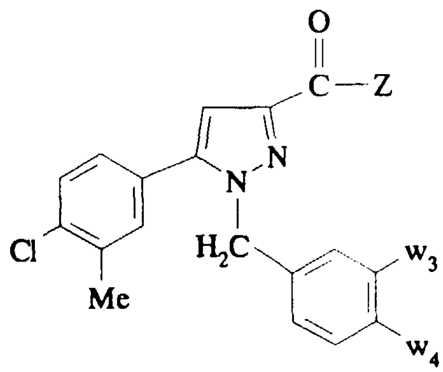

Z = OMe, OH

| Préparations | $w_3$ | $w_4$ |
|---|---|---|
| 1.11 | H | F |
| 1.12 | Cl | Me |
| 1.13 | Cl | F |

Préparation 1.11 : Z = OMe : RMN : δ (ppm) : 2,35 : s : 3H ; 3,85 : s : 3H ; 5,5 : s : 2H ; 6,8 à 7,6 : m : 8H

Préparation 1.11 : Z = OMe : RMN : δ (ppm) : 2,35 : s : 3H ; 3,85 : s : 3H ; 5,5 : s : 2H ; 6,8 à 7,6 : m : 8H

Préparation 1.14

Acide 1-[1-(2,4-dichlorophényl)éthyl]-5-(4-chlorophényl)pyrazole-3-carboxylique.

A) Ester méthylique de l'acide 1-[1-(2,4-dichlorophényl)éthyl]-5-(4-chlorophényl) pyrazole-3-carboxylique.

A une suspension de 1,5 g du composé obtenu à l'étape B de la Préparation 1.7 dans 50 ml de toluène on ajoute à TA et par portions 0,3 g d'hydrure de sodium à 60 % dans l'huile, puis chauffe à 65°C pendant 30 minutes. Après refroidissement à TA, on ajoute goutte à goutte 1,77 g de 1-(1-bromoéthyl)-2,4-dichlorobenzène (Préparation 3.4) puis chauffe à reflux pendant 5 jours. Après refroidissement à TA, on verse le mélange réactionnel sur 100 ml d'une solution à 50 % de $NH_4Cl$ dans l'eau refroidie à 0°C. On extrait à l'AcOEt, lave la phase organique à l'eau, par une solution saturée de NaCl, sèche sur $MgSO_4$ et évapore sous vide le solvant. On chromatographie le résidu sur silice en éluant par le mélange toluène/AcOEt (95/5 ; v/v). On obtient 1,02 g du produit attendu.

B) Acide 1-[1-(2,4-dichlorophényl)éthyl]-5-(4-chlorophényl)-pyrazole-3-carboxylique.

A une solution de 1,02 g du composé obtenu à l'étape précédente dans 20 ml de MeOH, on ajoute à TA une solution de 0,35 g de KOH dans 5 ml d'eau, puis chauffe à reflux pendant 2 heures. On verse le mélange réactionnel dans 100 ml d'une solution d'HCl à 5 % refroidie à 0°C, essore le précipité formé, le lave à l'eau et le sèche sous vide. On obtient 0,74 g du produit attendu, F = 80°C.

Préparation 1.15

Acide 1-(3,4-dichlorobenzyl)-5-(2,6-diméthoxyphényl)pyrazole-3-carboxylique.

A) Sel de potassium du 4-(2,6-diméthoxyphényl)-2-oxo-4-oxydobut-3-énoate d'éthyle.

On chauffe à 50°C un mélange de 18 g de 2',6'-diméthoxyacétophénone dans 54 ml d'EtOH et ajoute en 5

minutes une solution de 13,4 g de *tert*-butylate de potassium dans 72 ml d'EtOH. On chauffe à reflux le mélange réactionnel, ajoute en 10 minutes 16,3 ml d'oxalate de diéthyle et poursuit le reflux pendant 1 heure. On distille 40 ml d'EtOH, puis laisse 2 heures et 30 minutes sous agitation en laissant redescendre la température à TA, essore le précipité formé, le lave à l'EtOH et le sèche sous vide à 60°C. On obtient 31 g du produit attendu.

B) Ester méthylique de l'acide 5-(2,6-diméthoxyphényl)pyrazole-3-carboxylique.

On prépare ce composé selon le mode opératoire décrit à l'étape B de la Préparation 1.4, à partir de 4 g du composé obtenu à l'étape précédente, 50 ml d'AcOH et 0,7 ml d'hydrazine monohydrate. Après une nuit sous agitation à TA, on verse le mélange réactionnel sur un mélange eau/glace, extrait à l'AcOEt, lave la phase organique par une solution saturée de NaCl, sèche sur $Na_2SO_4$ et concentre en partie le solvant sous vide. On essore le précipité formé et le sèche. On obtient 2,53 g du produit attendu.

C) Acide 1-(3,4-dichlorobenzyl)-5-(2,6-diméthoxyphényl)pyrazole-3-carboxylique.

On prépare ce composé selon le mode opératoire décrit à l'étape C de la Préparation 1.1 à partir de 2,5 g du composé obtenu à l'étape précédente, 50 ml de toluène, 0,88 g d'hydrure de sodium à 60 % dans l'huile et 2,16 g de bromure de 3,4-dichlorobenzyle. Après une nuit à reflux, on refroidit le mélange réactionnel à TA, ajoute goutte à goutte 50 ml d'une solution à 50 % de $NH_4Cl$ dans l'eau, essore le précipité formé et le sèche. On obtient 1,1 g du produit attendu.

RMN : δ (ppm) : 3,5 : s : 6H ; 4,8 : signal très élargi : 1H; 4,95 : s : 2H ; 6,25 : s : 1H ; 6,6 : d : 2H ; 6,85 : dd : 1H ; 6,95 ; d : 1H ; 7,3 : t : 1H ; 7,4 : d : 1H.

Préparation 1.16

Acide 1-(4-fluorobenzyl)-5-(3,4-diméthylphényl)pyrazole-3-carboxylique

A) Ester méthylique de l'acide 1-(4-fluorobenzyl)-5-(3,4-diméthylphényl)pyrazole-3-carboxylique.

On prépare ce composé selon le mode opératoire décrit à l'étape C de la Préparation 1.1 à partir de 2,3 g du composé obtenu à l'étape B de la Préparation 1.8, 50 ml de toluène, 0,88 g d'hydrure de sodium à 60 % dans l'huile, et 2,08 g de bromure de 4-fluorobenzyle. On obtient 1 g du produit attendu, F = 93°C.

RMN : δ (ppm) : 2,25 : 2s : 6H ; 3,8 : s : 3H ; 5,4 : s : 2H ; 6,85 : s : 1H ; 6,9 à 7,3 : m : 7H

B) Acide 1-(4-fluorobenzyl)-5-(3,4-diméthylphényl)pyrazole-3-carboxylique.

On prépare ce composé selon le mode opératoire décrit à l'étape B de la Préparation 1.14 à partir de 1 g du composé obtenu à l'étape précédente, 15 ml de MeOH, 0,406 g de KOH et 15 ml d'eau. On obtient 0,94 g du produit attendu, F = 141°C.

RMN : δ (ppm) : 2 : 2s : 6H ; 3,4 : se : 1H ; 5,35 : s : 2H ; 6,75 : s : 1H ; 6,8 à 7,3 : m : 7H

Préparation 1.17

Acide 1-(2,4-dichlorobenzyl)-5-(4-méthylphényl)pyrazole-3-carboxylique.

A) Ester méthylique de l'acide 1-(2,4-dichlorobenzyl)-5-(4-méthylphényl)pyrazole-3-carboxylique.

On prépare ce composé selon le mode opératoire décrit à l'étape C de la Préparation 1.1 à partir de 2,5 g du composé obtenu à l'étape B de la Préparation 1.1, 1 g d'hydrure de sodium à 60 % dans l'huile, 50 ml de toluène et 2,3 g de chlorure de 2,4-dichlorobenzyle. On obtient 2,53 g du produit attendu, F = 105°C.

B) Acide 1-(2,4-dichlorobenzyl)-5-(4-méthylphényl)pyrazole-3-carboxylique.

A une suspension de 1,5 g du composé obtenu à l'étape précédente dans 15 ml de MeOH, on ajoute à TA une solution de 0,5 g de KOH dans 15 ml d'eau puis chauffe à reflux pendant 2 heures. On concentre sous vide le mélange réactionnel, verse le résidu sur un mélange HCl 1N/glace, essore le précipité formé, le lave à l'eau et le sèche sous vide. On obtient 1,4 g du produit attendu.

RMN : δ (ppm) : 2,3 : s : 3H ; 5,45 : s : 2H ; 6,6 à 7,7 : m : 8H ; 12,85 : se : 1H.

Préparation 1.18

Acide 1-(4-éthylbenzyl)-5-(4-chloro-3-méthylphényl)pyrazole-3-carboxylique.

A) Ester méthylique de l'acide 1-(4-éthylbenzyl)-5-(4-chloro-3-méthylphényl) pyrazole-3-carboxylique.

On prépare ce composé selon le mode opératoire décrit à l'étape C de la Préparation 1.1 à partir de 2,5 g du composé obtenu à l'étape B de la Préparation 1.10 dans 50 ml de toluène, 0,88 g d'hydrure de sodium à 60 % dans l'huile et 2 g de bromure de 4-éthylbenzyle (Préparation 3.5). Après hydrolyse par la solution à 50 % de $NH_4Cl$ dans l'eau puis décantation, on concentre sous vide la phase organique. On extrait le résidu à l'AcOEt, lave la phase organique par une solution saturée de NaCl, sèche sur $Na_2SO_4$ et évapore sous vide le solvant. On chromatographie le résidu sur silice en éluant par le mélange cyclohexane/AcOEt (80/20 ; v/v). On obtient 2,63 g du produit attendu.

B) Acide 1-(4-éthylbenzyl)-5-(4-chloro-3-méthylphényl)pyrazole-3-carboxylique.

On prépare ce composé selon le mode opératoire décrit à l'étape B de la Préparation 1.17 à partir de 2,5 g du composé obtenu à l'étape précédente dans 20 ml de MeOH et 0,57 g de KOH dans 20 ml d'eau. On obtient 2,2 g du produit attendu.

RMN : $\delta$ (ppm) : 1,1 : t : 3H ; 2,3 : s : 3H ; 2,5 : mt : 2H ; 5,4 : s : 2H ; 6,7 à 7,7 : m : 8H ; 12,9 : se : 1H.

Préparation 1.19

Acide 1-(3,4-dichlorobenzyl)-5-(4-chloro-3-méthylphényl)pyrazole-3-carboxylique.

A une suspension de 2,5 g du composé obtenu à l'étape B de la Préparation 1.10 dans 25 ml de toluène, on ajoute à TA et par portions 0,88 g d'hydrure de sodium à 60 % dans l'huile, puis chauffe le mélange réactionnel à 65°C pendant 1 heure. Après refroidissement à TA, on ajoute goutte à goutte une solution de 2,4 g de bromure de 3,4-dichlorobenzyle dans 25 ml de toluène puis chauffe à reflux pendant une nuit. On refroidit le mélange réactionnel à TA, essore le précipité formé et le sèche. On obtient 2 g du produit attendu utilisé tel quel.

Préparation 1.20

Acide 1-(2,4-dichlorobenzyl)-5-(4-chloro-3-méthylphényl)pyrazole-3-carboxylique.

A) Ester méthylique de l'acide 1-(2,4-dichlorobenzyl)-5-(4-chloro-3-méthylphényl) pyrazole-3-carboxylique.

On prépare ce composé selon le mode opératoire décrit à l'étape A de la Préparation 1.18 à partir de 2,5 g du composé obtenu à l'étape B de la Préparation 1.10 dans 25 ml de toluène, 0,88 g d'hydrure dc sodium à 60 % dans l'huile et 1,6 ml de chlorure de 2,4-dichlorobenzyle dans 25 ml de toluène. On obtient 0,86 g du produit attendu.

B) Acide 1-(2,4-dichlorobenzyl)-5-(4-chloro-3-méthylphényl)pyrazole-3-carboxylique.

On prépare ce composé selon le mode opératoire décrit à l'étape B de la Préparation 1.17 à partir de 0,5 g du composé obtenu à l'étape précédente dans 15 ml de MeOH et 0,205 g de KOH dans 15 ml d'eau. On obtient 0,31 g du produit attendu.

RMN : $\delta$ (ppm) : 2,25 : s : 3H ; 5,4 : s : 2H ; 6,6 à 7,6 : m : 7H ; 3,33 : avec DOH : 1H.

On observe un effet Overhauser (N.O.E.) entre les protons benzyliques ($R_4 = R_5 = H$) et les protons $g_2 = g_6 = H$.

Préparation 1.21

Acide 1-(4-méthylbenzyl)-5-(3,4-dichlorophényl)pyrazole-3-carboxylique.

A) Sel de sodium du 4-(3,4-dichlorophényl)-2-oxo-4-oxydobut-3-énoate de méthyle.

On dissout 15,17 g de sodium dans 500 ml de McOH. Après refroidissement à TA, on ajoute 124,97 g de 3', 4'-dichloroacétophénone puis une solution de 91 ml d'oxalate de diéthyle dans 400 ml de MeOH et laisse 2 heures sous agitation à TA. On ajoute 1 litre d'éther et laisse 30 minutes sous agitation à TA. On essore le précipité formé,

le lave à l'éther et le sèche. On obtient 140,77 g du produit attendu.

B) Ester méthylique de l'acide 5-(3,4-dichlorophényl)pyrazole-3-carboxylique.

On prépare ce composé selon le mode opératoire décrit à l'étape B de la Préparation 1.1 à partir de 15 g du composé obtenu à l'étape précédente dans 150 ml d'AcOH et 6 ml d'une solution à 55 % d'hydrazine dans l'eau. Après une nuit sous agitation à TA, on verse le mélange réactionnel dans de l'eau glacée, essore le précipité formé et le lave à l'eau. On obtient 12,9 g du produit attendu après séchage.

C) Ester méthylique de l'acide 1-(4-méthylbenzyl)-5-(3,4-dichlorophényl)pyrazole-3-carboxylique.

On prépare ce composé selon le mode opératoire décrit à l'étape A de la Préparation 1.18 à partir de 2,5 g du composé obtenu à l'étape précédente dans 25 ml de toluène, 0,88 g d'hydrure de sodium à 60 % dans l'huile et 1,9 g de bromure de 4-méthylbenzyle dans 25 ml de toluène. On obtient 0,82 g du produit attendu.

D) Acide 1-(4-méthylbenzyl)-5-(3,4-dichlorophényl)pyrazole-3-carboxylique.

On prépare ce composé selon le mode opératoire décrit à l'étape B de la Préparation 1.17 à partir de 0,7 g du composé obtenu à l'étape précédente dans 15 ml de MeOH et 0,252 g de KOH dans 15 ml d'eau. On obtient 0,65 g du produit attendu.

RMN : δ (ppm) : 2,2 : s : 3H ; 5,35 : s : 2H ; 6,7 à 7,2 : m : 5H ; 7,4 : dd : 1H ; 7,6 à 7,8 : m : 2H ; 12,85 : se : 1H.

Préparation 1.22

Acide 1-(3-chloro-4-méthylbenzyl)-5-(3,4-dichlorophényl)pyrazole-3-carboxylique.

A) Ester méthylique de l'acide 1-(3-chloro-4-méthylbenzyl)-5-(3,4-dichlorophényl) pyrazole-3-carboxylique.

On prépare ce composé selon le mode opératoire décrit à l'étape A de la Préparation 1.18 à partir de 20,51 g du composé obtenu à l'étape B de la Préparation 1.21 dans 350 ml de toluène, 2,18 g d'hydrure de sodium à 60 % dans l'huile et 21,96 g d'iodure de 3-chloro-4-méthylbenzyle (Préparation 3.2). On obtient 21,79 g du produit attendu après cristallisation dans l'hexane.

B) Acide 1-(3-chloro-4-méthylbenzyl)-5-(3,4-dichlorophényl)pyrazole-3-carboxylique.

On prépare ce composé selon le mode opératoire décrit à l'étape B de la Préparation 1.17 à partir de 21,79 g du composé obtenu à l'étape précédente dans 50 ml de MeOH et 8,93 g de KOH dans 50 ml d'eau. On obtient 17,43 g du produit attendu utilisé tel quel.

Préparation 1.23

Acide 1-(2,4-dichlorobenzyl)-5-(4-méthylthiophényl)pyrazole-3-carboxylique.

A) 4'-Méthylthioacétophénone.

On ajoute goutte à goutte et à une température comprise entre 0°C et 10°C, 11,8 ml de chlorure d'acétyle à une suspension de 20,4 g de chlorure d'aluminium dans 85 ml de chloroforme. Puis on ajoute, goutte à goutte et à 0-5°C, 15 ml de thioanisole et laisse 1 heure et 30 minutes sous agitation à TA. On refroidit le mélange réactionnel à 0°C et hydrolyse par ajout de 100 ml d'eau. On extrait au chloroforme, lave la phase organique à l'eau, sèche sur $Na_2SO_4$ et évapore sous vide le solvant. On obtient 10,7 g du produit attendu après cristallisation dans l'EtOH et recristallisation dans l'heptane.

B) Sel de sodium du 4-(4-méthylthiophényl)-2-oxo-4-oxydobut-3-énoate de méthyle.

On dissout 1,48 g de sodium dans 35 ml de MeOH et ajoute rapidement cette solution à une suspension de 10,7 g du composé obtenu à l'étape précédente et 9,8 ml d'oxalate de diéthyle dans 80 ml de MeOH refroidie à 0°C. On laisse 30 minutes sous agitation à TA, chauffe à reflux pendant 1 heure puis laisse 2 heures sous agitation à TA. On verse le mélange réactionnel dans 400 ml d'éther, laisse 15 minutes sous agitation, essore le précipité formé, le lave à l'éther et le sèche. On obtient 12,8 g du produit attendu.

C) Ester méthylique de l'acide 5-(4-méthylthiophényl)pyrazole-3-carboxylique.

On prépare ce composé selon le mode opératoire décrit à l'étape B de la Préparation 1.1 à partir de 15 g du composé obtenu à l'étape précédente dans 140 ml d'AcOH et 7 ml d'une solution à 55 % d'hydrazine dans l'eau. Après une nuit sous agitation à TA, on verse le mélange réactionnel dans un mélange eau/glace, essore le précipité formé et le lave à l'eau. On obtient 12,96 g du produit attendu après séchage sous vide et sur KOH.

D) Ester méthylique de l'acide 1-(2,4-dichlorobenzyl)-5-(4-méthylthiophényl) pyrazole-3-carboxylique.

On prépare ce composé selon le mode opératoire décrit à l'étape A de la Préparation 1.18 à partir de 5 g du

composé obtenu à l'étape précédente dans 100 ml de toluène, 1,063 g d'hydrure de sodium à 60 % dans l'huile et 3,4 ml de chlorure de 2,4-dichlorobenzyle. On obtient 3,2 g du produit attendu.

E) Acide 1-(2,4-dichlorobenzyl)-5-(4-méthylthiophényl)pyrazole-3-carboxylique.

On prépare ce composé selon le mode opératoire décrit à l'étape B de la Préparation 1.14 à partir de 1,52 g du composé obtenu à l'étape précédente dans 30 ml de MeOH et 0,7 g de KOH dans 25 ml d'eau. On obtient 1,4 g du produit attendu après séchage sous vide.

RMN : δ (ppm) : 2,5 : s : 3H ; 5,4 : s : 2H ; 6,6 à 7,6 : m : 8H ; 12,8 : se : 1H.

Préparation 1.24

Acide 1-(2,4-dichlorobenzyl)-5-(4-trifluorométhylphényl)pyrazole-3-carboxylique.

A) Sel de sodium du 4-(4-trifluorométhylphényl)-2-oxo-4-oxydobut-3-énoate de méthyle.

On prépare ce composé selon le mode opératoire décrit à l'étape A de la Préparation 1.4 à partir de 4'-trifluorométhylacétophénone.

B) Ester méthylique de l'acide 5-(4-trifluorométhylphényl)pyrazole-3-carboxylique.

On prépare ce composé selon le mode opératoire décrit à l'étape C de la Préparation 1.23 à partir de 6,37 g du composé obtenu à l'étape précédente dans 50 ml d'AcOH et 3 ml d'une solution à 55 % d'hydrazine dans l'eau. On obtient 5,43 g du produit attendu.

C) Ester méthylique de l'acide 1-(2,4-dichlorobenzyl)-5-(4-trifluorométhylphényl) pyrazole-3-carboxylique.

On prépare ce composé selon le mode opératoire décrit à l'étape A de la Préparation 1.18 à partir de 2,5 g du composé obtenu à l'étape précédente dans 150 ml de toluène, 0,44 g d'hydrure de sodium à 60 % dans l'huile et 2 ml de chlorure de 2,4-dichlorobenzyle. On obtient 1,82 g du produit attendu.

D) Acide 1-(2,4-dichlorobenzyl)-5-(4-trifluorométhylphényl)pyrazole-3-carboxylique.

On prépare ce composé selon le mode opératoire décrit à l'étape B de la Préparation 1.14 à partir de 1,6 g du composé obtenu à l'étape précédente dans 30 ml de MeOH et 0,63 g de KOH dans 30 ml d'eau. On obtient 1,5 g du produit attendu.

RMN : δ (ppm) : 5,45 : s : 2H ; 6,85 : d : 1H; 6,95 : s : 1H; 7,3 : dd : 1H ; 7,5 : d : 1H; 7,55 à 7,8 : système AA'-BB' : 4H ; 12,9 : se : 1H.

Préparation 1.25

Acide 1-[1-(3,4-dichlorophényl)éthyl]-5-(4-méthylphényl)pyrazole-3-carboxylique.

A) Ester méthylique de l'acide 1-[1-(3,4-dichlorophényl)éthyl]-5-(4-méthylphényl) pyrazole-3-carboxylique.

A une suspension de 2,5 g du composé obtenu à l'étape B de la Préparation 1.1 dans 25 ml de toluène on ajoute à TA et par portions 1 g d'hydrure de sodium à 60 % dans l'huile, puis chauffe à 65°C pendant 1 heure. Puis on ajoute goutte à goutte une solution de 3 g de 1-(1-bromoéthyl)-3,4-dichlorobenzène (Préparation 3.6) dans 25 ml de toluène puis chauffe à reflux pendant une nuit. On refroidit à 0°C et ajoute goutte à goutte 100 ml d'une solution à 50 % de $NH_4Cl$ dans l'eau. Après décantation, on lave la phase organique par une solution saturée de NaCl, sèche sur $Na_2SO_4$ et évapore sous vide le solvant. On chromatographie le résidu sur silice en éluant par le mélange cyclohexane/AcOEt (75/25 ; v/v). On obtient 1,6 g du produit attendu.

B) Acide 1-[1-(3,4-dichlorophényl)éthyl]-5-(4-méthylphényl)pyrazole-3-carboxylique.

A une suspension de 1,4 g du composé obtenu à l'étape précédente dans 15 ml de MeOH, on ajoute à TA une solution de 0,5 g de KOH dans 15 ml d'eau, puis chauffe à reflux pendant 2 heures. Après concentration sous vide du MeOH, on verse le mélange réactionnel sur un mélange HCl 1N/glace, essore le précipité formé, le lave à l'eau et le sèche sous vide sur KOH. On obtient 1,25 g du produit attendu.

RMN : δ (ppm) : 1,85 : d : 3H ; 2,4 : s : 3H ; 5,7 ; qd : 1H ; 6,85 : s : 1H ; 7,05 : dd : 1H ; 7,2 à 7,45 : m : 5H ; 7,65 : d : 1H; 12,95 : se : 1H.

Préparation 1.26

Acide 1-[1-(4-méthylphényl)éthyl]-5-(4-chloro-3-méthylphényl)pyrazole-3-carboxylique.

A) Ester méthylique de l'acide 1-[1-(4-méthylphényl)éthyl]-5-(4-chloro-3-méthylphényl)pyrazole-3-carboxylique.

On prépare ce composé selon le mode opératoire décrit à l'étape A de la Préparation 1.25 à partir de 2,5 g du composé obtenu à l'étape B de la Préparation 1.10 dans 25 ml de toluène, 0,88 g d'hydrure de sodium à 60 % dans l'huile et 2,3 g de 1-(1-bromoéthyl)-4-méthylbenzène (Préparation 3.7) dans 25 ml de toluène. Après addition de la solution aqueuse de $NH_4Cl$ et décantation, on concentre sous vide la phase organique, reprend le résidu à l'AcOEt, lave par une solution saturée de NaCl, sèche sur $Na_2SO_4$ et évapore sous vide le solvant. On chromatographie le résidu sur silice en éluant par le mélange cyclohexane/AcOEt (75/25 ; v/v). On obtient 0,7 g du produit attendu.

B) Acide 1-[1-(4-méthylphényl)éthyl]-5-(4-chloro-3-méthylphényl)pyrazole-3-carboxylique.

On prépare ce composé selon le mode opératoire décrit à l'étape B de la Préparation 1.25 à partir de 0,7 g du composé obtenu à l'étape précédente dans 15 ml de MeOH et une solution de 0,28 g de KOH dans 15 ml d'eau. On obtient 0,63 g du produit attendu utilisé tel quel.

Préparation 1.27

Acide 1-[1-(3,4-dichlorophényl)éthyl]-5-(4-chloro-3-méthylphényl)pyrazole-3-carboxylique.

A) Ester méthylique de l'acide 1-[1-(3,4-dichlorophényl)éthyl]-5-(4-chloro-3-méthyl phényl)pyrazole-3-carboxylique.

On prépare ce composé selon le mode opératoire décrit à l'étape A de la Préparation 1.25 à partir de 3,0 g du composé obtenu à l'étape B de la Préparation 1.10 dans 25 ml de toluène, 1 g d'hydrure de sodium à 60 % dans l'huile et 3 g de 1-(1-bromoéthyl)-3,4-dichlorobenzène (Préparation 3.6) dans 25 ml de toluène. Après addition de la solution aqueuse de $NH_4Cl$ et décantation, on concentre sous vide la phase organique, reprend le résidu à l'AcOEt, lave par une solution saturée de NaCl, sèche sur $Na_2SO_4$ et évapore sous vide le solvant. On chromatographie le résidu sur silice en éluant par le mélange cyclohexane/AcOEt (80/20 ; v/v). On obtient 1,4 g du produit attendu.

B) Acide 1-[1-(3,4-dichlorophényl)éthyl]-5-(4-chloro-3-méthylphényl)pyrazole-3-carboxylique.

On prépare ce composé selon le mode opératoire décrit à l'étape B de la Préparation 1.25 à partir de 1 g du composé obtenu à l'étape précédente dans 20 ml de MeOH et une solution de 0,40 g de KOH dans 20 ml d'eau. On obtient 0,96 g du produit attendu.

RMN : $\delta$ (ppm) : 1,8 : d : 3H ; 2,35 : s : 3H ; 5,7 : qd : 1H ; 6,7 à 7,7 : m : 7H ; 12,9 : se : 1H.

On observe un effet Overhauser (N.O.E.) entre le proton benzylique $R_4$ = H et les protons $g_2$ = $g_6$ = H.

Préparation 1.28

Acide 1-[1-(4-méthylphényl)propyl]-5-(4-chloro-3-méthylphényl)pyrazole-3-carboxylique.

A) N,N'-Dipropylidènehydrazine.

On refroidit à 0°C 58 g de propionaldéhyde, ajoute 10 ml d'eau, puis goutte à goutte 19,9 ml d'hydrazine monohydrate en maintenant la température en dessous de 10°C. On ajoute ensuite, par portions, 67,5 g de KOH en pastilles et laisse le mélange réactionnel au repos une nuit à TA. Après décantation, on distille sous pression réduite la phase organique. On obtient 24,37 g du produit attendu, Eb = 48°C sous 2400 Pa.

B) N-Propylidène-N'-[1-(4-méthylphényl)propyl]hydrazine.

On chauffe à reflux, sous atmosphère d'azote, 100 ml d'une solution 1M de bromure de *p*-tolylmagnésium dans l'éther puis ajoute goutte à goutte une solution de 10 g du composé obtenu à l'étape précédente dans 40 ml d'éther anhydre et laisse une nuit sous agitation à TA. Après refroidissement du mélange réactionnel à 5°C, on ajoute une solution aqueuse saturée de $NH_4Cl$, décante, extrait à l'éther, sèche les phases organiques jointes sur

$MgSO_4$ et évapore sous vide le solvant. On obtient 6 g du produit attendu, Eb = 102-103°C sous 20 Pa ; $n_D^{20}$ = 1,5180.

C) Oxalate de [1-(4-méthylphényl)propyl]hydrazine.

A une solution de 4,78 g d'acide oxalique dans 20 ml d'EtOH et 20 ml d'éther, on ajoute une solution de 6 g du composé obtenu à l'étape précédente dans 5 ml d'éther et laisse une nuit à 0-5°C. On essore le produit cristallisé formé et le lave à l'éther. On obtient 1,84 g du produit attendu.

D) Ester méthylique de l'acide 1-[1-(4-méthylphényl)propyl]-5-(4-chloro-3-méthyl phényl)pyrazole-3-carboxylique.

A une solution de 2 g du composé obtenu à l'étape A de la Préparation 1.10 dans 100 ml d'eau et 100 ml d'EtOH, on ajoute à TA une solution de 1,84 g du composé obtenu à l'étape précédente dans 20 ml d'eau et 30 ml d'EtOH et laisse 3 heures sous agitation à TA. On extrait le mélange réactionnel à l'éther, sèche la phase organique sur $Na_2SO_4$ et évapore sous vide le solvant. On reprend le résidu dans l'hexane, filtre un insoluble et concentre sous vide le filtrat. On chromatographie le résidu sur silice en éluant par le mélange cyclohexane/AcOEt (80/20 ; v/v). On obtient 1,12 g du produit attendu.

E) Acide 1-[1-(4-méthylphényl)propyl]-5-(4-chloro-3-méthylphényl)pyrazole-3-carboxylique.

A une solution de 1,1 g du composé obtenu à l'étape précédente dans 40 ml d'EtOH on ajoute à TA une solution de 0,47 g de KOH dans 10 ml d'eau puis chauffe à reflux pendant 3 heures. On concentre sous vide, reprend le résidu dans 50 ml d'eau, acidifie à pH = 2 par ajout d'une solution d'HCl 6N, essore le précipité formé, le lave à l'eau et le sèche. On obtient 0,89 g du produit attendu.

Préparation 1.29

Acide 1-[1-méthyl-1-(4-méthylphényl)éthyl]-5-(4-chloro-3-méthylphényl) pyrazole-3-carboxylique.

A) N,N'-Diisopropylidènehydrazine.

On prépare ce composé selon le mode opératoire décrit à l'étape A de la Préparation 1.28 à partir de 92 g d'acétone, 20 ml d'eau, 39,7 ml d'hydrazine monohydrate et 135 g de KOH. On obtient 66 g du produit attendu, Eb = 55°C sous 2100 Pa.

B) N-Isopropylidène-N'-[1-méthyl-1-(4-méthylphényl)éthyl]hydrazine.

On chauffe à reflux, sous atmosphère d'azote, 100 ml d'une solution 1M de bromure de *p*-tolylmagnésium dans l'éther, puis ajoute goutte à goutte une solution de 8,46 g du composé obtenu à l'étape précédente dans 200 ml d'éther anhydre et poursuit le reflux pendant 5 jours. Après refroidissement à 5°C, on ajoute une solution aqueuse saturée de $NH_4Cl$, décante, extrait à l'éther, sèche les phases organiques jointes sur $Na_2SO_4$ et évapore sous vide le solvant. On distille le résidu sous pression réduite. On obtient 6,45 g du produit attendu, Eb = 95°C sous 266,6 Pa.

C) Oxalate de [1-méthyl-1-(4-méthylphényl)éthyl]hydrazine.

A une solution de 5,12 g d'acide oxalique dans 24 ml d'EtOH et 24 ml d'éther, on ajoute une solution de 6,45 g du composé obtenu à l'étape précédente dans 5 ml d'éther et laisse une nuit à TA. On essore le produit cristallisé formé et le lave à l'hexane. On obtient 2,57 g du produit attendu.

D) Ester méthylique de l'acide 1-[1-méthyl-1-(4-méthylphényl)éthyl]-5-(4-chloro-3-méthylphényl)pyrazole-3-carboxylique.

A une solution de 2,66 g du composé obtenu à l'étape A de la Préparation 1.10 dans 600 ml d'eau et 1 litre d'EtOH, on ajoute à TA une solution de 2,54 g du composé obtenu à l'étape précédente dans 200 ml d'eau et 300 ml d'EtOH et laisse 3 heures sous agitation à TA puis 24 heures au repos. On essore le précipité formé, le lave à l'hexane et le sèche. On obtient 3,46 g du produit attendu.

E) Acide 1-[1-méthyl-1-(4-méthylphényl)éthyl]-5-(4-chloro-3-méthylphényl) pyrazole-3-carboxylique.

A une solution de 3,3 g du composé obtenu à l'étape précédente dans 80 ml d'EtOH on ajoute à TA une solution de 1,44 g de KOH dans 20 ml d'eau puis chauffe à reflux pendant 3 heures. On filtre un insoluble et concentre sous vide le filtrat jusqu'à 20 ml. On ajoute 30 ml d'eau glacée puis 50 ml de DCM, acidifie à pH = 3,5 par ajout d'HCl 1N, décante, sèche la phase organique sur $Na_2SO_4$ et évapore sous vide le solvant. On obtient 3,15 g du produit attendu.

Préparation 1.30

Acide 1-(2,4-dichlorobenzyl)-5-(4-chlorophényl)-4-méthylpyrazole-3-carboxylique.

A) Sel de lithium du 4-(4-chlorophényl)-3-méthyl-4-oxydo-2-oxobut-3-énoate d'éthyle.

On ajoute, sous atmosphère d'azote, 154 ml d'une solution 1M du sel de lithium de l'hexaméthyldisilazane dans le THF à 120 ml de cyclohexane refroidi à 0°C. On ajoute ensuite à 0°C, en 30 minutes et goutte à goutte, une solution de 21,6 g de 4'-chloropropiophénone dans 60 ml de cyclohexane et laisse 3 heures sous agitation à TA. Puis on ajoute rapidement 21,4 g d'oxalate de diéthyle en maintenant la température en dessous de 25°C et laisse 48 heures sous agitation à TA. On essore le précipité formé, le lave au cyclohexane et le sèche. On obtient 31,3 g du produit attendu.

B) Ester éthylique de l'acide 5-(4-chlorophényl)-4-méthylpyrazole-3-carboxylique.

A une solution de 15 g du composé obtenu à l'étape précédente dans 100 ml d'AcOH, on ajoute à TA et goutte à goutte, 2,04 ml d'hydrazine monohydrate, puis chauffe à reflux pendant 5 heures et laisse une nuit sous agitation à TA. On verse le mélange réactionnel sur un mélange eau/glace, essore le précipité formé, le lave à l'eau, puis à l'hexane et le sèche. On obtient 11,47 g du produit attendu.

C) Ester éthylique de l'acide 1-(2,4-dichlorobenzyl)-5-(4-chlorophényl)-4-méthylpyrazole-3-carboxylique.

A une solution de 2,17 g du composé obtenu à l'étape précédente dans 25 ml de toluène, on ajoute à TA et par portions, 0,72 g d'hydrure de sodium à 60 % dans l'huile, puis chauffe à 65°C pendant 1 heure. Après refroidissement à TA, on ajoute goutte à goutte une solution de 2,28 g de chlorure de 2,4-dichlorobenzyle dans 25 ml de toluène puis chauffe à reflux pendant 20 heures. Après refroidissement à TA, on ajoute une solution aqueuse saturée de $NH_4Cl$, décante, lave la phase organique par une solution saturée de NaCl, sèche sur $MgSO_4$ et évapore sous vide le solvant. On extrait le résidu à l'AcOEt, lave la phase organique par une solution saturée de $NaHCO_3$, par une solution tampon pH = 2, à l'eau, sèche sur $MgSO_4$ et évapore sous vide le solvant. On chromatographie le résidu sur silice en éluant par le mélange cyclohexane/AcOEt (75/25 ; v/v). On obtient 1,10 g du produit attendu, F = 82°C.

RMN : $\delta$ (ppm) : 1,2 : t : 3H ; 2,0 : s : 3H ; 4,2 : qd : 2H ; 5,3 : s : 2H ; 6,7 : d : 1H; 7,15 à 7,6 : m : 6H.

On observe un effet Overhauser (N.O.E.) entre les protons benzyliques ($R_4 = R_5 = H$) et les protons $g_2 = g_6 = H$.

D) Acide 1-(2,4-dichlorobenzyl)-5-(4-chlorophényl)-4-méthylpyrazole-3-carboxylique.

A une solution de 0,8 g du composé obtenu à l'étape précédente dans 50 ml d'EtOH, on ajoute à TA une solution de 0,166 g de KOH dans 10 ml d'eau puis chauffe à reflux pendant 4 heures. On concentre sous vide, reprend le résidu à l'eau, filtre un insoluble, acidifie le filtrat à pH = 2 par ajout d'HCl 6N, essore le précipité formé, le lave à l'eau et le sèche. On obtient 0,73 g du produit attendu.

Préparation 1.31

Acide 1-(3,4-dichlorobenzyl)-5-(4-chlorophényl)-4-méthylpyrazole-3-carboxylique.

A) Ester éthylique de l'acide 1-(3,4-dichlorobenzyl)-5-(4-chlorophényl)-4-méthyl pyrazole-3-carboxylique.

On prépare ce composé selon le mode opératoire décrit à l'étape C de la Préparation 1.30 à partir de 2,17 g du composé obtenu à l'étape B de la Préparation 1.30 dans 25 ml de toluène, 0,72 g d'hydrure de sodium à 60 % dans l'huile et 2,99 g de bromure de 3,4-dichlorobenzyle dans 25 ml de toluène. On obtient 1,87 g du produit attendu, F = 117,5°C.

RMN : $\delta$ (ppm) : 1,25 : t : 3H ; 2,05 : s : 3H ; 4,25 : qd : 2H ; 5,3 : s : 2H ; 6,8 : dd : 1H ; 7,15 : s : 1H ; 7,3 : d : 2H ; 7,4 à 7,6 : m : 3H.

On observe un effet Overhauser (N.O.E.) entre les protons benzyliques ($R_4 = R_5 = H$) et les protons $g_2 = g_6 = H$.

B) Acide 1-(3,4-dichlorobenzyl)-5-(4-chlorophényl)-4-méthylpyrazole-3-carboxylique.

On prépare ce composé selon le mode opératoire décrit à l'étape D de la Préparation 1.30 à partir de 1,5 g du composé obtenu à l'étape précédente dans 50 ml d'EtOH et 0,3 g de KOH dans 10 ml d'eau. On obtient 1,31 g du produit attendu.

Préparation 1.32

Acide 1-(3-chloro-4-méthylbenzyl)-5-(4-chlorophényl)-4-méthylpyrazole-3-carboxylique.

A une solution de 2,17 g du composé obtenu à l'étape B de la Préparation 1.30 dans 100 ml de toluène, on ajoute à TA et par portions, 0,72 g d'hydrure de sodium à 60 % dans l'huile, puis chauffe à 65°C pendant 1 heure. Après refroidissement à TA, on ajoute goutte à goutte 2,2 g de iodure de 3-chloro-4-méthylbenzyle puis chauffe à reflux pendant 20 heures. Après refroidissement à TA, on ajoute une solution aqueuse saturée de $NH_4Cl$, décante, lave la phase organique par une solution saturée de NaCl, sèche sur $MgSO_4$ et évapore sous vide le solvant. On extrait le résidu à l'AcOEt, lave la phase organique par une solution saturée de $NaHCO_3$, par une solution tampon pH = 2, à l'eau, sèche sur $MgSO_4$ et évapore sous vide le solvant. On reprend le résidu dans un mélange cyclohexane/AcOEt (75/25 ; v/v), essore le précipité et obtient 1,38 g de produit brut. On dissout 0,35 g de produit ainsi obtenu dans l'AcOEt, ajoute du cyclohexane jusqu'à précipitation et essore. On obtient 0,17 g du produit attendu purifié, F = 162°C.

RMN : δ (ppm) : 1,95 à 2,6 : m : 6H ; 5,2 : s : 2H ; 6,6 à 7,7 : m : 7H ; 12,85 : se : 1H.

On observe un effet Overhauser (N.O.E.) entre les protons benzyliques ($R_4 = R_5 = H$) et les protons $g_2 = g_6 = H$.

Préparation 1.33

Acide 1-(4-méthylbenzyl)-5-(4-chloro-3-méthylphényl)-4-méthylpyrazole-3-carboxylique.

A) 4'-Chloro-3'-méthylpropiophénone.

A un mélange de 18 ml de *o*-chlorotoluène et 22,7 g de chlorure d'aluminium, on ajoute goutte à goutte 16 g de chlorure de propionyle puis chauffe à 130°C pendant 3 heures. Après refroidissement à TA, on verse le mélange réactionnel dans un mélange de 100 ml d'HCl concentré et de glace, extrait à l'éther, lave la phase organique par une solution saturée de $NaHCO_3$, sèche sur $Na_2SO_4$ et évapore sous vide le solvant. On distille le résidu sous pression réduite. On obtient 20,7 g du produit attendu qui cristallise, Eb = 95°C sous 5 Pa.

B) Sel de lithium du 4-(4-chloro-3-méthylphényl)-3-méthyl-4-oxydo-2-oxobut-3-énoate d'éthyle.

On prépare ce composé selon le mode opératoire décrit à l'étape A de la Préparation 1.30 à partir de 50 ml d'une solution 1M du sel de lithium de l'hexaméthyldisilazane dans le THF, 40 ml de cyclohexane, 10 g du composé obtenu à l'étape précédente dans 70 ml de cyclohexane et 9,8 g d'oxalate de diéthyle. Après une nuit sous agitation à TA, on concentre sous vide le mélange réactionnel, reprend le résidu à l'éther et évapore sous vide le solvant. On reprend le résidu à l'heptane et essore le précipité formé. On obtient 15 g du produit attendu.

C) Ester éthylique de l'acide 5-(4-chloro-3-méthylphényl)-4-méthylpyrazole-3-carboxylique.

On refroidit à 5°C une solution de 15 g du composé obtenu à l'étape précédente dans 150 ml d'AcOH, ajoute goutte à goutte 2,5 ml d'hydrazine monohydrate, puis chauffe à reflux pendant 5 heures et laisse une nuit sous agitation à TA. On verse le mélange réactionnel sur un mélange eau/glace, extrait à l'AcOEt, lave trois fois la phase organique par une solution saturée de NaCl, sèche sur $Na_2SO_4$ et évapore sous vide le solvant. On reprend le résidu à l'éther iso, essore le précipité formé, le lave à l'éther iso et le sèche. On obtient 7,88 g du produit attendu.

D) Ester éthylique de l'acide 1-(4-méthylbenzyl)-5-(4-chloro-3-méthylphényl)-4-méthylpyrazole-3-carboxylique.

A une suspension de 5 g du composé obtenu à l'étape précédente dans 70 ml de toluène, on ajoute à TA et par portions, 0,98 g d'hydrure de sodium à 60 % dans l'huile, puis chauffe à 65°C pendant 1 heure. Après refroidissement à TA, on ajoute goutte à goutte 3,7 g de bromure de 4-méthylbenzyle puis chauffe à reflux pendant une nuit. Après refroidissement à TA, on ajoute une solution aqueuse à 50 % de $NH_4Cl$, décante et concentre sous vide la phase organique. On extrait le résidu à l'AcOEt, lave deux fois la phase organique par une solution saturée de NaCl, sèche sur $Na_2SO_4$ et évapore sous vide le solvant. On chromatographie le résidu sur silice en éluant par le mélange cyclohexane/AcOEt (80/20 ; v/v). On obtient 2,1 g du produit attendu.

E) Acide 1-(4-méthylbenzyl)-5-(4-chloro-3-méthylphényl)-4-méthylpyrazole-3-carboxylique.

A une solution de 2 g du composé obtenu à l'étape précédente dans 20 ml d'EtOH, on ajoute à TA une solution de 0,439 g de KOH dans 20 ml d'eau puis chauffe à reflux pendant 3 heures. Après refroidissement à TA, on verse le mélange réactionnel sur un mélange d'HCl 1N et de glace, essore le précipité formé, le lave à l'eau et le sèche sous vide. On obtient 1,8 g du produit attendu.

RMN : δ (ppm) : 2 à 2,4 : m : 9H ; 5,2 : s : 2H ; 6,7 à 7,6 : m : 7H ; 12,65 : se : 1H.

Préparation 1.34

Acide 1-(3,4-dichlorobenzyl)-5-(4-chloro-3-méthylphényl)-4-méthylpyrazole-3-carboxylique.

A) Ester éthylique de l'acide 1-(3,4-dichlorobenzyl)-5-(4-chloro-3-méthylphényl)-4-méthylpyrazole-3-carboxylique.

On prépare ce composé selon le mode opératoire décrit à l'étape D de la Préparation 1.33 à partir de 2,5 g du composé obtenu à l'étape C de la Préparation 1.33 dans 50 ml de toluène, 0,8 g d'hydrure de sodium à 60 % dans l'huile et 2,4 g de bromure de 3,4-dichlorobenzyle. Après hydrolyse par ajout d'une solution aqueuse à 50 % de $NH_4Cl$, on décante la phase organique, filtre un insoluble et concentre sous vide le filtrat. On extrait le résidu à l'AcOEt, lave deux fois la phase organique par une solution saturée de NaCl, sèche sur $Na_2SO_4$ et évapore sous vide le solvant. On chromatographie le résidu sur silice en éluant par le mélange cyclohexane/AcOEt (80/20 ; v/v). On obtient 3 g du produit attendu.

B) Acide 1-(3,4-dichlorobenzyl)-5-(4-chloro-3-méthylphényl)-4-méthylpyrazole-3-carboxylique.

On prépare ce composé selon le mode opératoire décrit à l'étape E de la Préparation 1.33 à partir de 3 g du composé obtenu à l'étape précédente dans 20 ml d'EtOH et 0,56 g de KOH dans 20 ml d'eau. On obtient 2,7 g du produit attendu.

RMN : $\delta$ (ppm) : 2 : s : 3H ; 2,3 : m : 3H ; 5,2 : s : 2H ; 6,7 à 7,6 : m : 6H ; 12,65 : se : 1H.

Préparation 1.35

Acide 1-(4-chloro-3-méthylbenzyl)-5-(4-chlorophényl)-4-(éthoxyméthyl) pyrazole-3-carboxylique.

A) Ester éthylique de l'acide 1-(tert-butoxycarbonyl)-5-(4-chlorophényl)-4-méthyl pyrazole-3-carboxylique.

A une solution de 6 g du composé obtenu à l'étape B de la Préparation 1.30 dans 150 ml de dioxane, on ajoute 4,62 ml de triéthylamine, 6,57 g de *di-tert*-butyldicarbonate et 1,18 g d'hydrure de sodium à 60 % dans l'huile puis laisse 72 heures sous agitation à TA. On filtre un insoluble et concentre sous vide le filtrat. On reprend le résidu au cyclohexane, essore le précipité formé, le lave au cyclohexane et le sèche. On obtient 6,23 g du produit attendu.

B) Ester éthylique de l'acide 1-(*tert*-butoxycarbonyl)-5-(4-chlorophényl)-4-(bromométhyl)pyrazole-3-carboxylique.

A une solution de 6,2 g du composé obtenu à l'étape précédente dans 150 ml de $CCl_4$, on ajoute 3,18 g de N-bromosuccinimide et 0,02 g de peroxyde de dibenzoyle puis chauffe à reflux pendant 1 nuit. On filtre un insoluble et concentre sous vide le filtrat. On reprend le résidu au cyclohexane, décante et évapore sous vide le solvant. On obtient 8,72 g du produit attendu sous forme d'huile.

C) Ester éthylique de l'acide 5-(4-chlorophényl)-4-(éthoxyméthyl)pyrazole-3-carboxylique.

A 100 ml d'EtOH on ajoute 0,23 g de sodium, puis après dissolution on ajoute goutte à goutte 4,5 g du composé obtenu à l'étape précédente et chauffe à reflux pendant une nuit. On concentre sous vide et chromatographie le résidu sur silice en éluant par le mélange cyclohexane/AcOEt (60/40 ; v/v). On obtient 2,26 g du produit attendu.

D) Ester éthylique de l'acide 1-(3-chloro-4-méthylbenzyl)-5-(4-chlorophényl)-4-(éthoxyméthyl)pyrazole-3-carboxylique.

A une solution de 1,65 g du composé obtenu à l'étape précédente dans 50 ml de toluène, on ajoute à TA et par portions, 0,254 g d'hydrure de sodium à 60 % dans l'huile, puis chauffe à 65°C pendant 1 heure. Après refroidissement à TA, on ajoute goutte à goutte 1,7 g de iodure de 3-chloro-4-méthylbenzyle puis chauffe à reflux pendant 20 heures. Après refroidissement à TA, on filtre un insoluble et concentre sous vide le filtrat. On extrait le résidu à l'AcOEt, lave la phase organique par une solution saturée de $NH_4Cl$, par une solution tampon pH = 2, à l'eau, sèche sur $MgSO_4$ et évapore sous vide le solvant. On chromatographie le résidu sur silice en éluant par le mélange cyclohexane/AcOEt (75/25 ; v/v). On obtient 0,90 g du produit que l'on rechromatographie sur silice en éluant par le mélange cyclohexane/AcOEt (80/20 ; v/v). On obtient 0,24 g du produit attendu.

E) Acide 1-(4-chloro-3-méthylbenzyl)-5-(4-chlorophényl)-4-(éthoxyméthyl) pyrazole-3-carboxylique.

A une solution de 0,23 g du composé obtenu à l'étape précédente dans 10 ml d'EtOH, on ajoute à TA une solution de 0,088 g de KOH dans 10 ml d'eau puis chauffe à reflux pendant 3 heures. On concentre sous vide jusqu'à un volume de 10 ml, ajoute 10 ml d'eau, acidifie à pH = 2,5 par ajout d'une solution d'HCl 1N, essore le précipité formé et le sèche. On obtient 0,21 g du produit attendu.

Préparation 1.36

Acide 1-(5-chloroindan-1-yl)-5-(4-chlorophényl)pyrazole-3-carboxylique.

A une suspension de 2,5 g du composé obtenu à l'étape B de la Préparation 1.7 dans 20 ml de toluène on ajoute par portions 0,88 g d'hydrure de sodium à 60 % dans l'huile puis chauffe à 65°C pendant 1 heure. Après refroidissement à TA, on ajoute goutte à goutte une solution de 2,3 g de 1-bromo-5-chloroindane (Préparation 3.8) dans 10 ml de toluène et chauffe une nuit à reflux. On refroidit le mélange réactionnel à 5°C, ajoute goutte à goutte 20 ml d'une solution aqueuse à 50 % de $NH_4Cl$, décante et concentre sous vide la phase organique. On extrait le résidu à l'AcOEt, lave la phase organique par une solution saturée de NaCl, sèche sur $Na_2SO_4$ et évapore sous vide le solvant. On obtient 1,04 g du produit attendu utilisé tel quel.

Préparation 1.37

Acide 1-(4-méthoxybenzyl)-5-(4-chloro-3-méthylphényl)pyrazole-3-carboxylique.

A) Ester méthylique de l'acide 1-(4-méthoxybenzyl)-5-(4-chloro-3-méthylphényl) pyrazole-3-carboxylique.

A une suspension de 2,5 g du composé obtenu à l'étape B de la Préparation 1.10 dans 25 ml de toluène, on ajoute à TA et par portions, 0,53 g d'hydrure de sodium à 60 % dans l'huile, puis chauffe à 65°C pendant 1 heure. Après refroidissement à TA, on ajoute goutte à goutte une solution de 1,7 g de chlorure de 4-méthoxybenzyle dans 25 ml de toluène puis chauffe à reflux pendant 20 heures. Après refroidissement à TA, on ajoute 20 ml d'une solution aqueuse à 50 % de $NH_4Cl$, décante et évapore sous vide la phase organique. On extrait le résidu à l'AcOEt, lave la phase organique par une solution saturée de NaCl, sèche sur $MgSO_4$ et évapore sous vide le solvant. On chromatographie le résidu sur silice en éluant par le mélange cyclohexane/AcOEt (80/20 ; v/v). On obtient 1,03 g du produit attendu utilisé tel quel.

B) Acide 1-(4-méthoxybenzyl)-5-(4-chloro-3-méthylphényl)pyrazole-3-carboxylique.

A une solution de 1,03 g du composé obtenu à l'étape précédente dans 20 ml de MeOH, on ajoute à TA une solution de 0,23 g de KOH dans 20 ml d'eau puis chauffe à reflux pendant 3 heures. Après refroidissement à TA, on verse le mélange réactionnel dans un mélange HCl 1N/glace, extrait à l'éther, sèche la phase organique sur $Na_2SO_4$ et évapore sous vide le solvant. On obtient 0,8 g du produit attendu utilisé tel quel.

Préparation 1.38

Acide 1-(2,4-dichlorobenzyl)-3-(4-chlorophényl)pyrazole-5-carboxylique.

A) Ester méthylique de l'acide 1-(2,4-dichlorobenzyl)-3-(4-chlorophényl)pyrazole-5-carboxylique.

A une solution de 0,8 g du composé obtenu à l'étape B de la Préparation 1.7 dans 15 ml de DMF on ajoute à TA et par portions 0,16 g d'hydrure de sodium à 60 % dans l'huile et laisse 30 minutes sous agitation à TA. On refroidit à 0°C le mélange réactionnel, ajoute goutte à goutte une solution de 0,49 ml de chlorure de 2,4-dichlorobenzyle dans 5 ml de DMF puis laisse 16 heures sous agitation à TA. On verse le mélange réactionnel dans l'eau glacée, lave la phase aqueuse au DCM, essore le précipité blanc, le lave à l'eau et le sèche. On obtient 0,86 g du produit attendu, F = 120°C.

B) Acide 1-(2,4-dichlorobenzyl)-3-(4-chlorophényl)pyrazole-5-carboxylique.

A une solution de 0,85 g du composé obtenu à l'étape précédente dans 15 ml de MeOH, on ajoute une solution de 0,3 g de KOH dans 5 ml d'eau puis chauffe à reflux pendant 3 heures. On verse le mélange réactionnel dans 100 ml d'eau glacée, essore le précipité formé, le lave à l'eau et le sèche sous vide. On obtient 0,79 g du produit attendu, F = 218°C.

Préparation 2.1

Chlorhydrate de (1S)-2*endo*-amino-1,3,3-triméthylbicyclo[2.2.1]heptane.

A) (1S)-1,3,3-triméthylbicyclo[2.2.1]heptan-2-one-oxime.

A une solution de 38,1 g de (1S)-(+)-fenchone dans 120ml de MeOH on ajoute à TA une solution de 23 g de chlorhydrate d'hydroxylamine et 41 g d'acétate de sodium dans 200 ml d'eau puis chauffe à reflux pendant 48 heures. Après refroidissement à TA, on essore le précipité formé, le lave à l'eau et le sèche sous vide. On obtient 41 g du produit attendu.

B) Chlorhydrate de (1S)-2*endo*-amino-1,3,3-triméthylbicyclo[2.2.1]heptane.

On hydrogène dans un appareil de Parr, à TA et sous une pression de 6 bars pendant 48 heures, un mélange de 8 g du composé obtenu à l'étape précédente, 0,8 g d'oxyde de platine, dans 700 ml d'EtOH et 20 ml de chloroforme. On filtre le catalyseur sur Célite® et concentre sous vide le filtrat. On reprend le résidu à l'éther et essore le précipité formé. On obtient 2,61 g du produit attendu.

$\alpha_D^{20}$ = -4,6° (c = 1 ; EtOH)

Préparation 2.2

(1S)-2*endo,exo*-amino-1,3,3-triméthylbicyclo[2.2.1]heptane.

On refroidit à 10°C une solution de 10 g du composé obtenu à l'étape A de la Préparation 2.1 dans 70 ml d'AcOH, ajoute 25 g du nickel de Raney et laisse la température du mélange revenir à TA. Puis on hydrogène le mélange pendant 24 heures, à TA et à pression atmosphérique. On filtre le catalyseur sur Célite®, ajoute au filtrat un mélange de 100 ml d'eau et de glace, amène à pH = 7 par ajout d'une solution concentrée de NaOH, extrait à l'éther, sèche la phase organique sur $MgSO_4$ et évapore sous vide le solvant. On obtient 7,37 g du produit attendu sous forme d'huile (mélange endo, exo).

Préparation 2.3

(1R)-2*endo,exo*-amino-1,3,3-triméthylbicyclo[2.2.1]heptane et (1R)-2-imino-1,3,3-triméthylbicyclo[2.2.1]heptane.

A) (1R)-1,3,3-triméthylbicyclo[2.2.1]heptan-2-one-oxime.

On prépare ce composé selon le mode opératoire décrit à l'étape A de la Préparation 2.1 à partir de la (1R)-(-)-fenchone.

B) (1R)-2*endo,exo*-amino-1,3,3-triméthylbicyclo[2.2.1]heptane et (1R)-2-imino-1,3,3-triméthylbicyclo[2.2.1]heptane.

On refroidit à 10°C une solution de 14 g du composé obtenu à l'étape précédente dans 100 ml d'AcOH, ajoute 35 g de nickel de Raney et laisse la température du mélange revenir à TA. Puis on hydrogène le mélange pendant 24 heures, à TA et à pression atmosphérique. On filtre le catalyseur sur Célite®, ajoute au filtrat un mélange de 100 ml d'eau et de glace, amène à pH = 7 par ajout d'une solution concentrée de NaOH, extrait à l'éther, sèche la phase organique sur $MgSO_4$ et évapore sous vide le solvant. On obtient 10,77 g du mélange des produits attendus.

Préparation 2.4

Chlorhydrate de 2*exo*-(propylamino)-bicyclo[2.2.1]heptane.

A) 2*exo*-(propionylamino)-bicyclo[2.2.1]heptane.

On refroidit au bain de glace une solution de 15 g de 2*exo*-aminonorbornane et 20,5 ml de triéthylamine dans 80 ml de DCM, ajoute goutte à goutte une solution de 11,2 ml de chlorure de propionyle dans 80 ml de DCM et laisse une nuit sous agitation à TA. Après filtration du mélange réactionnel, on lave le filtrat par une solution aqueuse saturée de $NaHCO_3$, à l'eau, sèche la phase organique sur $MgSO_4$ et évapore sous vide le solvant. On obtient 23

g du produit attendu.

B) Chlorhydrate de 2*exo*-(propylamino)-bicyclo[2.2.1]heptane.

A une suspension de 7,6 g d'hydrure d'aluminium et de lithium dans 100 ml de THF, on ajoute goutte à goutte une solution de 23 g du composé obtenu à l'étape précédente dans 100 ml de THF, puis chauffe à reflux pendant 2 heures et laisse une nuit sous agitation à TA. On hydrolyse le mélange réactionnel par addition de 10 ml d'eau, puis 5 ml d'une solution à 15 % de NaOH et 14 ml d'eau. Après 15 minutes d'agitation, on filtre les sels minéraux et concentre sous vide le filtrat. On reprend l'huile obtenue dans l'éther iso, ajoute jusqu'à pH = 1 une solution d'éther chlorhydrique et essore le précipité formé. On reprend le précipité dans l'AcOEt, extrait à l'eau, alcalinise à pH = 12 la phase aqueuse par ajout d'une solution de NaOH 5N, extrait à l'AcOEt, lave la phase organique à l'eau, sèche sur $MgSO_4$ et évapore sous vide le solvant. On reprend le produit obtenu dans l'éther iso, ajoute jusqu'à pH = 1 une solution d'éther chlorhydrique et essore le précipité formé. On obtient 14 g du produit attendu, F = 230°C(déc).

Préparation 2.5

Chlorhydrate de 3*endo*-amino-bicyclo[3.2.1)octane.

On prépare ce composé selon le mode opératoire décrit par H. Maskill et coll., J. Chem. Soc. Perkin Trans II, 1984, 1369-1376.

Préparation 2.6

Chlorhydrate de (1R)-2*endo*-amino-1,3,3-triméthylbicyclo[2.2.1]heptane.

On hydrogène dans un appareil de Parr, à TA et sous une pression de 8 bars, un mélange de 15,5 g du composé obtenu à l'étape A de la Préparation 2.3 et 2 g d'oxyde de platine dans 500 ml d'EtOH et 14 ml de chloroforme. On filtre le catalyseur sur Célite® et concentre sous vide le filtrat. On reprend le résidu à l'éther chlorhydrique et évapore sous vide le solvant. On reprend le résidu dans l'hexane, filtre l'insoluble et concentre sous vide le solvant. On obtient 5,64 g d'huile qui cristallise dans le temps. On essore les cristaux formés, les reprend dans l'hexane, essore à nouveau et les lave. On obtient 0,85 g du produit attendu.
$\alpha_D^{20}$ = + 1°(c=1;EtOH).

Préparation 2.7

2,2,6,6-Tétraméthylcyclohexylamine.

A) 2,2,6,6-Tétraméthylcyclohexanone-oxime.

A une solution de 3,4 g de 2,2,6,6-tétraméthylcyclohexanone dans 20 ml de MeOH, on ajoute à TA une solution de 2,3 g de chlorhydrate d'hydroxylamine et 3,6 g d'acétate de sodium dans 20 ml d'eau puis chauffe à reflux pendant 48 heures. Après refroidissement à TA, on essore le précipité formé, le lave à l'eau et le sèche sous vide. On obtient 1,2 g du produit attendu.

B) 2,2,6,6-Tétraméthylcyclohexylamine.

On refroidit à 10°C, sous atmosphère d'azote, une solution de 1 g du composé obtenu à l'étape précédente dans 30 ml d'AcOH, ajoute 2,5 g de nickel de Raney et laisse la température remonter à TA. On hydrogène à TA et à pression atmosphérique pendant 24 heures le mélange ainsi obtenu. On filtre le catalyseur sur Célite®, ajoute au filtrat un mélange eau/glace, amène à pH = 7 par ajout d'une solution concentrée de NaOH, extrait à l'AcOEt, sèche la phase organique sur $Na_2SO_4$ et évapore sous vide le solvant. On obtient 0,63 g du produit attendu.

Préparation 3.1

Bromure de 3-fluoro-4-méthylbenzyle.

A) Ester éthylique de l'acide 3-fluoro-4-méthylbenzoïque.

On refroidit au bain de glace 150 ml d'EtOH, ajoute lentement 10 ml de chlorure de thionyle puis ajoute 10 g

d'acide 3-fluoro-4-méthylbenzoïque et laisse sous agitation en laissant remonter la température à TA. On chauffe à reflux pendant 2 heures le mélange réactionnel puis concentre sous vide. On obtient 10,45 g du produit attendu sous forme d'huile.

B) Alcool 3-fluoro-4-méthylbenzylique.

On refroidit à 0°C une suspension de 3,26 g d'hydrure d'aluminium et de lithium dans 100 ml de THF, ajoute goutte à goutte une solution de 10,45 g du composé obtenu à l'étape précédente dans 50 ml de THF et laisse sous agitation jusqu'à ce que la température soit remontée à TA. Puis on chauffe à reflux pendant 3 heures le mélange réactionnel ; on rajoute 1,5 g d'hydrure d'aluminium et de lithium et poursuit le reflux pendant 48 heures. Après refroidissement à TA, on hydrolyse le mélange réactionnel par ajout d'une solution aqueuse saturée de $NH_4Cl$ puis décante et conserve la phase organique. On extrait la phase aqueuse au THF puis sèche les phases organiques jointes sur $MgSO_4$ et évapore sous vide le solvant. On obtient 7,87 g du produit attendu sous forme d'huile.

C) Bromure de 3-fluoro-4-méthylbenzyle.

On chauffe à 100°C pendant 2 heures un mélange de 7,8 g du composé obtenu à l'étape précédente dans 112 ml d'une solution à 47 % d'HBr dans l'eau. Après refroidissement à TA, on extrait le mélange réactionnel à l'éther, sèche la phase organique sur $MgSO_4$ et évapore sous vide le solvant. On obtient 11,15 g du produit attendu sous forme d'huile que l'on utilise tel quel.

Préparation 3.2

Iodure de 3-chloro-4-méthylbenzyle.

A une solution de 9,6 g de chlorure de 3-chloro-4-méthylbenzyle dans 15 ml d'acétone, on ajoute à TA et goutte à goutte une solution de 8,5 g d'iodure de sodium dans 25 ml d'acétone et laisse 48 heures sous agitation à TA. On filtre le chlorure de sodium formé, sèche le filtrat sur $Na_2SO_4$ et évapore sous vide le solvant. On obtient 13 g du produit attendu sous forme d'huile que l'on utilise tel quel.

Préparation 3.3

Bromure de 3-chloro-4-fluorobenzyle.

A un mélange de 5 g de 3-chloro-4-fluorotoluène dans 100 ml de $CCl_4$, on ajoute 0,05 g de peroxyde de dibenzoyle puis 6,1 g de N-bromosuccinimide et chauffe à reflux pendant 12 heures. On filtre l'insoluble et le lave avec du $CCl_4$. On lave le filtrat à l'eau, par une solution saturée de NaCl, sèche la phase organique sur $Na_2SO_4$ et évapore sous vide le solvant. On obtient 7,5 g du produit attendu sous forme d'huile que l'on utilise tel quel.

Préparation 3.4

1-(1-bromoéthyl)-2,4-dichlorobenzène.

On refroidit à 0°C 15 ml d'une solution à 33 % d'HBr dans AcOH, ajoute goutte à goutte 2 ml de 2,4-dichloro-1-(1-hydroxyéthyl)benzène et laisse 30 minutes sous agitation à 0°C puis 3 heures à TA. On verse le mélange réactionnel dans de l'eau glacée, extrait à l'AcOEt, lave la phase organique par une solution aqueuse saturée de $NaHCO_3$, à l'eau, par une solution saturée de NaCl, sèche sur $MgSO_4$ et évapore sous vide le solvant. On obtient 3,4 g du produit attendu.

Préparation 3.5

Bromure de 4-éthylbenzyle.

On refroidit à 0°C 35 ml d'une solution à 33 % d'HBr dans AcOH, ajoute goutte à goutte 5 g d'alcool 4-éthylbenzylique et laisse 30 minutes sous agitation à 0°C puis une nuit à TA. On verse le mélange réactionnel dans 200 ml d'eau glacée, extrait à l'AcOEt, lave la phase organique par une solution aqueuse saturée de $NaHCO_3$, par une solution saturée de NaCl, sèche sur $Na_2SO_4$ et évapore sous vide le solvant. On obtient 5,6 g du produit attendu.

Préparation 3.6

1-(1-Bromoéthyl)-3,4-dichlorobenzène.

On prépare ce composé selon le mode opératoire décrit à la Préparation 3.4 à partir de 25 ml d'une solution à 33 % d'HBr dans AcOH et 5 g de 3,4-dichloro-1-(1-hydroxyéthyl)benzène. On obtient 6,4 g du produit attendu.

Préparation 3.7

1-(1-Bromoéthyl)-4-méthylbenzène.

On prépare ce composé selon le mode opératoire décrit à la Préparation 3.4 à partir de 30 ml d'une solution à 33 % d'HBr dans AcOH et 7 ml de 1-(1-hydroxyéthyl)-4-méthylbenzène. On obtient 9 g du produit attendu.

Préparation 3.8

1-Bromo-5-chloroindane.

A) 1-Hydroxy-5-chloroindane.

On refroidit à 0-5°C un mélange de 2,5 g de 5-chloroindan-1-one dans 30 ml de THF, ajoute 2,5 ml d'une solution concentrée de NaOH, puis par portions 0,88 g de borohydrure de sodium et laisse une nuit sous agitation à TA. On verse le mélange réactionnel dans 100 ml d'eau, acidifie à pH = 2 par ajout d'une solution concentrée d'HCl, extrait à l'AcOEt, lave la phase organique par une solution saturée de NaCl, sèche sur $Na_2SO_4$ et évapore sous vide le solvant. On chromatographie le résidu sur silice en éluant par le mélange cyclohexane/AcOEt (80/20 ; v/v). On obtient 2,1 g du produit attendu.

B) 1-Bromo-5-chloroindane.

On refroidit à 0°C 12 ml d'une solution à 33 % d'HBr dans AcOH, ajoute goutte à goutte 2 g du composé obtenu à l'étape précédente, laisse 30 minutes sous agitation à 0°C puis 3 heures à TA. On verse le mélange réactionnel dans 200 ml d'eau glacée, extrait à l'AcOEt, lave la phase organique par une solution saturée de $NaHCO_3$, par une solution saturée de NaCl, sèche sur $Na_2SO_4$ et évapore sous vide le solvant. On obtient 2,8 g du produit attendu.

EXEMPLE 1

N-[(1S)-1,3,3-triméthylbicyclo[2.2,1]hept-2*endo*-yl]-1-(3,4-dichlorobenzyl)-5-(4-méthylphényl)pyrazole-3-carboxamide.

A) Chlorure de l'acide 1-(3,4-dichlorobenzyl)-5-(4-méthylphényl)pyrazole-3-carboxylique.

A une solution de 5,1 g du composé obtenu à la Préparation 1.1 dans 50 ml de toluène, on ajoute à TA 1,52 ml de chlorure de thionyle puis chauffe à reflux pendant 4 heures et laisse une nuit sous agitation à TA. On concentre sous vide le mélange réactionnel, reprend le résidu à l'éther et évapore sous vide le solvant. On obtient 5,17 g du produit attendu sous forme d'huile.

B) N-[(1S)-1,3,3-triméthylbicyclo[2.2.1]hept-2*endo*-yl]-1-(3,4-dichlorobenzyl)-5-(4-méthylphényl)pyrazole-3-carboxamide.

A une solution de 0,38 g du composé obtenu à la Préparation 2.1 et 0,554 ml de triéthylamine dans 50 ml de DCM, on ajoute à TA 0,759 g du composé obtenu à l'étape précédente et laisse une nuit sous agitation à TA. On concentre sous vide le mélange réactionnel, extrait le résidu à l'AcOEt, lave la phase organique par une solution aqueuse saturée de $NaHCO_3$, par une solution tampon pH = 2, à l'eau, sèche sur $MgSO_4$ et évapore sous vide le solvant. On reprend le résidu à l'hexane, essore le précipité formé et le sèche. On obtient 0,67 g du produit attendu, F = 137°C.

$\alpha_D^{20}$ = + 1° (c = 1 ; EtOH)

EXEMPLE 2

N-[(1S)-1,3,3-triméthylbicyclo[2.2.1]hept-2*exo*-yl]-1-(3,4-dichlorobenzyl)-5-(4-méthylphényl)pyrazole-3-carboxamide.

A une solution de 0,5 g du composé obtenu à la Préparation 2.2 et 0,364 ml de triéthylamine dans 50 ml de DCM, on ajoute à TA 1 g du composé obtenu à l'étape A de l'EXEMPLE 1 et laisse une nuit sous agitation à TA. On concentre sous vide le mélange réactionnel, extrait le résidu à l'AcOEt, lave la phase organique par une solution aqueuse saturée de $NaHCO_3$, par une solution tampon pH = 2, à l'eau, sèche sur $MgSO_4$ et évapore sous vide le solvant. On chromatographie le résidu sur silice en éluant par le mélange cyclohexane/AcOEt (75/25 ; v/v). On sépare les deux isomères :

- le moins polaire, composé de l'EXEMPLE 1 ;
- le plus polaire, composé attendu : on obtient 0,08 g, F = 101°C.
  RMN : δ(ppm) : 0,6 à 2 : m : 16 H ; 2,3 : s : 3H ; 3,45 : d : 1H ; 5,4 : s : 2H ; 6,8 : s : 1H ; 6,9 : dd : 1H ; 7,1 à 7,4 : m : 6H ; 7,5 : d : 1H

EXEMPLE 3 et EXEMPLE 4

N-[(1R)-1,3,3-triméthylbicyclo[2.2.1]hept-2*endo*-yl]-1-(3,4-dichlorobenzyl)-5-(4-méthylphényl)pyrazole-3-carboxamide (exemple 3) et N-[(1R)-1,3,3-triméthylbicyclo[2.2.1]hept-2*exo*-yl]-1-(3,4-dichlorobenzyl)-5-(4-méthylphényl)pyrazole-3-carboxamide (exemple 4)

A une solution de 0,5 g du mélange des composés obtenus à la Préparation 2.3 et 0,364 g de triéthylamine dans 50 ml de DCM, on ajoute à TA 1 g du composé obtenu à l'étape A de l'EXEMPLE 1 et laisse une nuit sous agitation à TA. On concentre sous vide le mélange réactionnel, extrait le résidu à l'AcOEt, lave la phase organique par une solution aqueuse saturée de $NaHCO_3$, par une solution tampon pH = 2, à l'eau, sèche sur $MgSO_4$ et évapore sous vide le solvant. On chromatographie le résidu sur silice en éluant par le mélange cyclohexane/AcOEt (75/25 ; v/v). On sépare les différents composés :

- le moins polaire, composé de l'EXEMPLE 3 : on obtient 0,07 g, F = 130°C
  RMN : δ (ppm) : 0,6 à 1,9 : m : 16 H ; 2,3 : s : 3H ; 3,65 : d : 1H ; 5,4 : s : 2H ; 6,85 : s : 1H ; 6,9 : dd : 1H ; 7,0 : d : 1H ; 7,15 à 7,4 : m : 5H ; 7,5 : d : 1H
- le composé de l'EXEMPLE 4 : on obtient 0,09 g, F = 121°C
  RMN : δ (ppm) : 0,6 à 2 : m : 16H ; 2,3 : s : 3H ; 3,4 : d : 1H ; 5,4 : s : 2H ; 6,8 : s : 1H ; 6,9 : dd : 1H ; 7,1 à 7.4 : m : 5H ; 7,5 : d : 1H
- et le composé le plus polaire : le N-[(1R)-1,3,3-triméthylbicyclo[2.2.1]hept-2-ylidène]-1-(3,4-dichlorobenzyl)-5-(4-méthylphényl)pyrazolc-3-carboxamide; on obtient 0,54 g.

EXEMPLE 5

N-[bicyclo[2.2.1]hept-2*exo*-yl]-N-propyl-1-(3,4-dichlorobenzyl)-5-(4-méthylphényl)pyrazole-3-carboxamide.

A une solution de 0,266 g du composé obtenu à la Préparation 2.4 et 0,48 ml de triéthylamine dans 50 ml de DCM, on ajoute à TA 0,66 g du composé obtenu à l'étape A de l'EXEMPLE 1 et laisse une nuit sous agitation à TA. On concentre sous vide le mélange réactionnel, extrait le résidu à l'AcOEt, lave la phase organique par une solution aqueuse saturée de $NaHCO_3$, par une solution tampon pH = 2, à l'eau, sèche sur $MgSO_4$ et évapore sous vide le solvant. On chromatographie le résidu sur silice en éluant par le mélange cyclohexane/AcOEt (70/30 ; v/v). On obtient 0,5 g du produit attendu, F = 108°C.

EXEMPLE 6

N-[(1S)-1,3,3-triméthylbicyclo[2.2.1]hept-2*endo*-yl]-1-(3-fluoro-4-méthylbenzyl)-5-(4-méthylphényl)pyrazole-3-carboxamide.

A) Chlorure de l'acide 1-(3-fluoro-4-méthylbenzyl)-5-(4-méthylphényl)pyrazole-3-carboxylique et chlorure de l'acide 1-(3-fluoro-4-méthylbenzyl)-3-(4-méthylphényl)pyrazole-5-carboxylique.

A une solution de 0,9 g du mélange des composés obtenus à la Préparation 1.3 dans 50 ml de toluène, on ajoute à TA 0,3 ml de chlorure de thionyle puis chauffe à reflux pendant 5 heures et laisse une nuit sous agitation à TA. On concentre sous vide le mélange réactionnel, reprend le résidu à l'éther et évapore sous vide le solvant. On obtient 1 g du mélange des produits attendus.

B) N-[(1S)-1,3,3-triméthylbicyclo[2.2.1]hept-2*endo*-yl]-1-(3-fluoro-4-méthylbenzyl)-5-(4-méthylphényl)pyrazole-3-carboxamide.

A une solution de 0,33 g du composé obtenu à la Préparation 2.1 et 0,48 ml de triéthylamine dans 50 ml de DCM, on ajoute à TA 0,63 g du mélange des composés obtenus à l'étape précédente et laisse 48 heures sous agitation à TA. On concentre sous vide le mélange réactionnel, extrait le résidu à l'AcOEt, lave la phase organique par une solution aqueuse saturée de $NaHCO_3$, par une solution tampon pH = 2, à l'eau, sèche sur $MgSO_4$ et évapore sous vide le solvant. On chromatographie le résidu sur silice en éluant par le mélange cyclohexane/AcOEt (75/25 ; v/v). On sépare deux composés :

- le moins polaire, composé de l'EXEMPLE 6 : on obtient 0,5 g, F = 53°C.
  $\alpha_D^{20}$ = - 3,3° (c = 1 ; EtOH)
- le plus polaire : le N-[(1S)-1,3,3-triméthylbicyclo[2.2,1]hept-2*endo*-yl]-1-(3-fluoro-4-méthylbenzyl)-3-(4-méthylphényl)pyrazolc-5-carboxamide.

EXEMPLE 7

N-[(1S)-1,3,3-triméthylbicyclo[2.2.1]hept-2*endo*-yl]-1-(3-chloro-4-méthylbenzyl)-5-(4-méthoxyphényl)pyrazole-3-carboxamide.

A) Chlorure de l'acide 1-(3-chloro-4-méthylbenzyl)-5-(4-méthoxyphényl)pyrazole-3-carboxylique et chlorure de l'acide 1-(3-chloro-4-méthylbenzyl)-3-(4-méthoxyphényl)pyrazole-5-carboxylique

On prépare le mélange de ces deux composés selon le mode opératoire décrit à l'étape A de l'EXEMPLE 6 à partir de 1,7 g du mélange des composés obtenus à la Préparation 1.5, 0,54 ml de chlorure de thionyle et 50 ml de toluène. On obtient 1,77 g du mélange des produits attendus.

B) N-[(1S)-1,3,3-triméthylbicyclo[2.2.1]hept-2*endo*-yl]-1-(3-chloro-4-méthylbenzyl)-5-(4-méthoxyphényl)pyrazole-3-carboxamide.

On prépare ce composé selon le mode opératoire décrit à l'étape B de l'EXEMPLE 6 à partir de 0,531 g du composé obtenu à la Préparation 2.1, 0,775 ml de triéthylamine, 100 ml de DCM et 1 g du mélange des composés obtenus à l'étape précédente. On chromatographie sur silice en éluant par le mélange cyclohexane/AcOEt (75/25 ; v/v). On sépare deux composés :

- le moins polaire, composé de l'EXEMPLE 7 : on obtient 0,77 g, F = 116°C.
  $\alpha_D^{20}$ = - 1,1° (c = 1 ; EtOH)
- le plus polaire : le N-[(1S)-1,3,3-triméthylbicyclo[2.2.1]hept-2*endo*-yl]-1-(3-chloro-4-méthylbenzyl)-3-(4-méthoxyphényl)pyrazole-5-carboxamide.

EXEMPLE 8

N-(adamant-2-yl)-1-(2,4-dichlorobenzyl)-5-(4-chlorophényl)pyrazole-3-carboxamide.

A) Chlorure de l'acide 1-(2,4-dichlorobenzyl)-5-(4-chlorophényl)pyrazole-3-carboxylique.

On prépare ce composé selon le mode opératoire décrit à l'étape A de l'EXEMPLE 1 à partir de 3,27 g du composé obtenu à la Préparation 1.7, 2,2 ml de chlorure de thionyle et 60 ml de toluène. On obtient 3,25 g du produit attendu que l'on utilise tel quel.

B) N-(adamant-2-yl)-1-(2,4-dichlorobenzyl)-5-(4-chlorophényl)pyrazole-3-carboxamide.

On refroidit à 0°C une solution de 0,39 g de chlorhydrate de 2-aminoadamantane et 0,58 ml de triéthylamine dans 15 ml de DCM, ajoute goutte à goutte une solution de 0,8 g du composé obtenu à l'étape précédente dans 15 ml de DCM, et laisse 16 heures sous agitation à TA. On verse le mélange réactionnel sur 50 ml d'eau glacée, après décantation lave la phase organique à l'eau, par une solution saturée de NaCl, sèche sur $MgSO_4$ et évapore sous vide le solvant. On obtient 0,42 g du produit attendu après cristallisation puis recristallisation dans le mélange

DCM/éther iso, F = 154°C.

EXEMPLE 9

N-[*endo*-bicyclo[3.2.1 ]oct-3-yl]-1-(2,4-dichlorobenzyl)-5-(4-chlorophényl) pyrazole-3-carboxamide.

On prépare ce composé selon le mode opératoire décrit à l'étape B de l'EXEMPLE 8 à partir de 0,17 g du composé obtenu à la Préparation 2.5, 0,3 ml de triéthylamine dans 10 ml de DCM et 0,41 g du composé obtenu à l'étape A de l'EXEMPLE 8 dans 10 ml de DCM. On purifie le produit obtenu par chromatographie sur silice en éluant par le gradient du mélange toluène/AcOEt (97/3 ; v/v) à (95/5 ; v/v). On obtient 0,43 g du produit attendu, F = 130°C.

EXEMPLE 10

N-[(1S)-1,3,3-triméthylbicyclo[2.2.1]hept-2*endo*-yl]-1-[(1R,S)-1-(2,4-dichlorophényl)éthyl]-5-(4-chlorophényl)pyrazole-3-carboxamide.

A) Chlorure de l'acide 1-[1-(2,4-dichlorophényl)éthyl]-5-(4-chlorophényl)pyrazole-3-carboxylique.

On prépare ce composé selon le mode opératoire décrit à l'étape A de l'EXEMPLE 1 à partir de 0,68 g du composé obtenu à la Préparation 1.14, 0,45 ml de chlorure de thionyle et 30 ml de toluène. On obtient 0,86 g du produit attendu.

B) N-[(1S)-1,3,3-triméthylbicyclo[2.2.1]hept-2*endo*-yl]-1-[(1R,S)-1-(2,4-dichlorophényl)éthyl]-5-(4-chlorophényl) pyrazole-3-carboxamide.

On prépare ce composé selon le mode opératoire décrit à l'étape B de l'EXEMPLE 8, à partir de 0,34 g du composé obtenu à la Préparation 2.1 et 0,5 ml de triéthylamine dans 15 ml de DCM et 0,72 g du composé obtenu à l'étape précédente dans 15 ml de DCM. On purifie le produit obtenu par chromatographie sur silice en éluant par le mélange toluène/AcOEt (96/4 ; v/v). On obtient 0,84 g du produit attendu, F = 70°C.

EXEMPLE 11

N-[(1S)-1,3,3-triméthylbicyclo[2.2.1]hept-2*endo*-yl]-1-(3,4-dichlorobenzyl)-5-(2,6-diméthoxyphényl)pyrazole-3-carboxamide.

A) Chlorure de l'acide 1-(3,4-dichlorobenzyl)-5-(2,6-diméthoxyphényl)pyrazole-3-carboxylique.

On prépare ce composé selon le mode opératoire décrit à l'étape A de l'EXEMPLE 1 à partir de 1,1 g du composé obtenu à la Préparation 1.15, 0,6 ml de chlorure de thionyle et 25 ml de toluène. On obtient 1 g du produit attendu.

B) N-[(1S)-1,3,3-triméthylbicyclo[2.2.1]hept-2*endo*-yl]-1-(3,4-dichlorobenzyl)-5-(2,6-diméthoxyphényl)pyrazole-3-carboxamide.

On prépare ce composé selon le mode opératoire décrit à l'étape B de l'EXEMPLE 1 à partir de 0,303 g du composé obtenu à la Préparation 2.1 et 0,4 ml de triéthylamine dans 15 ml de DCM, 0,6 g du composé obtenu à l'étape précédente dans 15 ml de DCM. On purifie par cristallisation dans le mélange cyclohexane/AcOEt (75/25 ; v/v). On obtient 0,53 g du produit attendu.

$\alpha_D^{20}$ = + 1,2° (c = 1 ; EtOH)

En procédant selon les modes opératoires décrits dans les EXEMPLES précédents, à partir des chlorures d'acides appropriés eux-mêmes obtenus à partir des acides décrits dans les Préparations, et du composé obtenu à la Préparation 2.1, on prépare les composés selon l'invention rassemblés dans le TABLEAU 2 ci-après.

TABLEAU 2

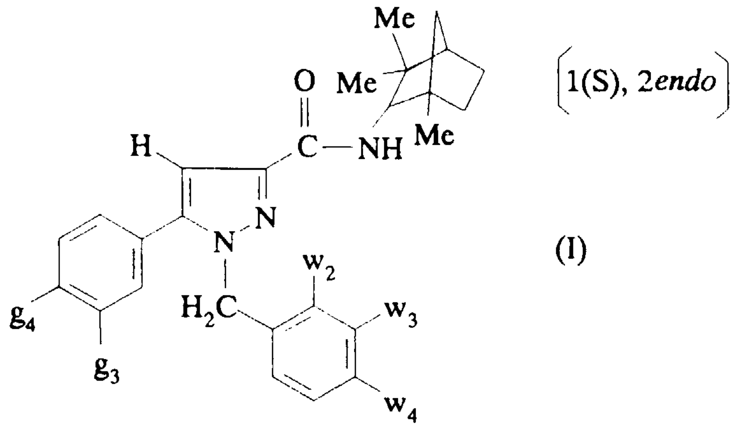

(I)

| Exemples | g3 | g4 | w2 | w3 | w4 | F°C | $[\alpha]_D^{20}$ (c = 1 ; EtOH) |
|---|---|---|---|---|---|---|---|
| 12 (a) | H | Me | H | Cl | Me | 116 | −3,3° |
| 13 (a) | H | OMe | H | Cl | Cl | 55 | – |
| 14 (a) | H | F | H | Cl | Me | 114 | −1,7° |
| 15 (b) | H | Cl | Cl | H | Cl | – | +0,6° |
| 16 (c) | Me | Me | H | Cl | Me | 65 | −1,5° |
| 17 (a) | Me | Me | H | Cl | F | 138 | −1,5° |
| 18 (a) | Me | Cl | H | H | Me | 59 | −3,1° |
| 19 (a) | Me | Cl | H | H | F | 158 | −2,4° |
| 20 (a) | Me | Cl | H | Cl | Me | 74 | −2,4° |
| 21 (a) | Me | Cl | H | Cl | F | 125 | −2,5° |

| | | | | | | | |
|---|---|---|---|---|---|---|---|
| 22 (d) | Me | Me | H | H | F | 175 | −3,3°<br>(c = 1 ; DMF) |
| 23 (e) | H | Me | Cl | H | Cl | 140 | +0,5° |
| 24 (f) | Me | Cl | H | H | Et | 80 | −2,1° |
| 25 (g) | Me | Cl | H | Cl | Cl | 112 | +0,4° |
| 26 (f) | Me | Cl | Cl | H | Cl | 64 | +1° |
| 27 (f) | Cl | Cl | H | H | Me | 110 | −1,1° |
| 28 (f) | Cl | Cl | H | Cl | Me | 49 | −1,2° |
| 29 (f) | H | SMe | Cl | H | Cl | 128,2 | +1,4° |
| 30 (f) | H | $CF_3$ | Cl | H | Cl | 113,8 | +1,3° |
| 31 (f) | Me | Cl | H | H | OMe | 115 | +2,3° |

(a) On prépare ce composé selon les modes opératoires décrits à l'EXEMPLE 1 étape A puis étape B. Le produit est purifié par chromatographie sur silice en éluant par le mélange cyclohexane/AcOEt (75/25 ; v/v).

(b) On prépare ce composé selon les modes opératoires décrits à l'EXEMPLE 8 étape A puis étape B. Le produit est purifié par chromatographie sur silice en éluant par le mélange toluène/AcOEt (90/10 ; v/v).

(c) On prépare ce composé selon les modes opératoires décrits à l'EXEMPLE 1 étape A puis étape B. Le produit est purifié par chromatographie sur silice en éluant par le mélange cyclohexane/AcOEt (80/20 ; v/v).

(d) On prépare ce composé selon les modes opératoires décrits à l'EXEMPLE 1 étape A puis étape B. Le produit est purifié par cristallisation dans l'AcOEt.

(e) On prépare ce composé selon les modes opératoires décrits à l'EXEMPLE 1 étape A puis étape B. Le produit est purifié par cristallisation dans l'éther iso.

(f) On prépare ce composé selon les modes opératoires décrits à l'EXEMPLE 32 étape A puis étape B en utilisant le composé obtenu à la Préparation 2.1. Le produit est purifié par chromatographie sur silice en éluant par le mélange cyclohexane/AcOEt (80/20 ; v/v).

(g) On prépare ce composé selon les modes opératoires décrits à l'EXEMPLE 32 étape A puis étape B en utilisant le composé obtenu à la Préparation 2.1. Après séchage sur $Na_2SO_4$, on concentre en partie le solvant, essore le précipité formé, le lave à l'éther iso et le sèche.

EXEMPLE 32

N-[(1R)-1,3,3-triméthylbicyclo[2.2.1]hept-2*endo*-yl]-1-(4-méthylbenzyl)-5-(4-chloro-3-méthylphényl)pyrazolc-3-carboxamide.

A) Chlorure de l'acide 1-(4-méthylbenzyl)-5-(4-chloro-3-méthylphényl)pyrazole-3-carboxylique.

On prépare ce composé selon le mode opératoire décrit à l'étape A de l'EXEMPLE 1 à partir de 2,8 g du composé obtenu à la Préparation 1.10, 1,7 ml de chlorure de thionyle et 50 ml de toluène. On obtient 2,9 g du produit attendu.

B) N-[(1R)-1,3,3-triméthylbicyclo[2.2.1]hept-2*endo*-yl]-1-(4-méthylbenzyl)-5-(4-chloro-3-méthylphényl)pyrazole-3-carboxamide.

A une solution de 0,765 g du composé obtenu à la Préparation 2.6 et 1 ml de triéthylamine dans 20 ml de DCM, on ajoute à TA et goutte à goutte une solution de 1,6 g du composé obtenu à l'étape précédente dans 20 ml de DCM et laisse une nuit sous agitation à TA. On concentre sous vide, reprend le résidu à l'AcOEt, filtre un insoluble, lave le filtrat par une solution aqueuse saturée de $NaHCO_3$, par une solution tampon pH = 2, par une solution aqueuse saturée de NaCl, sèche sur $Na_2SO_4$ et évapore sous vide le solvant. On chromatographie le résidu sur silice en éluant par le mélange cyclohexane/AcOEt (75/25 ; v/v). On obtient 1,08 g du produit attendu, F = 108°C.

$\alpha_D^{20}$ = + 0,3° (c = 1 ; EtOH).

EXEMPLE 33

N-[(1R)-1,3,3-triméthylbicyclo[2.2.1]hept-2*exo*-yl]-1-(4-méthylbenzyl)-5-(4-chloro-3-méthylphényl)pyrazole-3-carboxamide.

A une solution de 0,4 g du mélange des composés obtenus à la Préparation 2.3 dans 10 ml de DCM, on ajoute 0,6 ml de triéthylamine puis goutte à goutte une solution de 0,66 g du composé obtenu à l'étape A de l'EXEMPLE 32 dans 10 ml de DCM et laisse une nuit sous agitation à TA. On concentre sous vide, extrait le résidu à l'AcOEt, filtre un insoluble, lave deux fois le filtrat par une solution aqueuse saturée de $NaHCO_3$, deux fois par une solution tampon pH = 2, deux fois par une solution aqueuse saturée de NaCl, sèche sur $Na_2SO_4$ et évapore sous vide le solvant. On chromatographie le résidu sur silice en éluant par le mélange cyclohexane/AcOEt (80/20 ; v/v). On obtient un mélange contenant 58 % du composé de l'EXEMPLE 33 (forme *exo*) et 41,6 % du composé de l'EXEMPLE 32 (forme *endo)* (détermination par HPLC analytique). On sépare les deux isomères par HPLC préparative : échantillon à purifier de 0,31 g solubilisé dans 13 ml d'éluant de départ (A/B : 10 %/90 %) additionnés de 9 ml de MeOH et de 4 ml d'acétonitrile. On obtient après lyophilisation :

- composé de l'EXEMPLE 33 : m = 0,084 g ; pureté de 100 % (HPLC analytique) avec un TR = 41 mn.
  $\alpha_D^{20}$ = -8,8° (c = 1 ; EtOH).
  RMN : δ (ppm) : 0,8 à 1,15 : 3s : 9H ; 1,2 à 2 : m : 7H ; 2,2 à 2,25 : m : 6H ; 3,5 : d: 1H ; 5,4 : s : 2H; 6,8 à 7,7 : m : 9H.
- composé de l'EXEMPLE 32 : m = 0,056 g ; pureté de 98 % (HPLC analytique) avec un TR = 43,9 mn.

EXEMPLE 34

N-[(1S)-1,3,3-triméthylbicyclo[2.2.1]hept-2*exo*-yl]-1-(4-méthylbenzyl)-5-(4-chloro-3-méthylphényl)pyrazole-3-carboxamide.

A une solution de 0,853 g du composé obtenu à la Préparation 2.2 dans 20 ml de DCM, on ajoute 1,5 ml de triéthylamine puis goutte à goutte une solution de 1,6 g du composé obtenu à l'étape A de l'EXEMPLE 32 dans 20 ml de DCM et laisse une nuit sous agitation à TA. On concentre sous vide, extrait le résidu à l'AcOEt, filtre un insoluble, lave deux fois le filtrat par une solution aqueuse saturée de $NaHCO_3$, deux fois par une solution tampon pH = 2, deux fois par une solution aqueuse saturée de NaCl, sèche sur $Na_2SO_4$ et évapore sous vide le solvant. On chromatographie le résidu sur silice en éluant par le mélange cyclohexane/AcOEt (80/20 ; v/v).On obtient un mélange contenant 58 % du composé de l'EXEMPLE 34 (forme exo) et 37 % du composé de l'EXEMPLE 18 (forme *endo)* (détermination par HPLC analytique). On sépare les deux isomères par HPLC préparative : échantillon à purifier de 0,23 g solubilisé dans 13 ml d'éluant de départ (A/B : 10 %/90 %) additionnés de 3 ml de MeOH et de 9 ml d'acétonitrile. On obtient après

lyophilisation :

- composé de l'EXEMPLE 34 : m = 0,16 g ; pureté de 99,6 % (HPLC analytique) avec un TR =34,7 mn ; F = 49°C. $\alpha_D^{20}$ = -6,8° (c = 1 ; EtOH).
  RMN : δ (ppm) : 0,8 à 1,15 : 3s : 9H ; 1,2 à 2 : m : 7H ; 2,1 à 2,4 : m : 6H ; 3,5 : d: 1H ; 5,4 : s : 2H ; 6,8 à 7,6 : m : 9H.
- composé de l'EXEMPLE 18 : m = 0,066 g ; pureté de 94,7 % (HPLC analytique) avec un TR = 37,2 mn.

EXEMPLE 35

N-(2,2,6,6-Tétraméthylcyclohex-1-yl)-1-(4-méthylbenzyl)-5-(4-chloro-3-méthylphényl)pyrazole-3-carboxamide.

A une solution de 0,25 g du composé obtenu à la Préparation 2.7 dans 15 ml de DCM, on ajoute 0,4 ml de triéthylamine puis goutte à goutte une solution de 0,55 g du composé obtenu à l'étape A de l'EXEMPLE 32 dans 15 ml de DCM et laisse une nuit sous agitation à TA. On concentre sous vide, extrait le résidu à l'AcOEt, lave deux fois le filtrat par une solution aqueuse saturée de $NaHCO_3$, deux fois par une solution tampon pH = 2, deux fois par une solution aqueuse saturée de NaCl, sèche sur $Na_2SO_4$ et évapore sous vide le solvant. On chromatographie le résidu sur silice en éluant par le mélange cyclohexane/AcOEt (80/20 ; v/v). On obtient 0,1 g du produit attendu.

EXEMPLE 36

N-[bicyclo[2.2.1]hept-2*exo*-yl]-1-(3-chloro-4-méthylbenzyl)-5-(3,4-dichlorophényl)pyrazole-3-carboxamide.

A) Chlorure de l'acide 1-(3-chloro-4-méthylbenzyl)-5-(3,4-dichlorophényl)pyrazole-3-carboxylique.
On prépare ce composé selon le mode opératoire décrit à l'étape A de l'EXEMPLE 1 à partir de 10 g du composé obtenu à la Préparation 1.22, 5,5 ml de chlorure de thionyle et 125 ml de toluène. On obtient 9,41 g du produit attendu.

B) N-[bicyclo[2.2.1]hept-2*exo*-yl]-1-(3-chloro-4-méthylbenzyl)-5-(3,4-dichlorophényl)pyrazole-3-carboxamide.
A une solution de 0,411 g de 2*exo*-aminonorbornane et 1 ml de triéthylamine dans 15 ml de DCM, on ajoute à TA une solution de 1,5 g du composé obtenu à l'étape précédente dans 15 ml de DCM et laisse une nuit sous agitation à TA. On concentre sous vide le mélange réactionnel, extrait le résidu à l'AcOEt, filtre un insoluble, lave la phase organique par une solution aqueuse saturée de $NaHCO_3$, par une solution tampon pH = 2, à l'eau, sèche sur $MgSO_4$ et évapore sous vide le solvant. On chromatographie le résidu sur silice en éluant par le mélange cyclohexane/AcOEt (80/20 ; v/v). On obtient 0,5 g du produit attendu, F = 145,6°C.

EXEMPLE 37

N-[bicyclo[2.2.1]hept-2*exo*-yl]-N-propyl-1-(3-chloro-4-méthylbenzyl)-5-(3,4-dichlorophényl)pyrazole-3-carboxamide.

On prépare ce composé selon le mode opératoire décrit à l'étape B de l'EXEMPLE 36 à partir de 0,695 g du composé obtenu à la Préparation 2.4, 1 ml de triéthylamine dans 15 ml de DCM et 1,5 g du composé obtenu à l'étape A de l'EXEMPLE 36 dans 15 ml de DCM. On obtient 0,417 g du produit attendu.

EXEMPLE 38

N-(2-méthylcyclohex-1-yl)-1-(3-chloro-4-méthylbenzyl)-5-(3,4-dichlorophényl)pyrazole-3-carboxamide.

On prépare ce composé selon le mode opératoire décrit à l'étape B de l'EXEMPLE 36 à partir de 1 ml de 2-méthylcyclohexylamine, 1 ml de triéthylamine dans 15 ml de DCM et 1,5 g du composé obtenu à l'étape A de l'EXEMPLE 36 dans 15 ml de DCM. On purifie le composé par chromatographie sur silice en éluant par le mélange DCM/MeOH (98/2 ; v/v). On obtient 0,27 g du produit attendu, F = 165°C.

EXEMPLE 39

N-(2,6-diméthylcyclohex-1-yl)-1-(3-chloro-4-méthylbenzyl)-5-(3,4-dichlorophényl) pyrazole-3-carboxamide.

On prépare ce composé selon le mode opératoire décrit à l'étape B de l'EXEMPLE 36 à partir de 0,605 g de

chlorhydrate de 2,6-diméthylcyclohexylamine, 1 ml de triéthylamine dans 15 ml de DCM et 1,5 g du composé obtenu à l'étape A de l'EXEMPLE 36 dans 15 ml de DCM. On obtient 0,6 g du composé attendu, F = 59,9°C.

EXEMPLE 40

N-[(1S)-1,3,3-triméthylbicyclo[2.2.1]hept-2*endo*-yl]-1-[(1R,S)-1-(3,4-dichlorophényl)éthyl]-5-(4-méthylphényl)pyrazole-3-carboxamide.

A) Chlorure de l'acide 1-[1-(3,4-dichlorophényl)éthyl]-5-(4-méthylphényl)pyrazole-3-carboxylique.

A une suspension de 1,2 g du composé obtenu à la Préparation 1.25 dans 50 ml de toluène, on ajoute à TA 0,7 ml de chlorure de thionyle puis chauffe à reflux pendant 2 heures. Après refroidissement à TA, on concentre sous vide le mélange réactionnel, reprend le résidu au toluène et évapore sous vide le solvant. On obtient 1,3 g du produit attendu.

B) N-[(1S)-1,3,3-triméthylbicyclo[2.2.1]hept-2*endo*-yl]-1-[(1R,S)-1-(3,4-dichlorophényl)éthyl]-5-(4-méthylphényl) pyrazole-3-carboxamide.

A une solution de 0,341 g du composé obtenu à la Préparation 2.1 dans 15 ml de DCM, on ajoute 0,6 ml de triéthylamine puis goutte à goutte, une solution de 0,7 g du composé obtenu à l'étape précédente dans 15 ml de DCM et laisse une nuit sous agitation à TA. On concentre sous vide, extrait le résidu à l'AcOEt, filtre un insoluble, lave le filtrat par une solution aqueuse saturée de $NaHCO_3$, par une solution tampon pH = 2, par une solution aqueuse saturée de NaCl, sèche sur $Na_2SO_4$ et évapore sous vide le solvant. On chromatographie le résidu sur silice en éluant par le mélange cyclohexane/AcOEt (80/20 ; v/v). On obtient 0,54 g du produit attendu, F = 112°C.

RMN : δ (ppm) : 0,6 à 1,9 : m : 19H ; 2,3 : s : 3H ; 3,6 : mt : 1H ; 5,55 : qd : 1H ; 6,6 à 7,6 : m : 9H.

EXEMPLE 41

N-[(1S)-1,3,3-triméthylbicyclo[2.2.1]hept-2*endo*-yl]-1-[(1R,S)-1-(4-méthylphényl)éthyl]-5-(4-chloro-3-méthylphényl) pyrazole-3-carboxamide.

A) Chlorure de l'acide 1-[1-(4-méthylphényl)éthyl]-5-(4-chloro-3-méthylphényl)pyrazole-3-carboxylique.

On prépare ce composé selon le mode opératoire décrit à l'étape A de l'EXEMPLE 40 à partir de 0,61 g du composé obtenu à la Préparation 1.26 dans 30 ml de toluène et 0,4 ml de chlorure de thionyle. On obtient 0,7 g du produit attendu.

B) N-[(1S)-1,3,3-triméthylbicyclo[2.2.1]hept-2*endo*-yl]-1-[(1R,S)-1-(4-méthylphényl)éthyl]-5-(4-chloro-3-méthylphényl)pyrazole-3-carboxamide.

On prépare ce composé selon le mode opératoire décrit à l'étape B de l'EXEMPLE 40 à partir de 0,36 g du composé obtenu à la Préparation 2.1 dans 15 ml de DCM, 0,45 g de triéthylamine et une solution de 0,7 g du composé obtenu à l'étape précédente dans 15 ml de DCM. On purifie le composé par chromatographie sur silice en éluant par le mélange cyclohexane/AcOEt (75/25 ; v/v). On obtient 0,13 g du produit attendu, F = 65°C.

RMN : δ (ppm) : 0,8 à 2,05 : m : 19H ; 2,2 à 2,5 : m : 6H ; 3,75 : d : 1H ; 5,65 : qd : 1H ; 6,8 à 7,7 : m : 9H.

EXEMPLE 42

N-[(1S)-1,3,3-triméthylbicyclo[2.2,1]hept-2*endo*-yl]-1-[(1R,S)-1-(3,4-dichlorophényl)éthyl]-5-(4-chloro-3-méthylphényl)pyrazole-3-carboxamide.

A) Chlorure de l'acide 1-[1-(3,4-dichlorophényl)éthyl]-5-(4-chloro-3-méthylphényl) pyrazole-3-carboxylique.

On prépare ce composé selon le mode opératoire décrit à l'étape A de l'EXEMPLE 40 à partir de 0,9 g du composé obtenu à la Préparation 1.27 dans 20 ml de toluène et 0,5 ml de chlorure de thionyle. On obtient 0,95 g du produit attendu.

B) N-[(1S)-1,3,3-triméthylbicyclo[2.2.1]hept-2*endo*-yl]-1-[(1R,S)-1-(3,4-dichlorophényl)éthyl]-5-(4-chloro-3-méthylphényl)pyrazole-3-carboxamide.

On prépare ce composé selon le mode opératoire décrit à l'étape B de l'EXEMPLE 40 à partir de 0,455 g du

composé obtenu à la Préparation 2.1 dans 15 ml de DCM, 0,8 g de triéthylamine et une solution de 0,95 g du composé obtenu à l'étape précédente dans 15 ml de DCM. Après séchage de la phase organique sur $Na_2SO_4$ on concentre partiellement sous vide le solvant et essore les cristaux formés. On obtient 0,76 g du produit attendu, F = 156°C.

RMN : δ (ppm) : 0,6 à 2 : m : 19H ; 2,35 : s : 3H ; 3,7 : mt : 1H ; 5,7 : qd : 1H ; 6,7 à 7,7 m : 8H.

EXEMPLE 43

N-[(1S)-1,3,3-triméthylbicyclo[2.2.1]hept-2*endo*-yl]-1-[(1R,S)-1-(4-méthylphényl)propyl]-5-(4-chloro-3-méthylphényl) pyrazole-3-carboxamide.

A) Chlorure de l'acide 1-[1-(4-méthylphényl)propyl]-5-(4-chloro-3-méthylphényl) pyrazole-3-carboxylique.

On prépare ce composé selon le mode opératoire décrit à l'étape A de l'EXEMPLE 40 à partir de 0,87 g du composé obtenu à la Préparation 1.28 dans 50 ml de toluène et 0,26 ml de chlorure de thionyle. On obtient 0,89 g du produit attendu.

B) N-[(1S)-1,3,3-triméthylbicyclo[2.2.1]hept-2*endo*-yl]-1-[(lR,S)-1-(4-méthylphényl) propyl]-5-(4-chloro-3-méthylphényl)pyrazole-3-carboxamide.

A une solution de 0,51 g du composé obtenu à l'étape précédente et 0,25 g du composé obtenu à la Préparation 2.1 dans 50 ml de DCM, on ajoute 0,363 ml de triéthylamine et laisse 72 heures sous agitation à TA. On concentre sous vide, extrait le résidu à l'AcOEt, filtre un insoluble, lave le filtrat par une solution aqueuse saturée de $NaHCO_3$, par une solution tampon pH = 2, par une solution saturée de NaCl, sèche sur $Na_2SO_4$ et évapore sous vide le solvant. On chromatographie le résidu sur silice en éluant par le mélange cyclohexane/AcOEt (75/25 ; v/v). On obtient 0,44 g du produit attendu, F = 136°C.

RMN : δ (ppm) : 0,5 à 2,6 : m : 27H ; 3,75 : d : 1H; 4,95 : dd : 1H ; 6,6 à 7,4 : m : 9H.

EXEMPLE 44

N-[(1S)-1,3,3-triméthylbicyclo[2.2.1]hept-2*endo*-yl]-1-[1-méthyl-1-(4-méthylphényl)éthyl]-5-(4-chloro-3-méthylphényl) pyrazole-3-carboxamide.

A) Chlorure de l'acide 1-[1-méthyl-1-(4-méthylphényl)éthyl]-5-(4-chloro-3-méthyl phényl)pyrazole-3-carboxylique.

On prépare ce composé selon le mode opératoire à l'étape A de l'EXEMPLE 40 à partir de 1,5 g du composé obtenu à la Préparation 1.29 dans 100 ml de toluène et 0,44 ml de chlorure de thionyle. On obtient 1,55 g du produit attendu.

B) N-[(1S)-1,3,3-triméthylbicyclo[2.2.1 ]hept-2*endo*-yl]-1-[1-méthyl-1-(4-méthylphényl)éthyl]-5-(4-chloro-3-méthylphényl)pyrazole-3-carboxamide.

A une solution de 0,59 g du composé obtenu à l'étape précédente et 0,29 g du composé obtenu à la Préparation 2.1 dans 50 ml de DCM, on ajoute 0,424 ml de triéthylamine et laisse une nuit sous agitation à TA. On concentre sous vide, extrait le résidu à l'AcOEt, filtre un insoluble, lave le filtrat par une solution aqueuse saturée de $NaHCO_3$, par une solution tampon pH = 2, par une solution saturée de NaCl, sèche sur $Na_2SO_4$ et évapore sous vide le solvant. On chromatographie le résidu sur silice en éluant par le mélange DCM/MeOH (97,5/2,5 ; v/v). On obtient 0,3 g du produit attendu, F = 195°C.

$\alpha_D^{20}$ = -6,5° (c = 0,64 ; DCM).

EXEMPLE 45

N-[(1S)-1,3,3-triméthylbicyclo[2.2.1]hept-2*endo*-yl]-1-(2,4-dichlorobenzyl)-5-(4-chlorophényl)-4-méthylpyrazole-3-carboxamide.

A) Chlorure de l'acide 1-(2,4-dichlorobenzyl)-5-(4-chlorophényl)-4-méthylpyrazole-3-carboxylique.

A une solution de 0,71 g du composé obtenu à la Préparation 1.30 dans 50 ml de toluène, on ajoute à TA 0,191 ml de chlorure de thionyle puis chauffe à reflux pendant une nuit. On concentre sous vide, reprend le résidu

au toluène et évapore sous vide le solvant. On obtient 0,72 g du produit attendu sous forme d'huile qui se solidifie.

B) N-[(1S)-1,3,3-triméthylbicyclo[2.2.1]hept-2*endo*-yl]-1-(2,4-dichlorobenzyl)-5-(4-chlorophényl)-4-méthylpyrazole-3-carboxamide.

On prépare ce composé selon le mode opératoire décrit à l'étape B de l'EXEMPLE 43 à partir de 0,315 g du composé obtenu à la Préparation 2.1, 0,46 ml de triéthylamine et 0,653 g du composé obtenu à l'étape précédente dans 50 ml de DCM. On obtient 0,68 g du produit attendu, F = 125°C.

$\alpha_D^{20}$ = -0,3° (c = 1 ; EtOH).

EXEMPLE 46

N-[(1S)-1,3,3-triméthylbicyclo[2.2.1]hept-2*endo*-yl]-1-(3,4-dichlorobenzyl)-5-(4-chlorophényl)-4-méthylpyrazole-3-carboxamide.

A) Chlorure de l'acide 1(3,4-dichlorobenzyl)-5-(4-chlorophényl)-4-méthylpyrazole-3-carboxylique.

On prépare ce composé selon le mode opératoire décrit à l'étape A de l'EXEMPLE 45 à partir de 1 g du composé obtenu à la Préparation 1.31 dans 50 ml de toluène et 0,28 ml de chlorure de thionyle. On obtient 1,03 g du produit attendu sous forme d'huile qui se solidifie.

B) N-[(1S)-1,3,3-triméthylbicyclo[2.2.1]hept-2*endo*-yl]-1-(3,4-dichlorobenzyl)-5-(4-chlorophényl)-4-méthylpyrazole-3-carboxamide.

On prépare ce composé selon le mode opératoire décrit à l'étape B de l'EXEMPLE 43 à partir de 0,315 g du composé obtenu à la Préparation 2.1, 0,46 ml de triéthylamine et 0,687 g du composé obtenu à l'étape précédente dans 50 ml de DCM. On obtient 0,72 g du produit attendu, F = 69°C.

$\alpha_D^{20}$ = -1,8° (c = 1 ; EtOH).

EXEMPLE 47

N-[(1S)-1,3,3-triméthylbicyclo[2.2.1]hept-2*endo*-yl]-1-(3-chloro-4-méthyl benzyl)-5-(4-chlorophényl)-4-méthylpyrazole-3-carboxamide.

A) Chlorure de l'acide 1-(3-chloro-4-méthylbenzyl)-5-(4-chlorophényl)-4-méthylpyrazole-3-carboxylique.

On prépare ce composé selon le mode opératoire décrit à l'étape A de l'EXEMPLE 45 à partir de 1 g du composé brut obtenu à la Préparation 1.32 dans 50 ml de toluène et 0,29 ml de chlorure de thionyle. On obtient 1,05 g du produit attendu.

B) N-[(1S)-1,3,3-triméthylbicyclo[2.2.1]hept-2*endo*-yl]-1-(3-chloro-4-méthyl benzyl)-5-(4-chlorophényl)-4-méthylpyrazole-3-carboxamide.

On prépare ce composé selon le mode opératoire décrit à l'étape B de l'EXEMPLE 43 à partir de 0,29 g du composé obtenu à la Préparation 2.1, 0,24 ml de triéthylamine et 0,6 g du composé obtenu à l'étape précédente dans 50 ml de DCM. On obtient 0,43 g du produit attendu, F = 58°C.

$\alpha_D^{20}$ = -1,7° (c = 1 ; EtOH).

EXEMPLE 48

N-[(1S)-1,3,3-triméthylbicyclo[2.2.1]hept-2*endo*-yl]-1-(4-méthylbenzyl)-5-(4-chloro-3-méthylphényl)-4-méthylpyrazole-3-carboxamide.

A) Chlorure de l'acide 1-(4-méthylbenzyl)-5-(4-chloro-3-méthylphényl)-4-méthyl pyrazole-3-carboxylique.

A une suspension de 1,5 g du composé obtenu à la Préparation 1.33 dans 40 ml de toluène, on ajoute 1 ml de chlorure de thionyle et chauffe à reflux pendant 2 heures. On concentre sous vide le mélange réactionnel, reprend le résidu au toluène et évapore sous vide le solvant. On obtient 1,5 g du produit attendu.

B) N-[(1S)-1,3,3-triméthylbicyclo[2.2.1]hept-2*endo*-yl)-1-(4-méthylbenzyl)-5-(4-chloro-3-méthylphényl)-4-méthylpyrazole-3-carboxamide.

On prépare ce composé selon le mode opératoire décrit à l'étape B de l'EXEMPLE 43 à partir de 0,42 g du

composé obtenu à la Préparation 2.1, 0,7 ml de triéthylamine et 0,8 g du composé obtenu à l'étape précédente dans 40 ml de DCM. On obtient 0,62 g du produit attendu, F = 58°C.
$\alpha_D^{20}$ = -2,4° (c = 1 ; EtOH).

EXEMPLE 49

N-[(1S)-1,3,3-triméthylbicyclo[2.2.1]hept-2*endo*-yl]-1-(3,4-dichlorobenzyl)-5-(4-chloro-3-méthylphényl)-4-méthylpyrazole-3-carboxamide.

A) Chlorure de l'acide 1-(3,4-dichlorobenzyl)-5-(4-chloro-3-méthylphényl)-4-méthyl pyrazole-3-carboxylique.
A une suspension de 2,7 g du composé obtenu à la Préparation 1.34 dans 50 ml de toluène, on ajoute 1,5 ml de chlorure de thionyle et chauffe à reflux pendant 2 heures. On concentre sous vide le mélange réactionnel, reprend le résidu au toluène et évapore sous vide le solvant. On obtient 2,8 g du produit attendu.
B) N-[(1S)-1,3,3-triméthylbicyclo[2.2.1]hept-2*endo*-yl]-1-(3,4-dichlorobenzyl)-5-(4-chloro-3-méthylphényl)-4-méthylpyrazole-3-carboxamide.
On prépare ce composé selon le mode opératoire décrit à l'étape B de l'EXEMPLE 43 à partir de 0,683 g du composé obtenu à la Préparation 2.1, 1,2 ml de triéthylamine et 1,5 g du composé obtenu à l'étape précédente dans 40 ml de DCM. On obtient 1,03 g du produit attendu, F = 69°C.
$\alpha_D^{20}$ = -2,1° (c = 1 ; EtOH).

EXEMPLE 50

N-[(1S)-1,3,3-triméthylbicyclo[2.2.1]hept-2*endo*-yl]-1-(3-chloro-4-méthyl benzyl)-5-(4-chlorophényl)-4-(éthoxyméthyl) pyrazole-3-carboxamide.

A) Chlorure de l'acide 1-(3-chloro-4-méthylbenzyl)-5-(4-chlorophényl)-4-(éthoxyméthyl)pyrazole-3-carboxylique.
A une solution de 0,2 g du composé obtenu à la Préparation 1.35 dans 30 ml de toluène on ajoute 0,053 ml de chlorure de thionyle et chauffe à reflux pendant 2 heures. On concentre sous vide, reprend le résidu au toluène et évapore sous vide le solvant. On obtient 0,21 g du produit attendu sous forme d'huile.
B) N-[(1S)-1,3,3-triméthylbicyclo[2.2.1]hept-2*endo*-yl]-1-(3-chloro-4-méthyl benzyl)-5-(4-chlorophényl)-4-(éthoxyméthyl)pyrazole-3-carboxamide.
On prépare ce composé selon le mode opératoire décrit à l'étape B de l'EXEMPLE 40 à partir de 0,086 g du composé obtenu à la Préparation 2.1 dans 25 ml de DCM, 0,125 ml de triéthylamine et 0,2 g du composé obtenu à l'étape précédente dans 25 ml de DCM. On obtient 0,15 g du produit attendu, F = 50-51°C.
$\alpha_D^{20}$ = -2,8° (c = 1 ; EtOH).

EXEMPLE 51

N-[(1S)-1,3,3-triméthylbicyclo[2.2.1]hept-2*endo*-yl]-1-(5-chloroindan-1-yl)-5-(4-chlorophényl)pyrazole-3-carboxamide.

A) Chlorure de l'acide 1-(5-chloroindan-1-yl)-5-(4-chlorophényl)pyrazole-3-carboxylique.
A une suspension de 1 g du composé obtenu à la Préparation 1.36 dans 20 ml de toluène, on ajoute 0,6 ml de chlorure de thionyle et chauffe à reflux pendant 2 heures. Après refroidissement à TA, on concentre sous vide, reprend le résidu au toluène et évapore sous vide le solvant. On obtient 1 g du produit attendu.
B) N-[(1S)-1,3,3-triméthylbicyclo[2.2.1]hept-2*endo*-yl]-1-(5-chloroindan-1-yl)-5-(4-chlorophényl)pyrazole-3-carboxamide.
A une solution de 0,493 g du composé obtenu à la Préparation 2.1 et 1 ml de triéthylamine dans 15 ml de DCM, on ajoute à TA et goutte à goutte une solution de 1 g du composé obtenu à l'étape précédente dans 15 ml de DCM et laisse une nuit sous agitation à TA. On concentre sous vide, reprend le résidu à l'AcOEt, filtre un insoluble, lave le filtrat par une solution aqueuse saturée de $NaHCO_3$, par une solution tampon pH = 2, par une solution aqueuse saturée de NaCl, sèche sur $Na_2SO_4$ et évapore sous vide le solvant. On chromatographie le

résidu sur silice en éluant par le mélange cyclohexane/AcOEt (80/20 ; v/v). On obtient 0,71 g du produit attendu, F = 86°C.

$\alpha_D^{20}$ = +0,5° (c = 1 ; EtOH).

RMN : δ (ppm) : 0,4 à 1,8 : m : 16H ; 2,5 : mt : 2H ; 2,95 : mt : 2H ; 3,5 : d : 1H ; 5,8 : t : 1H ; 6,6 à 7,7 : m : 9H.

On observe un effet Overhauser (N.O.E.) entre le proton en position 1 du groupe indan-1-yle et les protons $g_2 = g_6 = H$.

EXEMPLE 52

1-(3,4-Dichlorobenzyl)-5-(4-méthylphényl)pyrazole-3-carboxylate de (1R)-1,3,3-triméthylbicyclo[2.2.1]hept-2*endo*-yle.

On laisse 72 heures sous agitation à TA un mélange de 1,5 g du composé obtenu à l'étape A de l'EXEMPLE 1, 0,73 g d'alcool (1R)-*endo*-(+)-fenchyliquc, 50 ml de pyridine et 0,02 g de 4-diméthylaminopyridine. On concentre sous vide le mélange réactionnel et chromatographie le résidu sur silice en éluant au DCM. On obtient 0,23 g du produit attendu, F = 107°C.

$\alpha_D^{20}$ = +6,8° (c = 1 ; MeOH).

EXEMPLE 53

1-(3-chloro-4-méthylbenzyl)-5-(3,4-dichlorophényl)pyrazole-3-carboxylate de (1R)-1,3,3-triméthylbicyclo[2.2.1]hept-2*endo*-yle.

A une solution de 2,6 g du composé obtenu à l'étape A de l'EXEMPLE 36 dans 50 ml de pyridine, on ajoute 0,97 g d'alcool (1R)-*endo*-(+)-fenchylique et 0,02 g de 4-diméthylaminopyridine puis laisse 48 heures sous agitation à TA. On concentre sous vide, reprend le résidu à l'éther, essore le précipité formé et le sèche. On chromatographie le précipité sur silice en éluant par le mélange cyclohexane/AcOEt/DCM (85/15/3 ; v/v/v). On obtient 0,40 g du produit attendu, F = 161°C.

$\alpha_D^{20}$ = +3,9° (c = 1 ; DCM).

EXEMPLE 54

N-[(1S)-1,3,3-triméthylbicyclo[2.2.1]hept-2*endo*-yl]-1-(2,4-dichlorobenzyl)-3-(4-chlorophényl)pyrazole-5-carboxamide.

A) Chlorure de l'acide 1-(2,4-dichlorobenzyl)-3-(4-chlorophényl)pyrazole-5-carboxylique.

A une suspension de 0,79 g du composé obtenu à la Préparation 1.38 dans 20 ml de toluène on ajoute 0,54 ml de chlorure de thionyle et chauffe à reflux pendant 3 heures. On concentre sous vide, reprend le résidu dans 20 ml de toluène et évapore sous vide le solvant. On obtient 0,9 g du produit attendu.

B) N-[(1S)-1,3,3-triméthylbicyclo[2.2.1]hept-2*endo*-yl]-1-(2,4-dichlorobenzyl)-3-(4-chlorophényl)pyrazole-5-carboxamide.

On refroidit à 0°C une solution de 0,21 g du composé obtenu à la Préparation 2.1 et 0,3 ml de triéthylamine dans 10 ml de DCM, ajoute goutte à goutte une solution de 0,45 g du composé obtenu à l'étape précédente dans 15 ml de DCM et laisse 16 heures sous agitation à TA. On verse le mélange réactionnel dans 100 ml d'eau glacée, extrait au DCM, lave la phase organique à l'eau, par une solution saturée de NaCl, sèche sur $Na_2SO_4$ et évapore sous vide le solvant. On obtient 0,37 g du produit attendu après cristallisation dans le mélange DCM/éther iso, F = 171°C.

$\alpha_D^{20}$ = -3,2° (c = 0,5 ; EtOH

EXEMPLE 55 Gélule

| | |
|---|---|
| Composé de l'EXEMPLE 18 | 1 mg |

(suite)

| | |
|---|---|
| Amidon de maïs modifié | 47 mg |
| Lactose monohydrate EFK | 150 mg |
| Stéarate de magnésium | 2 mg |
| Pour une gélule blanc opaque, taille 3, remplie à : | 200 mg |

EXEMPLE 56 Gélule

| | |
|---|---|
| Composé de l'EXEMPLE 18 | 30 mg |
| Amidon de maïs modifié | 30 mg |
| Stéarate de magnésium | 2 mg |
| Lactose monohydrate EFK Q/S | |
| Pour une gélule blanc opaque, taille 3, remplie à : | 200 mg |

EXEMPLE 57 Comprimé

| | |
|---|---|
| Composé de l'EXEMPLE 18 | 10 mg |
| PVP (polyvinylpyrrolidone) K3O | 4,5 mg |
| Carboxyméthylcellulose sodique réticulée | 3 mg |
| Stéarate de magnésium | 1,5 mg |
| Lactose monohydrate 200 mesh Q/S | |
| Eau purifiée Q/S | |
| Pour un comprimé sécable terminé à : | 150 mg |

EXEMPLE 58 Gélule

| | |
|---|---|
| Composé de l'EXEMPLE 48 | 30 mg |
| Hydroxypropylméthylcellulose 6 mPas | 7,5 mg |
| Lactose monohydrate 200 mesh Q/S | |
| Stéarate de magnésium | 2,5 mg |
| Eau purifiée Q/S | |
| Pour une gélule de taille 1, remplie à : | 250 mg |

EXEMPLE 59 Comprimé

| | |
|---|---|
| Composé de l'EXEMPLE 48 | 40 mg |
| Amidon de maïs | 50 mg |
| PVP K30 | 9 mg |
| Carboxyméthylamidon sodique | 9 mg |
| Stéarate de magnésium | 3 mg |

(suite)

| | |
|---|---|
| Lactose monohydrate 200 mesh Q/S | |
| Pour un comprimé sécable terminé à : | 300 mg |

**Revendications**

**1.** Composé de formule :

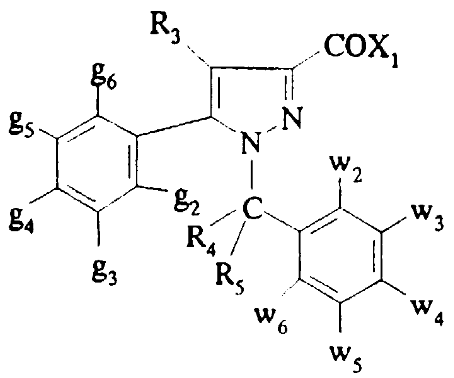

(I)

dans laquelle :

- $X_1$ représente un groupe $-NR_1R_2$ ou un groupe $-OR_2$ ;
- $g_2$, $g_3$, $g_4$, $g_5$, $g_6$ et $w_2$, $w_3$, $w_4$, $w_5$, $w_6$ sont identiques ou différents, et représentent chacun indépendamment l'hydrogène, un atome d'halogène, un $(C_1-C_4)$alkyle, un $(C_1-C_4)$alcoxy, un trifluorométhyle, un nitro, un $(C_1-C_4)$alkylthio ; à la condition qu'au moins un des substituants $g_2$, $g_3$, $g_4$, $g_5$, $g_6$ et au moins un des substituants $w_2$, $w_3$, $w_4$, $w_5$, $w_6$ soient différents de l'hydrogène ;
- $R_1$ représente l'hydrogène ou un $(C_1-C_4)$alkyle ;
- $R_2$ représente un radical carbocyclique non aromatique en $(C_3-C_{15})$ non substitué ou substitué une ou plusieurs fois par un substituant choisi parmi un atome d'halogène, un $(C_1-C_4)$alkyle ou un $(C_1-C_4)$alcoxy ;
- $R_3$ représente l'hydrogène ou un groupe $-CH_2-R_6$ ;
- $R_4$ et $R_5$ représentent chacun indépendamment un hydrogène, un $(C_1-C_4)$alkyle ou un trifluorométhyle ;
- ou bien $R_4$ représente l'hydrogène et $R_5$ et $w_6$ ensemble constituent un radical éthylène ou un radical triméthylène ;
- $R_6$ représente l'hydrogène, ou lorsque les substituants $g_2$, $g_3$, $g_4$, $g_5$ et/ou $g_6$ sont autres qu'un $(C_1-C_4)$alkyle, $R_6$ représente l'hydrogène, un $(C_1-C_4)$alkyle, un fluor, un hydroxy, un $(C_1-C_5)$alcoxj,, un $(C_1-C_5)$alkylthio, un hydroxy$(C_1-C_5)$alcoxy, un cyano, un $(C_1-C_5)$alkylsulfinyle, un $(C_1$-C5)alkylsulfonyle ;

et ses sels.

**2.** Composé de formule (I) selon la revendication 1 dans laquelle $X_1$ représente un groupe $-NR_1R_2$ dans lequel $R_1$ représente l'hydrogène ; et ses sels.

**3.** Composé de formule (I) selon la revendication 1 dans laquelle $X_1$ représente un groupe $-NR_1R_2$ ou un groupe $-OR_2$ dans lesquels $R_2$ représente un radical 1,3,3-triméthylbicyclo[2.2.1]hept-2-yle ou un radical bicyclo[3.2.1]oct-3-yle ; et ses sels.

**4.** Composé de formule (I) selon la revendication 1 dans laquelle $R_3$ représente l'hydrogène ou un groupe $-CH_2-R_6$ dans lequel $R_6$ représente l'hydrogène ; et ses sels.

**5.** Composé de formule (I) selon la revendication 1 dans laquelle soit $R_4$ et $R_5$ représentent chacun l'hydrogène, soit $R_4$ représente l'hydrogène et $R_5$ représente un $(C_1-C_4)$alkyle ; et ses sels.

**6.** Composé de formule (I) selon la revendication 1 dans laquelle $g_2$, $g_5$ et $g_6$ représentent chacun l'hydrogène et $g_3$ et $g_4$ sont tels que définis pour les composés de formule (I) dans la revendication 1 ; et ses sels.

**7.** Composé de formule (I) selon la revendication 1 dans laquelle $w_5$ et $w_6$ représentent chacun l'hydrogène, $w_4$ représente un atome d'halogène, un $(C_1-C_4)$alkyle, un $(C_1-C_4)$alcoxy, un trifluorométhyle, ou un $(C_1-C_4)$alkylthio, et soit $w_2$ et $w_3$ représentent chacun l'hydrogène, soit l'un représente l'hydrogène et l'autre représente un atome d'halogène, un $(C_1-C_4)$alkyle ou un trifluorométhyle ; et ses sels.

**8.** Composé selon la revendication 1 de formule:

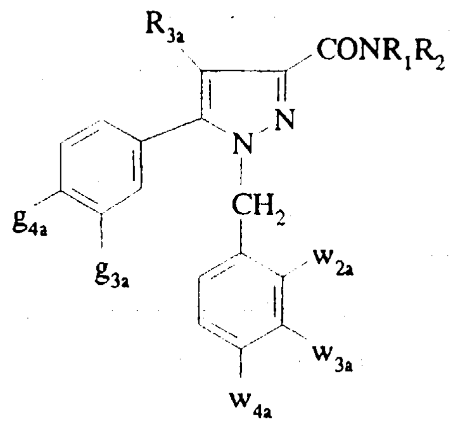

(Ia)

dans laquelle :

- $R_1$ et $R_2$ sont tels que définis pour les composés de formule (I) dans la revendication 1 ;
- $R_{3a}$ représente l'hydrogène ou un groupe $-CH_2-R_{6a}$;
- $R_{6a}$ représente l'hydrogène ou à la condition que les substituants $g_{3a}$ et $g_{4a}$ soient autres qu'un $(C_1-C_4)$alkyle, $R_{6a}$ représente l'hydrogène, un groupe méthyle ou un groupe éthyle ;
- $g_{3a}$ représente l'hydrogène; un atome d'halogène, un $(C_1-C_4)$alkyle ou un trifluorométhyle ;
- $g_{4a}$ représente un atome d'halogène, un $(C_1-C_4)$alkyle ou un trifluorométhyle ;
- $w_{4a}$ représente un atome d'halogène, un $(C_1-C_4)$alkyle ou un trifluorométhyle ;
- $w_{2a}$ et $w_{3a}$ représentent chacun l'hydogène ou l'un représente l'hydrogène et l'autre représente un atome d'halogène, un $(C_1-C_4)$alkyle ou un trifluorométhyle ;

et ses sels.

**9.** Composé selon la revendication 1 de formule :

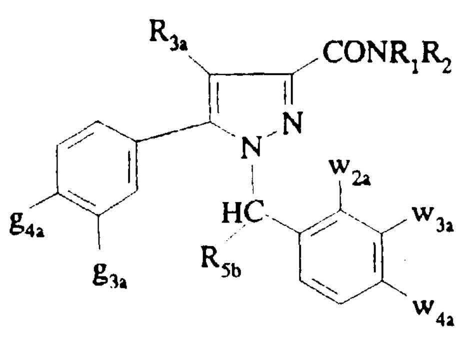

(Ib)

dans laquelle :

- $R_1$ et $R_2$ sont tels que définis pour les composés de formule (I) dans la revendication 1 ;
- $R_{3a}$, $g_{3a}$, $g_{4a}$, $w_{2a}$, $w_{3a}$ et $w_{4a}$ sont tels que définis pour les composés de formule (Ia) dans la revendication 8 ;
- $R_{5b}$ représente un $(C_1-C_4)$alkyle ;

et ses sels.

**10.** Composé selon la revendication 1 de formule :

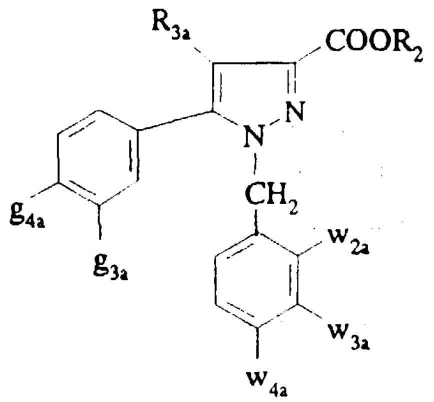

(Ic)

dans laquelle :

- $R_2$ est tel que défini pour les composés de formule (I) dans la revendication 1 ;
- $R_{3a}$, $w_{2a}$, $w_{3a}$, $w_{4a}$, $g_{3a}$ et $g_{4a}$ sont tels que définis pour les composés de formule (Ia) dans la revendication 8 ;

et ses sels.

**11.** Procédé pour la préparation des composés de formule (I) selon la revendication 1 et de leurs sels, caractérisé en ce que :

1) on traite un dérivé fonctionnel de l'acide pyrazole-3-carboxylique de formule :

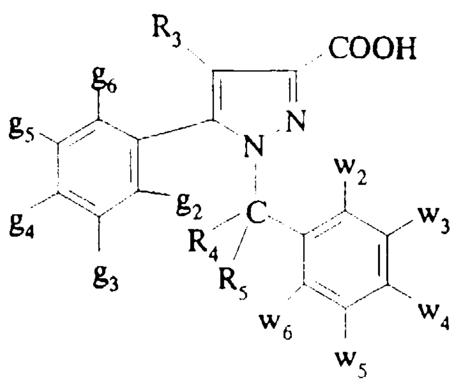

(II)

dans laquelle $w_2$, $w_3$, $w_4$, $w_5$, $w_6$, $g_2$, $g_3$, $g_4$, $g_5$, $g_6$, $R_3$, $R_4$ et $R_5$ sont tels que définis pour les composés de formule (I) dans la revendication 1, avec un composé de formule :

$$H\text{-}X_1 \qquad \text{(XXIV)}$$

dans laquelle $X_1$ est tel que défini pour les composés de formule (I) dans la revendication 1 ;
2) et, éventuellement on transforme le composé ainsi obtenu en l'un de ses sels.

**12.** Procédé selon la revendication 11 pour la préparation des composés de formule (I) dans laquelle $X_1$ représente un groupe $-NR_1R_2$ et de leurs sels, caractérisé en ce que :

1) on traite un dérivé fonctionnel de l'acide pyrazole-3-carboxylique de formule (II) tel que défini dans la revendication 11, avec une amine de formule

$$HNR_1R_2 \qquad (III)$$

dans laquelle $R_1$ et $R_2$ sont tels que définis pour les composés de formule (I) dans la revendication 1 ;
2) et, éventuellement on transforme le composé ainsi obtenu en l'un de ses sels.

**13.** Procédé selon la revendication 11 pour la préparation des composés de formule (I) dans laquelle $X_1$ représente un groupe $-OR_2$ et de leurs sels, caractérisé en ce que :

1) on traite un dérivé fonctionnel de l'acide pyrazole-3-carboxylique de formule (II) tel que défini dans la revendication 11 avec un alcool de formule :

$$HO\text{-}R_2 \qquad (XIV)$$

dans laquelle $R_2$ est tel que défini pour les composés de formule (I) dans la revendication 1 ;
2) et, éventuellement on transforme le composé ainsi obtenu en l'un de ses sels.

**14.** Composé de formule :

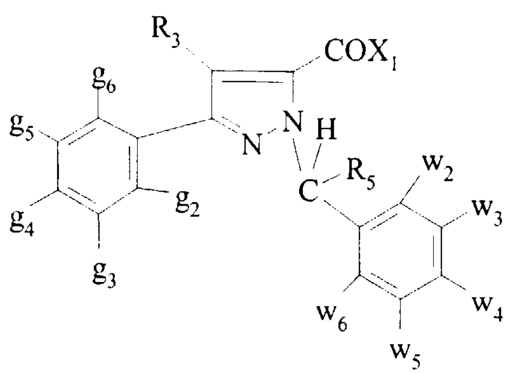

(XIII)

dans laquelle :

- $X_1$, $g_2$, $g_3$, $g_4$, $g_5$, $w_2$, $w_3$, $w_4$, $w_5$, $w_6$, $R_3$ et $R_5$ sont tels que définis pour les composés de formule (I) dans la revendication 1 ;

et ses sels.

**15.** Procédé pour la préparation des composés de formule (XIII) selon la revendication 14, et de leurs sels, caractérisé en ce que :

1) on traite un dérivé fonctionnel de l'acide de formule (XII):

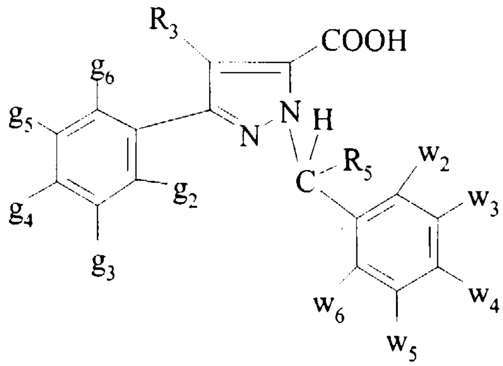

(XII)

dans laquelle $g_2$, $g_3$, $g_4$, $g_5$, $g_6$ $w_2$, $w_3$, $w_4$, $w_5$, $w_6$, $R_3$ et $R_5$ sont tels que définis pour les composés de formule (I) dans la revendication 1, avec un composé de formule :

$$H\text{-}X_1 \qquad (XXIV)$$

dans laquelle $X_1$ est tel que défini pour les composés de formule (I) dans la revendication 1, pour obtenir le composé de formule :

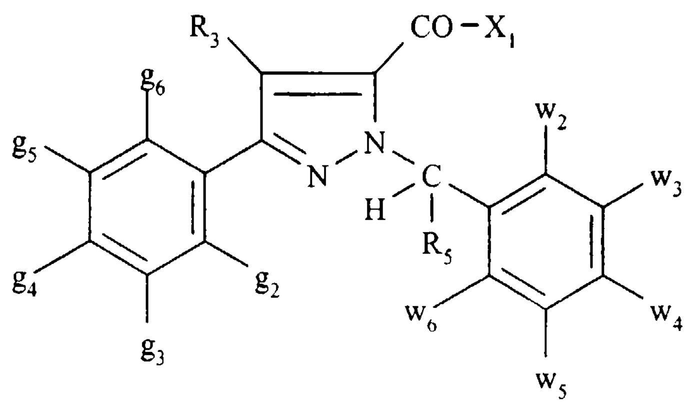

(XIII) ;

2) et, éventuellement on transforme le composé ainsi obtenu en l'un de ses sels.

**16.** Composé selon l'une quelconque des revendications 1 à 10 ou 14, qui est sous forme radiomarquée.

**17.** Composition pharmaceutique contenant à titre de principe actif, un composé selon l'une quelconque des revendications 1 à 10, ou un de ses sels pharmaceutiquement acceptables.

**18.** Composition pharmaceutique selon la revendication 17, sous forme d'unité de dosage, dans laquelle le principe actif est mélangé à au moins un excipient pharmaceutique.

**19.** Composition pharmaceutique selon la revendication 18, contenant 0,5 à 1000 mg de principe actif.

**20.** Composition pharmaceutique selon la revendication 19, contenant 2,5 à 250 mg de principe actif.

**21.** Utilisation d'un composé selon l'une quelconque des revendications 1 à 10 ou d'un de ses sels pharmaceutiquement acceptables pour la préparation de médicaments destinés au traitement des troubles dans lesquels les récepteurs $CB_2$ sont impliqués.

**Patentansprüche**

**1.** Verbindung der Formel:

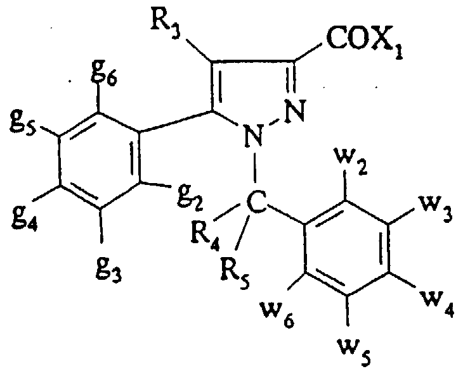

(I),

worin:

- $X_1$ eine Gruppe $-NR_1R_2$ oder eine Gruppe $-OR_2$ bedeutet;
- $g_2$, $g_3$, $g_4$, $g_5$, $g_6$ und $w_2$, $w_3$, $w_4$, $w_5$, $w_6$ identisch oder verschieden sind, und jeweils unabhängig Wasserstoff, ein Halogenatom, ein $(C_1-C_4)$Alkyl, ein $(C_1-C_4)$Alkoxy, ein Trifluormethyl, ein Nitro, ein $(C_1-C_4)$Alkylthio darstellen; mit der Maßgabe, daß zumindest einer der Substituenten $g_2$, $g_3$, $g_4$, $g_5$, $g_6$ und zumindest einer der Substituenten $w_2$, $w_3$, $w_4$, $w_5$, $w_6$ von Wasserstoff verschieden sind;
- $R_1$ Wasserstoff oder ein $(C_1-C_4)$Alkyl bedeutet;
- $R_2$ einen nicht-aromatischen carbocyclischen $(C_3-C_{15})$ Rest, nichtsubstituiert oder ein- oder mehrfach substituiert durch einen Substituenten, ausgewählt aus einem Halogenatom, einem $(C_1-C_4)$Alkyl oder einem $(C_1-C_4)$Alkoxy, darstellt;
- $R_3$ Wasserstoff oder eine Gruppe $-CH_2-R_6$ ist;
- $R_4$ und $R_5$ jeweils unabhängig einen Wasserstoff, ein $(C_1-C_4)$Alkyl oder ein Trifluormethyl bedeuten;
- oder auch $R_4$ Wasserstoff darstellt, und $R_5$ und $w_6$ gemeinsam einen Ethylenrest oder einen Trimethylenrest bilden;
- $R_6$ Wasserstoff ist, oder, wenn die Substituenten $g_2$, $g_3$, $g_4$, $g_5$ und/oder $g_6$ von einem $(C_1-C_4)$Alkyl verschieden sind, $R_6$ Wasserstoff, ein $(C_1-C_4)$Alkyl, ein Fluor, ein Hydroxy, ein $(C_1-C_5)$Alkoxy, ein $(C_1-C_5)$Alkylthio, ein Hydroxy$(C_1-C_5)$alkoxy, ein Cyano, ein $(C_1-C_5)$Alkylsulfinyl, ein $(C_1-C_5)$Alkylsulfonyl bedeutet; und ihre Salze.

**2.** Verbindung der Formel (I) nach Anspruch 1, worin $X_1$ eine Gruppe $-NR_1R_2$ bedeutet, wobei $R_1$ Wasserstoff darstellt; und ihre Salze.

**3.** Verbindung der Formel (I) nach Anspruch 1, worin $X_1$ eine Gruppe $-NR_1R_2$ oder eine Gruppe $-OR_2$ bedeutet, wobei $R_2$ einen 1,3,3-Trimethylbicyclo[2.2.1]hept-2-ylrest oder einen Bicyclo[3.2.1]oct-3-ylrest darstellt; und ihre Salze.

**4.** Verbindung der Formel (I) nach Anspruch 1, worin $R_3$ Wasserstoff oder eine Gruppe $-CH_2-R_6$ bedeutet, wobei $R_6$ Wasserstoff darstellt; und ihre Salze.

**5.** Verbindung der Formel (I) nach Anspruch 1, worin entweder $R_4$ und $R_5$ jeweils Wasserstoff bedeuten, oder $R_4$ Wasserstoff darstellt, und $R_5$ ein $(C_1-C_4)$Alkyl ist; und ihre Salze.

**6.** Verbindung der Formel (I) nach Anspruch 1, worin $g_2$, $g_5$ und $g_6$ jeweils Wasserstoff bedeuten, und $g_3$ und $g_4$ wie für die Verbindungen der Formel (I) in Anspruch 1 definiert sind; und ihre Salze.

**7.** Verbindung der Formel (I) nach Anspruch 1, worin $w_5$ und $w_6$ jeweils Wasserstoff bedeuten, $w_4$ ein Halogenatom, ein $(C_1-C_4)$Alkyl, ein $(C_1-C_4)$Alkoxy, ein Trifluormethyl oder ein $(C_1-C_4)$Alkylthio darstellt, und entweder $w_2$ und $w_3$ jeweils Wasserstoff sind oder eines Wasserstoff bedeutet und das andere ein Halogenatom, ein $(C_1-C_4)$Alkyl oder ein Trifluormethyl darstellt; und ihre Salze.

**8.** Verbindung nach Anspruch 1 der Formel:

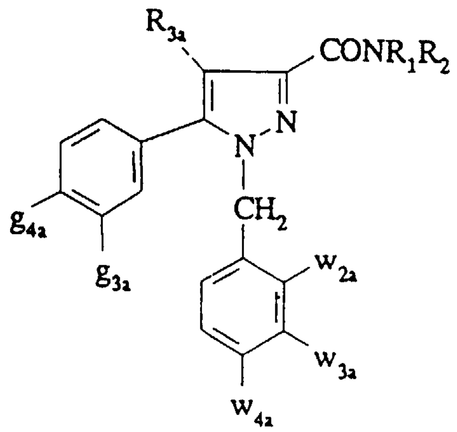

(Ia),

worin:

- $R_1$ und $R_2$ wie für die Verbindungen der Formel (I) in Anspruch 1 definiert sind;
- $R_{3a}$ Wasserstoff oder eine Gruppe $-CH_2-R_{6a}$ bedeutet;
- $R_{6a}$ Wasserstoff darstellt oder, mit der Maßgabe, daß die Substituenten $g_{3a}$ und $g_{4a}$ von einem $(C_1-C_4)$Alkyl verschieden sind, $R_{6a}$ Wasserstoff, eine Methyl-Gruppe oder eine Ethyl-Gruppe bedeutet;
- $g_{3a}$ Wasserstoff, ein Halogenatom, ein $(C_1-C_4)$Alkyl oder ein Trifluormethyl darstellt;
- $g_{4a}$ ein Halogenatom, ein $(C_1-C_4)$Alkyl oder ein Trifluormethyl ist;
- $w_{4a}$ ein Halogenatom, ein $(C_1-C_4)$Alkyl oder ein Trifluormethyl bedeutet;
- $w_{2a}$ und $w_{3a}$ jeweils Wasserstoff darstellen oder eines Wasserstoff ist und das andere ein Halogenatom, ein $(C_1-C_4)$Alkyl oder ein Trifluormethyl bedeutet; und ihre Salze.

**9.** Verbindung nach Anspruch 1 der Formel:

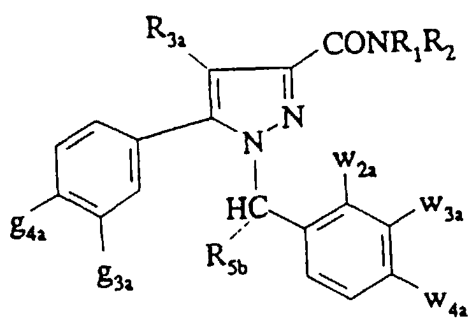

(Ib),

worin:

- $R_1$ und $R_2$ wie für die Verbindungen der Formel (I) in Anspruch 1 definiert sind;
- $R_{3a}$, $g_{3a}$, $g_{4a}$, $w_{2a}$, $w_{3a}$ und $w_{4a}$ wie für die Verbindungen der Formel (Ia) in Anspruch 8 definiert sind;
- $R_{5b}$ ein $(C_1-C_4)$Alkyl bedeutet;

und ihre Salze.

**10.** Verbindung nach Anspruch 1 der Formel:

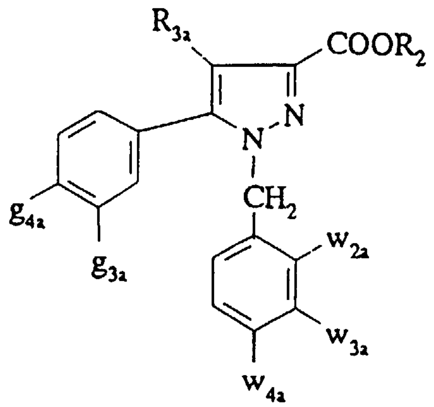

(Ic),

worin:

- $R_2$ wie für die Verbindungen der Formel (I) in Anspruch 1 definiert ist;
- $R_{3a}$, $w_{2a}$, $w_{3a}$, $w_{4a}$, $g_{3a}$ und $g_{4a}$ wie für die Verbindungen der Formel (Ia) in Anspruch 8 definiert sind; und ihre Salze.

**11.** Verfähren zur Herstellung von Verbindungen der Formel (I) nach Anspruch 1 und ihren Salzen, dadurch gekennzeichnet, daß:

1) ein funktionelles Derivat von Pyrazol-3-carbonsäure der Formel:

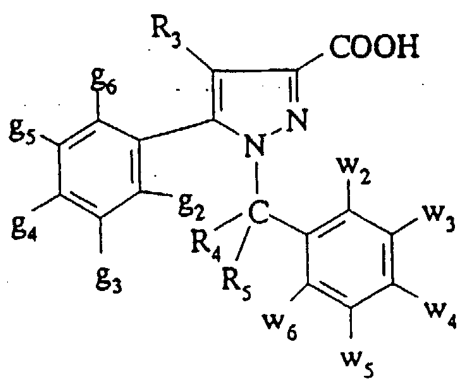

(II),

worin $w_2$, $w_3$, $w_4$, $w_5$, $w_6$, $g_2$, $g_3$, $g_4$, $g_5$, $g_6$, $R_3$, $R_4$ und $R_5$ wie für die Verbindungen der Formel (I) in Anspruch 1 definiert sind, behandelt wird mit einer Verbindung der Formel:

$$H\text{-}X_1 \qquad (XXIV),$$

worin $X_1$ wie für die Verbindungen der Formel (I) in Anspruch 1 definiert ist;
2) und gegebenenfalls die so erhaltene Verbindung in eines ihrer Salze übergeführt wird.

**12.** Verfahren nach Anspruch 11 zur Herstellung von Verbindungen der Formel (I), worin $X_1$ eine Gruppe $-NR_1R_2$ bedeutet, und ihren Salzen, dadurch gekennzeichnet, daß:

1) ein funktionelles Derivat von Pyrazol-3-carbonsäure der Formel (II), wie in Anspruch 11 definiert, behandelt wird mit einem Amin der Formel

$$HNR_1R_2 \qquad (III),$$

worin $R_1$ und $R_2$ wie für die Verbindungen der Formel (I) in Anspruch 1 definiert sind;
2) und gegebenenfalls die so erhaltene Verbindung in eines ihrer Salze übergeführt wird.

**13.** Verfahren nach Anspruch 11 zur Herstellung von Verbindungen der Formel (I), worin $X_1$ eine Gruppe $-OR_2$ bedeutet, und ihren Salzen, dadurch gekennzeichnet, daß:

1) ein funktionelles Derivat von Pyrazol-3-carbonsäure der Formel (II), wie in Anspruch 11 definiert, behandelt wird mit einem Alkohol der Formel

$$HO-R_2 \qquad (XIV),$$

worin $R_2$ wie für die Verbindungen der Formel (I) in Anspruch 1 definiert ist;
2) und gegebenenfalls die so erhaltene Verbindung in eines ihrer Salze übergeführt wird.

**14.** Verbindung der Formel:

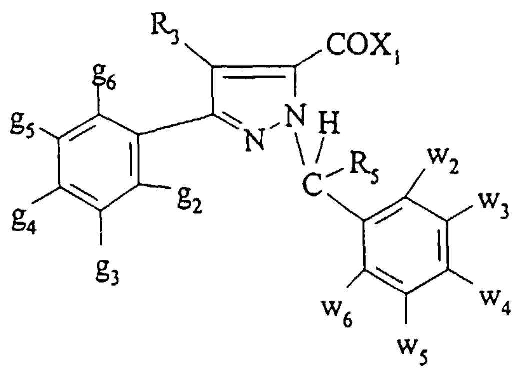

(XIII),

worin:

- $X_1$, $g_2$, $g_3$, $g_4$, $g_5$, $w_2$, $w_3$, $w_4$, $w_s$, $w_6$, $R_3$ und $R_5$ wie für die Verbindungen der Formel (I) in Anspruch 1 definiert sind; und ihre Salze.

**15.** Verfahren zur Herstellung von Verbindungen der Formel (XIII) nach Anspruch 14 und ihren Salzen, dadurch gekennzeichnet, daß:

1) ein funktionelles Derivat der Säure der Formel (XII):

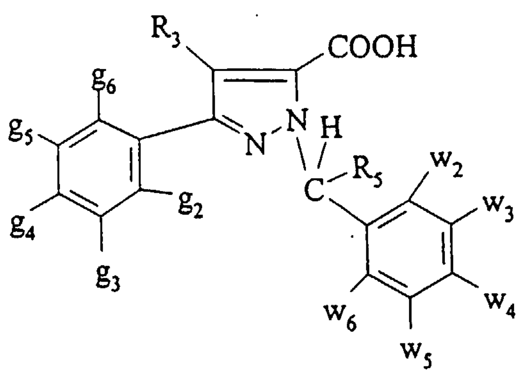

(XII),

worin $g_2$, $g_3$, $g_4$, $g_5$, $g_6$, $w_2$, $w_3$, $w_4$, $w_5$, $w_6$, $R_3$ und $R_5$ wie für die Verbindungen der Formel (I) in Anspruch 1 definiert sind, behandelt wird mit einer Verbindung der Formel:

$$H\text{-}X_1 \qquad (XXIV),$$

worin $X_1$ wie für die Verbindungen der Formel (I) in Anspruch 1 definiert ist; wobei die Verbindung der Formel:

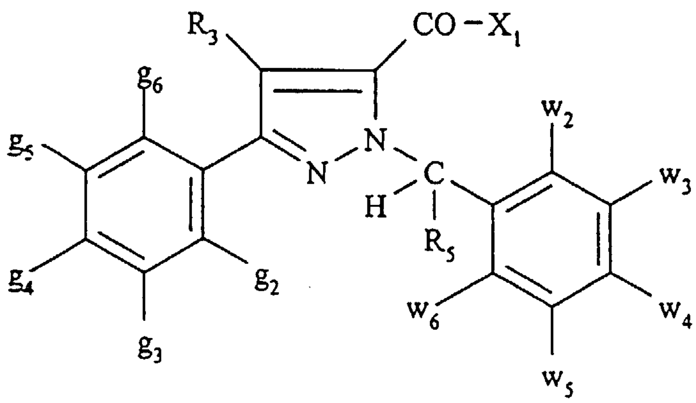

(XIII)

erhalten wird;

2) und gegebenenfalls die so erhaltene Verbindung in eines ihrer Salze übergeführt wird.

**16.** Verbindung nach einem der Ansprüche 1 bis 10 oder 14, welche in radioaktiv markierter Form vorliegt.

**17.** Pharmazeutische Zusammensetzung, welche als aktiven Bestandteil eine Verbindung nach einem der Ansprüche 1 bis 10 oder eines ihrer pharmazeutisch annehmbaren Salze enthält.

**18.** Pharmazeutische Zusammensetzung nach Anspruch 17 in Einheitsdosierungsform, worin der aktive Bestandteil mit zumindest einem Arzneimittelträger gemischt ist.

**19.** Pharmazeutische Zusammensetzung nach Anspruch 18, welche 0,5 bis 1000 mg aktiven Bestandteil enthält.

**20.** Pharmazeutische Zusammensetzung nach Anspruch 19, welche 2,5 bis 250 mg aktiven Bestandteil enthält.

**21.** Verwendung einer Verbindung nach einem der Ansprüche 1 bis 10 oder eines ihrer pharmazeutisch annehmbaren Salze zur Herstellung von Medikamenten zur Behandlung von Störungen, bei denen $CB_2$-Rezeptoren impliziert sind.

## Claims

**1.** A compound of the formula:

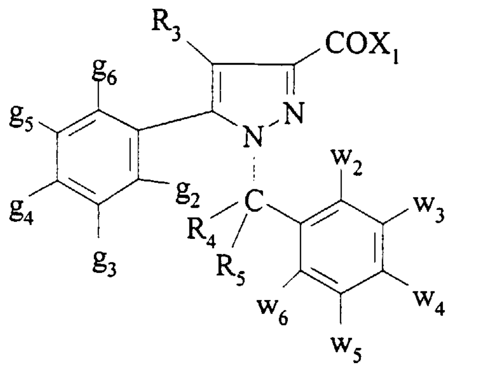

(I)

in which:

- $X_1$ is a group $-NR_1R_2$ or a group $-OR_2$;
- $g_2$, $g_3$, $g_4$, $g_5$, $g_6$ and $w_2$, $w_3$, $w_4$, $w_5$, $w_6$ are identical or different and are each independently hydrogen, a halogen atom, a $(C_1-C_4)$alkyl, a $(C_1-C_4)$alkoxy, a trifluoromethyl, a nitro or a $(C_1-C_4)$alkylthio, with the proviso that at least one of the substituents $g_2$, $g_3$, $g_4$, $g_5$, $g_6$ and at least one of the substituents $w_2$, $w_3$, $w_4$, $w_5$, $w_6$ are other than hydrogen;
- $R_1$ is hydrogen or a $(C_1-C_4)$alkyl;
- $R_2$ is a non-aromatic $(C_3-C_{15})$carbocyclic radical which is unsubstituted or monosubstituted or polysubstituted by a substituent selected from a halogen atom, a $(C_1-C_4)$alkyl and a $(C_1-C_4)$alkoxy;
- $R_3$ is hydrogen or a group $-CH_2R_6$;
- $R_4$ and $R_5$ are each independently a hydrogen, a $(C_1-C_4)$alkyl or a trifluoromethyl;
- or else $R_4$ is hydrogen and $R_5$ and $w_6$ together constitute an ethylene or trimethylene radical; and
- $R_6$ is hydrogen, or when the substituents $g_2$, $g_3$, $g_4$, $g_5$ and/or $g_6$ are other than a $(C_1-C_4)$, $R_6$ is hydrogen, a $(C_1-C_4)$alkyl, a fluorine, a hydroxyl, a $(C_1-C_5)$alkoxy, a $(C_1-C_5)$alkylthio, a hydroxy$(C_1-C_5)$alkoxy, a cyano, a $(C_1-C_5)$alkylsulfinyl or a $(C_1-C_5)$alkylsulfonyl;

and its salts.

**2.** A compound of formula (I) according to claim 1, in which $X_1$ is a group $-NR_1R_2$ in which $R_1$ is hydrogen ;
and its salts.

**3.** A compound of formula (I) according to claim 1, in which $X_1$ is a group $-NR_1R_2$ or a group $-OR_2$, where $R_2$ is a 1,3,3-trimethylbicyclo[2.2.1]kept-2-yl radical or a bicyclo[3.2.1]oct-3-yl radical ;
and its salts.

**4.** A compound of formula (I) according to claim 1, in which $R_3$ is hydrogen or a group $-CH_2R_6$, $R_6$ being hydrogen ;
and its salts.

**5.** A compound of formula (I) according to claim 1, in which either $R_4$ and $R_5$ are each hydrogen or $R_4$ is hydrogen and $R_5$ is a $(C_1-C_4)$alkyl ;
and its salts

**6.** A compound of formula (I) according to claim 1, in which $g_2$, $g_5$ and $g_6$ are each hydrogen and $g_3$ and $g_4$ are as defined for the compounds of formula (I) in claim 1 ;
and its salts.

**7.** A compound of formula (I) according to claim 1, in which $w_5$ and $w_6$ are hydrogen, $w_4$ is a halogen atom, a $(C_1-C_4)$alkyl, a $(C_1-C_4)$alkoxy, a trifluoromethyl or a $(C_1-C_4)$alkylthio and either $w_2$ and $w_3$ are each hydrogen or one is hydrogen and the other is a halogen atom, a $(C_1-C_4)$alkyl or a trifluoromethyl;
and its salts.

**8.** A comnound according to claim 1 of the formula:

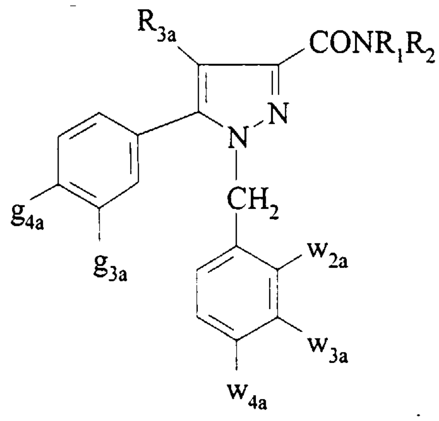

(Ia)

in which:

- $R_1$ and $R_2$ are as defined for the compounds of formula (I) in claim 1;
- $R_{3a}$ is hydrogen or a group $-CH_2R_{6a}$;
- $R_{6a}$ is hydrogen, or when the substituents $g_{3a}$ and $g_{4a}$ are other than a $(C_1-C_4)$ alkyl, $R_{6a}$ is hydrogen, a methyl group or an ethyl group;
- $g_{3a}$ is hydrogen, a halogen atom , a $(C_1-C_4)$alkyl or a trifluoromethyl;
- $g_{4a}$ is a halogen atom, a $(C_1-C_4)$alkyl or a trifluoromethyl;
- w4a is a halogen atom, a $(C_1-C_4)$alkyl or a trifluoromethyl; and
- $w_{2a}$ and $w_{3a}$ are each hydrogen or one is hydrogen and the other is a halogen atom, a $(C_1-C_4)$alkyl or a trifluoromethyl;

and its salts.

**9.** A compound according to claim 1, of the formula:

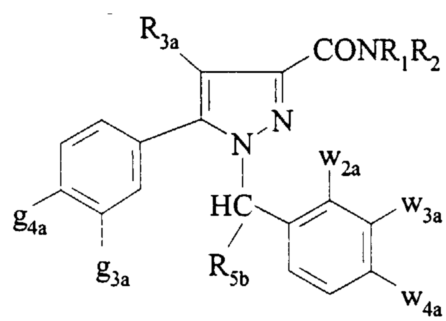

(Ib)

in which:

- $R_1$ and $R_2$ are as defined for the compounds of formula (I) in claim 1;
- $R_{3a}$, $w_{2a}$, $w_{3a}$, $w_{4a}$, $g_{3a}$ and $g_{4a}$ are as defined for the compounds of formula (Ia) in claim 8; and
- $R_{5b}$ is a $(C_1-C_4)$alkyl;

and its salts.

**10.** A compound according to claim 1, of the formula :

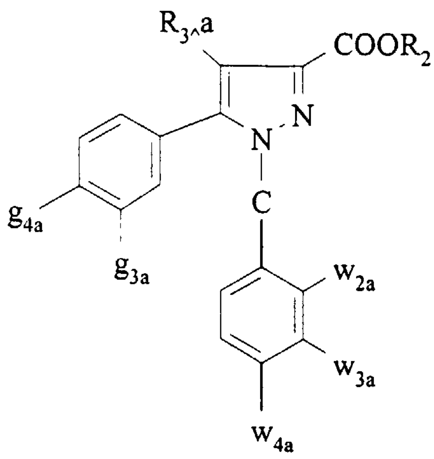

(Ic)

in which:

- $R_2$ is as defined for the compounds of formula (I) in claim 1; and
- $R_{3a}$, $w_{2a}$, $w_{3a}$, $w_{4a}$, $g_{3a}$ and $g_{4a}$ are as defined for the compounds of formula (Ia) in claim 8;

and its salts.

**11.** A process for the preparation of the compounds of formula (I) according to claim 1 and their salts, which comprises the steps of:

1) treating a functional derivative of the pyrazole-3-carboxylic acid of the formula

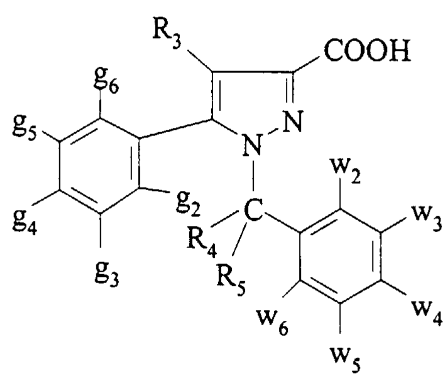

(II)

in which $w_2$, $w_3$, $w_4$, $w_5$, $w_6$, $g_2$, $g_3$, $g_4$, $g_5$, $g_6$, $R_3$, $R_4$ and $R_5$ are as defined for the compounds of formula (I) in claim 1, with a compound of the formula

$$H\text{-}X_1 \qquad \text{(XXIV)}$$

in which $X_1$ is as defined for the compounds of formula (I) in claim 1; and
2) optionally converting the resulting compound to one of its salts.

**12.** A process according to claim 11, for the preparation of the compounds of formula (I) in which $X_1$ is a group $-NR_1R_2$ and their salts, which comprises the steps of:

1) treating a functional derivative of the pyrazole-3-carboxylic acid of formula (II) as defined in claim 11 with an amine of the formula :

$$HNR_1R_2 \quad \text{(III)}$$

in which $R_1$ and $R_2$ are as defined for the compounds of formula (I) in claim 1 ; and
2) optionally converting the resulting compound to one of its salts.

**13.** A process according to claim 11, for the preparation of the compounds of formula (I) in which $X_1$ is a group $-OR_2$ and their salts, which comprises the steps of :

1) treating a functional derivative of the pyrazole-3-carboxylic acid of formula (II) as defined in claim 11 with an alcohol of the formula :

$$HOR_2 \quad \text{(XIV)}$$

in which $R_2$ is as defined for the compounds of formula (I) in claim 1; and
2) optionally converting the resulting compound to one of its salts.

**14.** A comnound of the formula:

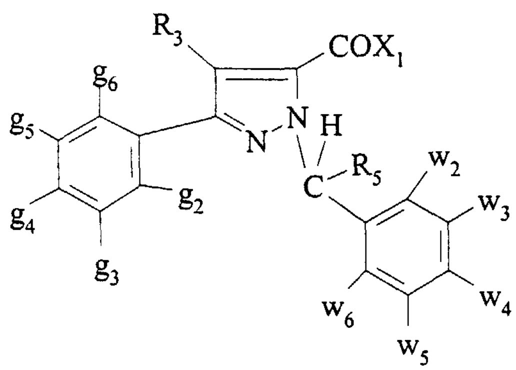

(XIII)

in which:

- $X_1$, $g_2$, $g_3$, $g_4$, $g_5$, $w_2$, $w_3$, $w_4$, $w_5$, $w_6$, $R_3$ and $R_5$ are as defined for the compounds of formula (I) in claim 1 ;

and its salts.

**15.** A process for the preparation of the compounds of formula (XIII) according to claim 14 and their salts, which comprises the steps of:

1) treating a functional derivative of the acid of formula (XII)

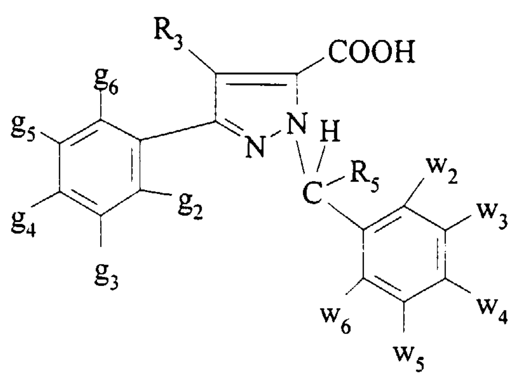

(XII)

in which $g_2$, $g_3$, $g_4$, $g_5$, $g_6$, $w_2$, $w_3$, $w_4$, $w_5$, $w_6$, $R_3$ and $R_5$ are as defined for the compounds of formula (I) in claim 1, with a compound of the formula:

$$HX_1 \quad (XXIV)$$

in which $X_1$ is as defined for the compounds of formula (I) in claim 1 to give the compound of the formula:

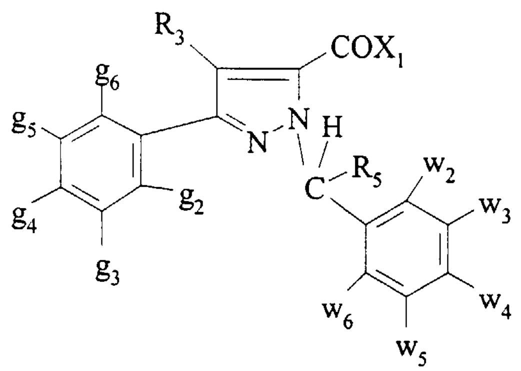

(XIII) ;

and

2/ optionally converting the resulting compound to one of its salts.

**16.** A compound according to any one of claims 1 to 10 or 14, which is in radiolabelled form.

**17.** A pharmaceutical composition which contains a compound according to any one of claims 1 to 10, or one of its pharmaceutically acceptable salts, as the active principle.

**18.** A pharmaceutical composition according to claim 17 in the form of a dosage unit, in which the active principle is mixed with at least one pharmaceutical excipient.

**19.** A pharmaceutical composition according to claim 18 which contains from 0.5 to 1000 mg of active principle.

**20.** A pharmaceutical composition according to claim 19, which contains from 2.5 to 250 mg of active principle.

**21.** Use of a compound according to any one of claims 1 to 10 or a pharmaceutically acceptable salt thereof for the preparation of medicaments intended for the treatment of diseases in which the $CB_2$ receptors are involved.